# NEW EUROPEAN PATENT SPECIFICATION

(11) **EP 0 975 755 B2**
(45) Date of publication and mention of the opposition decision: **03.08.2011**
(45) Mention of the grant of the patent: 14.02.2007
(21) Application number: 98918453.6
(22) Date of filing: 16.04.1998
(51) Int. Cl.: C12N 15/12, C07K 14/47, C07K 16/18, G01N 33/50, G01N 33/53, C12Q 1/68

(54) **CRSP PROTEIN (CYSTEINE-RICH SECRETED PROTEINS), NUCLEIC ACID MOLECULES ENCODING THEM AND USES THEREFOR**
CRSP-PROTEINE (CYSTEIN-REICHE, SEKRETIERTE PROTEINE), DIE DAFÜR KODIERENDEN NUKLEINSÄUREN UND IHRE VERWENDUNGEN
PROTEINES CRSP (PROTEINES SECRETEES RICHES EN CYSTEINE), MOLECULES D'ACIDES NUCLEIQUES CODANT CES PROTEINES ET LEURS UTILISATIONS

(30) Priority: 16.04.1997 US 843704; 17.04.1997 US 842898; 15.01.1998 US 71589 P; 20.01.1998 US 9802
(43) Date of publication of application: 02.02.2000
(60) Divisional application: 06021943.3
(73) Proprietor: MILLENNIUM PHARMACEUTICALS, INC., Cambridge, MA 02139 (US)
(72) Inventor: MCCARTHY, Sean, A., Boston, MA 02116 (US)
(74) Representative: von Menges, Albrecht
(86) International application number: PCT/US1998/007894
(87) International publication number: WO 1998/046755

(56) References cited:
- US-A- 5 525 486
- EMBL database entry HS266245; accession number H99266; 16. Dec. 1995; Hiller et al.: 'The WashU-Merck EST Project.' XP002077278
- EMBL database entry HSAA53464; accession number AA253464; 15. March 1997; Hillier et al.: The WashU-Merck EST Project.' XP002077279
- EMBL database entry HSA37322; accession number AA037322; 28. Aug. 1996; Hillier et al.: 'The WashU-Merck EST project.' XP002077280
- EMBL database entry HS979350; accession number W55979; 6. June 1996; Hillier et al.: 'The WashU-Merck EST Project.' XP002077281
- KLEIN R. D. ET AL.: "Selection for genes encoding secreted proteins and receptors." PROC. NATL. ACAD. SCI. USA, vol. 93, July 1996, pages 7108-7113, XP002077277
- EMBL database entry AF034208; accession number AF034208; 6. Jan. 1998; Ligon A.H. et al.: 'Homo sapiens RIG-like 7-1 mRNA.' XP002077282 cited in the application
- EMBL database entry AA565546; accession number AA565546; 11. Sept. 1997; Robert Strausberg: 'NCI, Cancer Genome Anatomy Project.' XP002077283
- OJ EPO 6/2002, 293
- OJ EPO 2/1999, 57
- Glinda et al., Nature 39(22):357-362 (Jan. 1998)
- Krupnik et al., Gene 238:301-313 (1999)

## Description

### Background of the Invention

Secreted proteins play an integral role in the formation, differentiation, and maintenance of cells in multicellular organisms. For instance, secretory proteins are known in the art to be involved in signalling between cells which are hot in direct contact. Such secreted signaling molecules are particularly important in the development of vertebrate tissue during embryogenesis as well as in the maintenance of the differentiated state of adult tissues. For example, inductive interactions that occur between neighboring cell layers and tissues In the developing embryo are largely dependent on the existence and regulation of secreted signaling molecules. In inductive interactions, biochemical signals secreted by one cell population influence the developmental fate of second cell population, typically by altering the fate of the second cell population. For example, the Wnt proteins are now recognized as one of the major families of developmentally important signaling molecules in organisms ranging from Drosophila to mice. For review see Cadigan, K.M. and R.Nusse (1997) Genes & Development, 11:3286-3305.

It is also now well recognized that major families of signaling molecules have a dual role to play in both the development of an organism as well as in promoting or maintaining the differentiated state of tissues in the adult animal. Furthermore, major families of signaling molecules have been implicated in controlling proliferation of cells in mature adult tissue, for example, during normal cell turnover in the adult organism as well as in tissue regeneration activated as a result of damage to the adult tissue.

### Summary of the Invention

The present invention provides an isolated nucleic acid molecule selected from the group consisting of:
a) a nucleic acid comprising SEQ ID NO:3 or SEQ ID NO:7;
b) a nucleic acid which encodes a polypeptide comprising the amino acid sequence of amino acid residues 24-350 of SEQ ID NO:2;
c) a nucleic acid which encodes a polypeptide that comprises a naturally occurring allelic variant of the amino acid sequence of amino acid residues 20-224 of SEQ ID NO:5, SEQ ID NO:11, or SEQ ID NO:14, wherein said naturally occurring allelic variant modulates cellular signal transduction or cellular proliferation, and wherein (i) said nucleic acid which encodes a naturally occurring allelic variant of the amino acid sequence of amino acid residues 20-224 of SEQ ID NO:5 hybridizes to the complement of SEQ ID NO:6 under stringent wash conditions. (ii) said nucleic acid which encodes a naturally occurring altelic variant of the amino acid sequence of SEQ ID NO:11 hybridizes to the complement of SEQ ID NO:12 under stringent wash conditions, and consists of SEQ ID NO:12, (iii) said nucleic acid which encodes a naturally occurring allelic variant of the amino acid sequence of SEQ ID NO:14 hybridizes to the complement of SEQ ID NO:15 under stringent wash conditions, wherein stringent wash conditions are 0.2X SSC, 0.1% SDS at 50-65°C;
d) nucleic acid comprising a nucleotide sequence that is the complement of a), b), c)(i) or c)(iii);
e) a nucleic acid consisting of SEQ ID NO:9; and
f) a recombinant eukaryotic cell expression vector comprising the nucleic acid of a), b), c)(i), c) (iii) or d), or a nucleic acid comprising SEQ ID NO:9.

The invention further provides a host cell comprising the recombinant eukaryotic cell expression vector as described above.

In another aspect the present invention provides an isolated polypeptide selected from the group consisting of:
a) a polypeptide comprising at least 25 contiguous amino acids of SEQ ID NO:5 or SEQ ID NO: 14, or a polypeptide comprising at least 100 contiguous amino acids of SEQ ID NO:11, or a polypeptide comprising an amino acid sequence selected from the group consisting of SEQ ID NO:8 and amino acid residues 21-266 of SEQ ID NO:8, which polypeptide modulates cellular signal transduction or cellular proliferation;
b) a polypeptide comprising a naturally occurring allelic variant of the amino acid sequence of amino acid residues 20-224 of SEQ ID NO:5, amino acid residues 21-266 of SEQ ID NO:8, or SEQ ID NO:14, wherein said naturally occurring allelic variant modulates cellular signal transduction or cellular proliferation and wherein (i) said naturally occurring allelic variant of the amino acid sequence of amino acid residues 20-224 of SEQ ID NO:5 is encoded by a nucleic acid molecule which hybridizes to the complement of SEQ ID NO:6 under stringent wash conditions, (ii) said naturally occurring allelic variant of the amino arid sequence of SEQ ID NO:8 is encoded by a nucleic acid molecule which hybridizes to the complement of SEQ ID NO:9 under stringent wash conditions, and wherein the protein encoded by the nucleic acid is at least 85 % homologous to SEQ ID NO:8, and (iii) said naturally occurring allelic variant of the amino acid sequence of SEQ ID NO:14 is encoded by a nucleic acid molecule which hybridizes to the complement of SEQ ID NO:15 under stringent wash conditions, and wherein stringent wash conditions are 0.2X SSC, 0.1% SDS at 50-65°C;
c) a polypeptide comprising amino acid residues 24-350 of SEQ ID NO:2; and
d) a polypeptide comprising an amino acid sequence which has at least 80% amino acid sequence identity with the amino acid sequence of SEQ ID NO:5 or SEQ ID NO:11 over the whole length of SEQ ID NO:5 or SEQ ID NO:11, respectively, or a polypeptide comprising an amino acid sequence which has at least 85% amino acid sequence identity with the amino acid sequence of SEQ ID NO:8 over the whole length of SEQ ID NO:8, wherein the polypeptide modulates cellular signal transduction or cellular proliferation.

Further provided is an antibody or immunologically active portion thereof which specifically binds to a polypeptide selected from the group consisting of:
a) a polypeptide consisting of 15 contiguous amino acids of SEQ ID NO:5, SEQ ID NO: 11 or SEQ ID NO:14 or a polypeptide consisting of 30 contiguous amino acids of SEQ ID NO:8; and
b) a polypeptide consisting of a naturally occurring allelic variant of the amino acid sequence of amino acid residues 20-224 of SEQ ID NO:5. amino acid residues 21-266 of SEQ ID NO:8, SEQ ID NO:11 or SEQ ID NO:14, wherein said naturally occurring allelic variant modulates cellular signal transduction or cellular proliferation and wherein (i) said naturally occurring allelic variant of the amino acid sequence of amino acid residues 20-224 of SEQ ID NO:5 is encoded by a nucleic acid molecule which hybridizes to the complement of SEQ ID NO:6 under stringent wash conditions, (ii) said naturally occurring allelic variant of the amino acid sequence of amino acid residues 21-266 of SEQ ID NO:8 is encoded by a nucleic acid molecule which hybridizes to the complement of SEQ ID NO:9 under stringent wash conditions, (iii) said naturally occurring allelic variant of the amino acid sequence of SEQ ID NO:11 is encoded by a nucleic acid molecule which hybridizes to the complement of SEQ ID NO:12 under stringent wash conditions, (iv) said naturally occurring allelic variant of the amino acid sequence of SEQ ID NO: 14 is encoded by a nucleic acid molecule which hybridizes to the complement of SEQ ID NO: 15 under stringent wash conditions, and wherein stringent wash conditions are 0.2X SSC, 0.1 % SDS at 50-65°C.

The present invention further provides a method for producing a polypeptide as defined above comprising culturing the host cell as defined above under conditions in which the recombinant expression vector is expressed, and said polypeptide is produced.

In a related aspect the present invention provides a method for detecting the presence of a polypeptide as described above In a sample comprising:
a) contacting the sample with an antibody or immunologically active portion thereof as described above; and
b) determining whether said antibody binds to a polypeptide in the sample to thereby detect the presence of said polypeptide as described above in the sample.

In an alternative embodiment the invention provides a kit comprising an antibody or immunologically active portion thereof as described above and instructions for use.

In another aspect, the invention provides the use of a kit for detecting a polypeptide as described above in a sample, said kit comprising an antibody or immunologically active portion thereof as described above and instructions for use.

The invention further provides a method for detecting the presence of a nucleic acid molecule of as defined above in a sample comprising:
a) contacting the sample with a nucleic acid probe or primer which specifically hybridizes to a nucleic acid molecule as defined above; and
b) determining whether the nucleic acid probe or primer binds to a nucleic acid molecule in the sample to thereby detect the presence of a nucleic acid molecule as defined above in the sample.

The invention further provides the use of a kit for detecting a nucleic acid molecule as defined above in a sample, said kit comprising a nucleic acid probe or primer which specifically hybridizes to a nucleic acid molecule as defined above and instructions for use.

The invention further provides an in vitro method for identifying a compound which binds to a polypeptide as defined above comprising:
a) contacting a polypeptide as defined above, or a cell expressing said polypeptide with a test compound; and
b) determining whether the polypeptide binds to the test compound.

The present invention also provides an in vitro method of modulating the cellular signal transduction or cellular proliferation activity of a polypeptide as defined above comprising contacting a polypeptide as defined above or a cell expressing said polypeptide with an antibody or immunologically active portion thereof as defined above in a sufficient concentration to modulate the cellular signal transduction or cellular proliferation activity of said polypeptide.

In a further embodiment the invention provides a method for identifying a compound which modulates the cellular signal transduction or cellular proliferation activity of a polypeptide as defined above comprising:
a) contacting a polypeptide as defined above with a test compound; and
b) determining the effect of the test compound on the cellular signal transduction or cellular proliferation activity of said polypeptide to thereby identify a compound which modulates the cellular signal transduction or cellular proliferation activity of said polypeptide.

The invention also provides the use of an antibody or immunologically active portion thereof as defined above for the manufacture of a medicament for treatment of a developmental, differentiative, or proliferative disorder.

In a different aspect, the invention relates to a polypeptide as defined above for use in therapy or diagnosis.

Accordingly the invention provides the use of a polypeptide as defined above for the manufacture of a medicament for treatment of a developmental, differentiative, or proliferative disorder.

In another aspect the invention provides a nucleic acid molecule as defined above for use in therapy or diagnosis.

In accordance with this aspect, the invention provides the use of a nucleic acid molecule as defined above for the manufacture of a medicament for treatment of a developmental, differentiative, or proliferative disorder.

Alternatively, the present invention further provides the use of an antibody or immunologically active portion thereof as described above for the manufacture of a medicament or diagnostic for the treatment, prophylaxis or diagnosis of cancer.

A further aspect of the present invention is the use of a nucleic acid molecule as described above for the manufacture of a medicament or diagnostic for the treatment, prophylaxis or diagnosis of cancer.

The present invention also provides a non-human host cell comprising a transgene that comprises the nucleic acid molecule as defined above.

### Brief Description of the Drawings

*Figure 1* depicts the cDNA sequence and predicted amino acid sequence of human CRSP-1. The nucleotide sequence corresponds to nucleic acids 1 to 2479 of SEQ ID NO:1. The amino acid sequence corresponds to amino acids 1 to 350 of SEQ ID NO:2.
*Figure 2* depicts the cDNA sequence and predicted amino acid sequence of human CRSP-2. The nucleotide sequence corresponds to nucleic acids 1 to 848 of SEQ ID NO:4. The amino acid sequence corresponds to amino acids 1 to 224 of SEQ ID NO:5.
*Figure 3* depicts the cDNA sequence and predicted amino acid sequence of human CRSP-3. The nucleotide sequence corresponds to nucleic acids 1 to 1529 of SEQ ID NO:7. The amino acid sequence corresponds to amino acids 1 to 266 of SEQ ID NO:8.
*Figure 4* depicts the cDNA sequence and predicted amino acid sequence of human CRSP-4. The nucleotide sequence corresponds to nucleic acids 1 to 702 of SEQ ID NO:10. The amino acid sequence corresponds to amino acids 1 to 179 of SEQ ID NO:11.
*Figure 5* depicts the cDNA sequence and predicted amino acid sequence of human CRSP-like-N. The nucleotide sequence corresponds to nucleic acids 1 to 928 of SEQ ID NO: 13. The amino acid sequence corresponds to amino acids 1 to 242 of SEQ ID NO: 14.
*Figure 6* is depicts the amino acid sequences of human CRSP- 1, human CRSP-2, human CRSP-3, and human CRSP-4, as well as the consensus sequence generated form the alignment of these sequences. The figure details the relationship between the CRSP proteins of the instant invention.
*Figure 7* is a schematic diagram depicting the the relationship between the CRSP proteins of the instant invention. The figure depicts the biological and functional domains of human CRSP. Signal peptides are indicated by filled boxes. The cysteine-rich domains of a CRSP cysteine-rich region are depicted as CRD-1 and CRD-2.
*Figure 8* depicts the cDNA sequence and predicted amino acid sequence of murine CRSP-1. The nucleotide sequence corresponds to nucleic acids 1 to 928 of SEQ ID N0:16. The amino acid sequence corresponds to amino acids 1 to 349 of SEQ ID N0:17.
*Figure 9* is a schematic diagram depicting the relationship between the CRSP-1 nucleotide sequence and those of RIG and RIG-like 7-1 (Accession Nos. U32331 and AF034208, respectively).

### Detailed Description of the Invention

The present invention is based on the discovery of novel molecules, referred to herein as CRSP protein and nucleic acid molecules, which comprise a family of molecules having certain conserved structural and functional features.

The term 'family' when referring to the protein and nucleic acid molecules of the invention is intended to mean two or more proteins or nucleic acid molecules having a common structural domain end having sufficient amino add or nucleotide sequence homology as defined herein. Such family members can be naturally occurring and can be from either the same or different species. For example, a family can contain a first protein of human origin, as well as other, distinct proteins of human origin or alternatively, can contain homologues of non-human origin. Members of a family may also have common functional characteristics

A CRSP family member is identified based on the presence of at least one "cysteine-rich domain' In the protein or corresponding nucleic acid molecule. As defined herein, a "cysteine-rich domain" refers to a portion of a CRSP protein (e.g., CRSP-1) which is rich in cysteine residues, i.e., at least about 6, 7, 8, 9, 10, 11, 12, 13, 14, 15, 16, 17, 18, 19, or 20 amino acids are cysteine residues. Preferably, a "cysteine-rich domain" is a protein domain having an amino acid sequence of about 30-100 amino acids, preferably about 35-95 amino acids, more preferably about 40-90 amino acids, more preferably about 45-85 amino adds, even more preferably about 50-80 amino acids, and even more preferably about 55-75, 60-70, or 65 amino acids, of which at least about 3-20, preferably about 5-15, or more preferably about 6, 7, 8, 9, 10, 11, 12, 13, 14, 15, 16, 17, 18, 19, or 20 amino adds are cysteine residues.

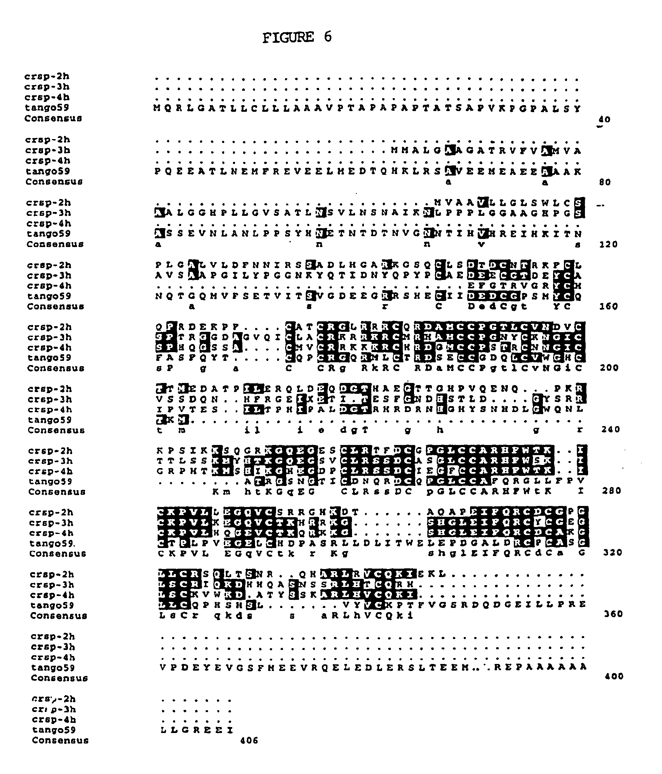

A preferred cysteine-rich domain has at least about 10, at least about 15, 16, 17, 18, 19, or preferably 20 cysteine residues located at the same relative amino acid position as the cysteine residues in human CRSP-1 having SEQ ID NO:2. For example, as shown in Figure 6, CRSP-2 has at least about 10 cysteine residues located at the same relative amino acid position as the cysteine residues in human CRSP-1 having SEQ ID NO:2 (e.g., cys151 in CRSP-2, SEQ ID NO:5, is located at the same relative amino acid position as cys214 in CRSP-1, SEQ ID NO:2; cys156 in CRSP-2, SEQ ID NO: 5, is located at the same relative amino add position as cys219 in CRSP-1, SEQ ID NO:2; and cys157 In CRSP-2, SEQ ID NO:5. is located at the same relative amino acid position as cys220 in CRSP-1, SEQ ID NO:2). Similarly, as shown in Figure 6, CRSP-3 has at least about 10 cysteine residues located at the same relative amino acid position as the cysteine residues in human CRSP-1 having SEQ ID NO:2. As also shown in Figure 6, CRSP-4 has at least about 10 cysteine residues located at the same relative amino acid position as the cysteine residues in human CRSP-1 having SEQ ID NO:2.

A preferred CRSP protein is a human protein (e.g., encoded by a nucleotide sequence corresponding to a naturally-occurring human gene.) For example, in one embodiment, a CASP-1 protein (human CRSP-1) contains a first cysteine-rich domain containing about amino acids 147-195 of SEQ ID NO:2, having 10 cysteine residues, and a second cysteine-rich domain containing about amino acids 201-284 of SEQ ID NO:2, having 10 cysteine residues (the positions of the cysteine residues are depicted in Figure 6). In another embodiment, a CRSP-2 (human CRSP-2) protein contains a first cysteine-rich domain containing about amino acids 41-90 of SEQ ID NO:5, having 10 cysteine residues, and a second cysteine-rich domain containing about amino acids 41-218 of SEQ ID NO:5, having 10 cysteine residues (the positions of the cysteine residues are depicted In Figure 6). In another embodiment, a CRSP-3 protein (human CRSP-3) contains a first cysteine-rich domain containing about amino acids 85-138 of SEQ 10 NO:8, having 10 cysteine residues, and a second cysteine-rich domain containing about amino acids 182-263 of SEQ ID NO: 8, having 10 cysteine residues (the positions of the cysteine residues are depicted in Figure 6). In another embodiment, a CRSP-4 protein (human CRSP-4) contains a first cysteine-rich domain containing about amino acids 1-47 of SEQ ID NO: 11, having 8 cysteine residues, and a second cysteine-rich domain containing about amino acids 96-176 of SEQ ID NO: 11, having 10 cysteine residues (the positions of the cysteine residues are depicted in Figure 6).

Preferred CRSP proteins have more than one cysteine-rich domains, more preferably have at least two cysteine-rich domains and, thus, have a cysteine-rich region. As used herein, the term "cysteine-rich region" refers to a protein domain which includes at least two cystelne-rich domains and has an amino acid sequence of about 120-200, preferably about 130-190, more preferably about 140-180 amino acid residues, and even more preferably at least about 135-175 amino acids of which at least about 10-30, preferably about 15-20, and more preferably about 16, 17, 18, or 19 of the amino acids are cysteine residues. In a preferred embodiment, a cysteine-rich region is located in the C-terminal region of a CRSP protein. For example, In one embodiment, a CRSP-1 protein contains a cysteine rich region containing about amino acids 147-284 of SEQ ID NO:2, having 20 cysteine residues at the positions indicated in Figure 6. In another embodiment, a CRSP-2 protein contains a cysteine rich region containing about amino acids 41-218 of SEQ ID NO:5, having 20 cysteine residues at the positions indicated in Figure 6. In another embodiment, a CRSP-3 protein contains a cysteine rich region containing about amino acids 85-263 of SEQ ID NO:8, having 20 cysteine residues at the positions Indicated in Figure 6. In another embodiment, a CRSP-4 protein contains a cysteine rich region containing about amino adds 1-176 of SEQ ID NO:2, having 18 cysteine residues at the positions indicated in Figure 6.

In addition to cysteine-rich domains, the cysteine-rich region may contain a spacer region which separates the first and second cysteine-rich domains. As used herein, the "spacer region" refers to amino acid residues which are located between the first and second cysteine-rich domains of a cysteine-rich region and includes amino acid residues located C-terminal to the first cysteine-rich domain and N-terminal to the second cysteine-rich domain. As defined herein, a "spacer region" refers to a protein domain of about 5-70 amino acids, preferably about 10-65 amino acids, more preferably about 15-60 amino acids, even more preferably about 20-55 amino acids, and even more preferably about 25-50, 30-45 or 35-40 amino acids. For example, CRSP-1 protein contains a spacer region of about amino acids 196-200 of SEQ ID NO:2; CRSP-2 protein contains a spacer region of about amino acids 91-137 of SEQ ID NO:5; CRSP-3 protein contains a spacer region of about amino acids 139-181 of SEQ ID NO:8; and CRSP-4 protein contains a spacer region of about amino acids 48-95 of SEQ ID NO: 11.

In another embodiment of the invention, the CRSP protein has at least one cysteine-rich domain, preferably a cysteine-rich region, and a signal sequence. As used herein, a "signal sequence" refers to a peptide containing about 20 amino acids which occurs at the N-terminus of secretory and integral membrane proteins and which contains a large number of hydrophobic amino acid residues. For example, a signal sequence contains at least about 14-28 amino acid residues, preferably about 16-26 amino acid residues, more preferably about 18-24 amino acid residues, and more preferably about 20-22 amino acid residues, and has at least about 40-70%, preferably about 50-65%, and more preferably about 55-60% hydrophobic amino acid residues (e.g., Alanine, Valine, Leucine, Isoleucine, Phenylalanine, Tyrosine, Tryptophan, or Proline). Such a "signal sequence", also referred to in the art as a "signal peptide", serves to direct a protein containing such a sequence to a lipid bilayer. For example, In one embodiment, a CRSP-1 protein contains a signal sequence of about amino acids 1-23 of SEQ ID NO:2. In another embodiment, a CRSP-2 protein contains a signal sequence of about amino adds 1-19 of SEQ ID NO:5. In another embodiment, a CRSP-3 protein contains a signal sequence of about amino adds 1-20 of SEQ ID NO:8.

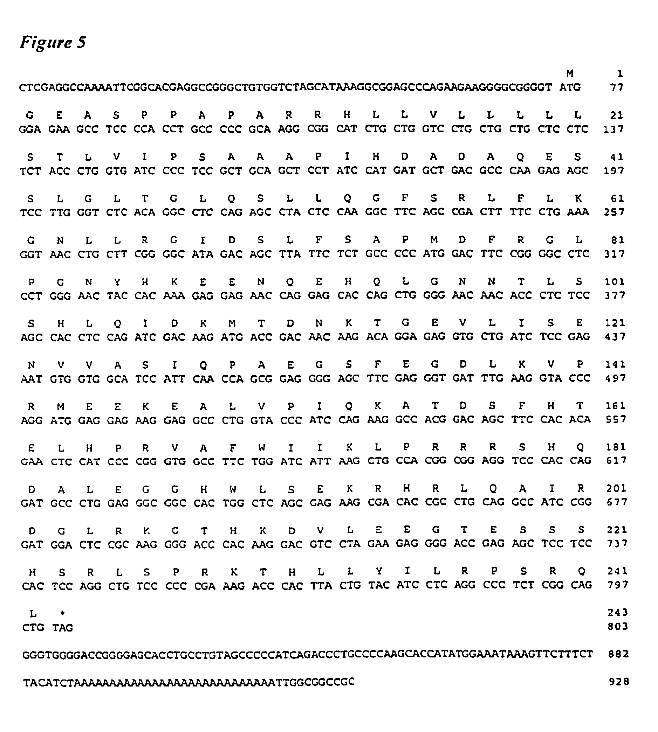

CRSP proteins of the present invention can be used to identify additional CRSP family members. For example, a protein having homology to CRSP-1 was identified using the nucleotide sequence encoding the N-terminal unique region of CRSP-1 to search a protein sequence database. Such a protein, referred to herein as "CRSP-like-N" is depicted in Figure 5. The nucleotide sequence of CRSP-like-N (SEQ ID NO:13) encodes a protein having 242 amino adds (SEQ ID NO:14). The nucleotide sequence of CRSP-like-N includes a 5' untranslated region containing nucleotides 1-74 of SEQ ID NO:13, a coding region containing nucleotides 75-800 of SEQ ID NO:13 (corresponding to nucleotides 1-726 of SEQ ID NO: 15), and a 3' untranslated region containing nucleotides 801-928 of SEQ ID NO:13.

Yet another embodiment of the invention features a CRSP protein as described above having at least one cysteine-rich domain, preferably at least one cysteine-rich region and a signal peptide. Another embodiment features a CRSP protein having at least one cysteine-rich domain, preferably at least one cysteine-rich region and a signal peptide, wherein the cysteine-rich region incudes at least two cysteine-rich domains. Another embodiment features a CRSP protein having at least one cysteine-rich domain, preferably at least one cysteine-rich region and a signal peptide, wherein the cysteine-rich region includes at least two cysteine-rich domains and a spacer.

Preferred CRSP molecules of the present invention are defined in the attached claims. As used herein, the term "sufficiently homologous" refers to a first amino acid or nucleotide sequence which contains a sufficient or minimum number of identical or equivalent (e.g., an amino acid residue which has a similar side chain) amino acid residues or nucleotides to a second amino acid or nucleotide sequence such that the first and second amino acid or nucleotide sequences share common structural domains and/or acommon functional activity. For example, amino acid or nucleotide sequences which share common structural domains have at least about 40% homology, preferably 50% homology, more preferably 60%-70% homology across the amino acid sequences of the domains and contain at least one, preferably two, more preferably three, and even more preferably four, five or six structural domains, are defined herein as sufficiently homologous. Furthermore, amino acid or nucleotide sequences which share at least 40%, preferably 50%, more preferably 60, 70, or 80% homology and share a common functional activity are defined herein as sufficiently homologous.

As used interchangeably herein, a "CRSP activity", "biological activity of CRSP" or "functional activity of CRSP", refers to an activity exerted by a CRSP protein, polypeptide or nucleic acid molecule on a CRSP responsive cell as determined *in vivo,* or *in vitro,* according to standard techniques. In one embodiment a CRSP activity is a direct activity, such as an association with a CRSP-target molecule. As used herein, a "target molecule' is a molecule with which a CRSP protein binds or interacts in nature, such that CRSP-mediated function is acheived A CRSP target molecule can be a non-CRSP molecule or a CRSP protein or polypeptide of the present invention. In an exemplary embodiment, a CRSP target molecule is a membrane-bound protein (e.g., a cell-surface receptor or "CRSP receptor) or a modified form of such a protein which has been altered such that the protein is soluble (e.g., recombinantly produced such that the protein does not express a membrane-binding domain). In another embodiment, a CRSP target is a second soluble protein molecule (e.g., a "CRSP binding partner" or "CRSP substrate"). In such an exemplary embodiment, a CRSP binding partner can be a second soluble non-CRSP protein or a second CRSP protein.molecule of the present invention. Alternatively, a CRSP activity is an indirect activity, such as a cellular signaling activity mediated by interaction of the-CRSP protein with a second protein (e.g., a CRSP receptor). As used herein, the term "CRSP receptor" refers to a protein or protein complex, to which a CRSP protein, e.g., human CRSP, can bind. A receptor can be a cell surface receptor, e.g., a nuclear hormone receptor. CRSP receptors can be isolated by methods known in the art and further described herein. Interaction of a CRSP protein with a CRSP receptor can result in transduction of a signal from the cell surface to the nucleus. The signal transduced can be, an increase in intracellular calcium, an increase in phosphatidylinositol or other molecule, and can result, e.g., in phosphorylation of specific proteins, a modulation of gene transcription and any of the other biological activities set forth herein.

In a preferred embodiment, a CRSP activity is at least one or more of the following activities: (i) interaction of a CRSP protein with and/or binding to a second molecule, (e.g., a protein, such as a CRSP receptor, a soluble form of a CRSP receptor, a receptor for a member of the *wnt* family of signaling proteins, or a non-CRSP signaling molecule); (ii) interaction of a CRSP protein with an intracellular protein via a membrane-bound CRSP receptor; (iii) complex formation between a soluble CRSP protein and a second soluble CRSP binding partner (e.g., a non-CRSP protein molecule or a second CRSP protein molecule); (iv) interaction with other extracellular proteins (e.g., regulation of *wnt*-dependent cellular adhesion to extracellular matrix components); (v) binding to and eliminating an undesirable molecule (e.g., a detoxifying activity or defense function); and/or (vi) an enzymatic activity. In yet another preferred embodiment, a CRSP activity is at least one or more of the following activities: (1) modulation of cellular signal transduction, either *in vitro or in vivo* (e.g., antagonism of the activity of members of the *wnt* family of secreted proteins or supression of *wnt-*dependent signal transduction); (2) regulation of communication between cells (e.g., regulation of wnt-dependent cell-cell interactions); (3) regulation of expression of genes whose expression is modulated by binding of CRSP (e.g., CRSP-1) to a receptor; (4) regulation of gene transcription in a cell involved in development or differentiation, either *in vitro or in vivo* (e.g., induction of cellular differentiation); (5) regulation of gene transcription in a cell involved in development or differentiation, wherein at least one gene encodes a differentiation-specific protein; (6) regulation of gene transcription in a cell involved in development or differentaition, wherein at least one gene encodes a second secreted protein; (7) regulation of gene transcription in a cell involved in development or differentiation, wherein at least one gene encodes a signal transduction molecule; (8) regulation of cellular proliferation, either *in vitro or in vivo* (e.g., induction of cellular proliferation or inhibition of proliferation as in the case of supression of tumorogenesis (e.g., supression of glioblastoma formation)); (9) formation and maintenance of ordered spatial arrangements of differentiated tissues in vertebrates, both adult and embryonic (e.g., induction of head formation during vertebrate development or maintenance of hematopoietic progenitor cells); (10) modulation of cell death, such as stimulation of cell survival; (11) regulating cell migration; and/or (12) immune modulation.

As referred to herein, "differentiation-specific proteins" include proteins involved in the transition of a cell from the undifferentiated to the differentiated phenotype. For example, such proteins can be differentiation specific structural proteins or differentiation-specific transcription factors. Such differentiation-specif proteins are generally expressed at higher levels in cells which are making the transition from the undifferentiated to the differentiated phenotype (e.g., during embryonic development or during regeneration of mature tissue in the adult animal), or are expresed at higher levels in fully-differentiated or terminally-differentiated cells as compared to their undifferentiated counterparts. Also, as referred to herein, "differentiation-specific genes" include nucleic acid molecules which encode differentiation-specific proteins.

Accordingly, another embodiment of the invention features isolated CRSP proteins and polypeptides having a CRSP activity. Preferred CRSP proteins have at least one cysteine-rich region and a CRSP activity. In another preferred embodiment, the CRSP protein has at least one cysteine-rich region, wherein the cysteine-rich region comprises at least one cysteine-rich domain, and a CRSP activity. In another preferred embodiment, the CRSP protein has at least one cysteine-rich region, wherein the cysteine-rich region comprises at least two cysteine-rich domains, and a CRSP activity. In yet another preferred embodiment, a CRSP protein further comprises a signal sequence. In still another preferred embodiment, a CRSP protein has a cysteine-rich region, a CRSP activity, and an amino acid sequence as defined in the attached claims.

The human CRSP-1 cDNA, which is approximately 2479 nucleotides in length, encodes a protein which is approximately 350 amino acid residues in length. The human CRSP protein contains an N-terminal signal sequence and a cysteine-rich region comprising two cysteine-rich domains. A CRSP cysteine-rich region can be found at least, for example, from about amino acids 147-284 of SEQ ID NO:2. The CRSP-1 cysteine-rich region comprises a first cysteine-rich domain from about amino acids 147-195 of SEQ ID NO:2 and a second cysteine-rich domain from about amino acids 201-284 of SEQ ID NO:2. The human CRSP-1 protein is a secreted protein which further contains a signal sequence at about amino acids 1-23 of SEQ ID NO:2. The prediction of such a signal peptide can be made, for example, utilizing the computer algorithm SIGNALP (Henrik, et al. (1997) Protein Engineering 10:1-6).

The human CRSP-2 cDNA, which is approximately 848 nucleotides in length, encodes a protein which is approximately 224 amino acid residues in length. The human CRSP protein contains an N-terminal signal sequence and a cysteine-rich region comprising two cysteine-rich domains. A CRSP cysteine-rich region can be found at least, for example, from about amino acids 41-218 of SEQ ID NO:5. The CRSP-2 cysteine-rich region comprises a first cysteine-rich domain from about amino acids 41-90 of SEQ ID NO:5 and a second cysteine-rich domain from about amino acids 138-218 of SEQ ID NO:5. The human CRSP-2 protein is a secreted protein which further contains a signal sequence at about amino acids 1-19 of SEQ ID NO:5.

The human CRSP-3 cDNA, which is approximately 1529 nucleotides in length, encodes a protein which Is approximately 266 amino acid residues In length. The human CRSP protein contains an N-termirrai signal sequence and a cysteine-rich region comprising two cysteine-rich domains. A CRSP cysteine-rich region can be found at least, for example, from about amino acids 85-263 of SEQ ID NO:8. The CRSP.3 cysteine-rich region comprises a first cysteine-rich domain from about amino acids 85-138 of SEO ID NO:8 and a second cysteine-rich domain from about amino acids 182-263 of SEQ ID NO:2. The human CRSP-3 protein is a secreted protein which further contains a signal sequence at about amino acids 1-20 of SEQ ID NO:8.

The human CRSP-4 cDNA, which is approximately 702 nucleotides in length, encodes a protein which is approximately 179 amino acid residues in length. The human CRSP protein contains a cysteine-rich region comprising two cysteine-rich domains. A CRSP cysteine-rich region can be found at least, for example, from about amino acids 1-178 of SEQ ID NO:11. The CRSP-4 cysteine-rich region comprises a first cysteine-rich domain from about amino adds 1-47 of SEQ ID NO:11 and a second cysteine-rich domain from about amino acids 98-176 of SEQ ID NO:11.

Various aspects of the invention are described in further detail in the following subsections:

### I. Isolated Nucleic Acid Molecules

One aspect of the invention pertains to isolated nucleic acid molecules as defined in the attached claims that encode CASP proteins or biologically active portions thereof. As used herein, the term 'nucleic acid molecule' is intended to include DNA molecules (e.g., cDNA or genomic DNA) and RNA molecules (e.g., mRNA) and analogs of the DNA or RNA generated using nucleotide analogs. The nucleic acid molecule can be single-stranded or double-stranded, but preferably is double-stranded DNA.

An 'isolated' nucleic add molecule is one which is separated from other nucleic add molecules which are present in the natural source of the nucleic acid. Preferably, an "isolated" nucleic acid is free of sequences which naturally frank the nucleic acid (i.e.. sequences located at the 5' and 3' ends of the nucleic acid) in the genomic DNA of the organism from which the nucleic acid is derived. For example, In various embodiments, the isolated CRSP nucleic acid molecule can contain less than about 5 kb, 4kb, 3kb, 2kb, 1 kb, 0.5 kb or 0.1 kb of nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material, or culture medium when produced by recombinant techniques, or substantially free of chemical precursors or other chemicals when chemically synthesized.

A nucleic add molecule the present invention e.g., a nucleic acid molecule having the nucleotide sequence as defined in the attached claims, can be isolated using standard molecular biology techniques and the sequence information provided herein. Using all or portion of the nucleic acid sequence of SEQ 10 N0:1, SEQ ID NO:4, SEQ ID NO:7, or SEQ ID NO:10, or the nucleotide sequence of the DNA insert of the plasmid deposited with ATCC as Accession Number 98634, the nucleotide sequence of the DNA insert of the plasmid deposited with ATCC as Accession Number 98633. or the nucleotide sequence of the DNA insert of the plasmid deposited with ATCC as Accession Number 98452 as a hybridization probe, CRSP nucleic acid molecules can be isolated using standard hybridization and cloning techniques (e.g., as described in Sambrook, J., Fritsh, E. F., and Maniatis, T. Molecular Cloning: A Laboratory Manual. 2nd,ed., Cold Spring Harbor Laboratory, Cold Spring Harbor Laboratory Press, Cold Spring Harbor, NY, 1989).

Moreover, a nucleic acid molecule encompassing all or a portion of SEQ ID NO:1, 1, sea ID NO:4, SEQ ID NO: 7, or SEO ID NO:10, or the nucleotide sequence of the DNA insert of the plasmid deposited with ATCC as Accession Number 98634, the nucleotide sequence of the DNA insert of the plasmid deposited with ATCC as Accession Number 98633, or the nucleotide sequence of the DNA insert of the plasmid deposited with ATCC as Accession Number 98452 can be isolated by the polymerase chain reaction (PCR) using synthetic oligonucleotide primers designed based upon the sequence of SEQ ID NO:1, SEQ ID NO:4, SEQ ID NO:7, or SEQ ID NO:10, or the nucleotide sequence of the DNA insert of the plasmid deposited with ATCC as Accession Number 98634, the nucleotide sequence of the DNA insert of the plasmid deposited with ATCC as Accession Number 98633, or the nucleotide sequence of the DNA insert of the plasmid deposited with ATCC as Accession Number 98452.

A nucleic acid of the invention can be amplified using cDNA, mRNA or alternatively, genomic DNA, as a template and appropriate oligonucleotide primers according to standard PCR amplification techniques. The nucleic acid so amplified can be cloned into an appropriate vector and characterized by DNA sequence analysis. Furthermore, oligonucleotides corresponding to CRSP nucleotide sequences can be prepared by standard synthetic techniques, e.g., using an automated DNA synthesizer.

In a preferred embodiment, an isolated nucleic acid molecule of the invention comprises a nucleotide sequence as defined in the claims.

The sequence of SEQ ID NO:1 corresponds to the human CRSP-1 cDNA. This cDNA comprises sequences encoding the human CRSP-1 protein (i.e., "the coding region", from nucleotides 38-1087), as well as 5' untranslated sequences (nucleotides I to 37) and 3' untranslated sequences (nucleotides 1088 to 2479). Alternatively, the nucleic add molecule can comprise only the coding region of SEQ 10 NO:1, (e.g., nucleotides 38 to 1087, corresponding to SEQ ID NO:3). A plasmid containing the full-length nucleotide sequence encoding CRSP-1 was deposited with the - American Type Culture Collection (ATCC), Manassas, Virginia, U.S.A. 20110-2209 on June 11, 1997 and assigned Accession Number 98452. A plasmid containing the full-length nucleotide sequence encoding CRSP-1 was deposited with the American Type Culture Collection (ATCC), Manassas, Virginia, U.S.A. 20110-2209 on January 16, 1998 and assigned Accession Number 98634. -

The sequence of SEQ ID NO:4 corresponds to the human CRSP-2 cDNA. This cDNA comprises sequences encoding the human CRSP-2 protein (i.e., "the coding region", from nucleotides 126-796), as well as 5' untranslated sequences (nucleotides 1 to 125) and 3' untranslated sequences (nucleotides 797 to 848). Alternatively, the nucleic acid molecule can comprise only the coding region of SEQ ID NO:4 (e.g., nucleotides 126 to 796, corresponding to SEQ ID NO:6).

SEQ ID NO:7 corresponds to the human CRSP-3 cDNA. This cDNA comprises sequences encoding the human CRSP-3 protein (i.e., "the coding region", from nucleotides 93-890), as well as 5' untranslated sequences (nucleotides 1 to 92) and 3' untranslated sequences (nucleotides 891 to 1529). Alternatively, the nucleic acid molecule can comprise only the coding region of SEQ ID NO:7 (e.g., nucleotides 93 to 890 corresponding to SEQ ID NO:9). A plasmid containing the full-length nucleotide sequence encoding CRSP-3 was deposited with the American Type Culture Collection (ATCC), Manassas, Virginia, U.S.A. 20110-2209. on January 16, 1998 and assigned Accession Number 98633.

The sequence of SEQ ID NO:10 corresponds to the human CRSP-4 cDNA. This cDNA comprises sequences encoding the human CRSP-4 protein (i.e., "the coding region", from nucleotides 1-537), as well as 3'untranslated sequences (nucleotides 538 to 702). Alternatively, the nucleic acid molecule can comprise only the coding region of SEO ID NO: 10 (e.g., nucleotides 1 to 537, corresponding to SEQ ID NO:12).

In another preferred embodiment, an isolated nucleic add molecule of the invention comprises a nucleic acid molecule which is a complement of the nucleotide sequence claimed. A nucleic acid molecule which is complementary to the nucleic sequence claimed is one which is sufficiently complementary to the nucleotide sequence claimed such that It can hybridize to the nucleotide sequence claimed thereby forming a stable duplex.

Moreover, a useful nucleic acid molecule can comprise only a portion of the nucleic acid sequence of SEQ ID NO:1, SEQ ID NO:4, SEQ ID NO:7, SEQ ID NO:10, the nucleotide sequence of the DNA insert of the plasmid deposited with ATCC as Accession Number 98634, the nucleotide sequence of the DNA insert of the plasmid deposited with ATCC as Accession Number 98633, or the nucleotide sequence of the DNA insert of the plasmid deposited with ATCC as Accession Number 98452, for example a fragment which can be used as a probe or primer or a fragment encoding a biologically active portion of a CRSP protein. The nucleotide sequence determined from the cloning of the human CRSP genes allows for the generation of probes and primers designed for use in identifying and/or cloning CRSP homologues in other cell types, e.g., from other tissues, as well as CRSP homologues from other mammals. The probe/primer typically comprises substantially purified oligonucleotide. The oligonucleotide typically comprises a region of nucleotide sequence that hybridizes under stringent conditions to at least about 12, preferably about 25, more preferably about 40, 50 or 75 consecutive nucleotides of a sense sequence of SEQ ID NO: 1, SEQ ID NO:4, SEQ ID NO:7, SEQ ID NO:10, the nucleotide sequence of the DNA insert of the plasmid deposited with ATCC as Accession Number 98634. the nucleotide sequence of the DNA insert of the plasmid deposited with ATCC as Accession Number 98633, or the nucleotide sequence of the DNA insert of the plasmid deposited with ATCC as Accession Number 98452, of an anti-sense sequence of SEQ ID NO:1, SEQ ID NO:4, SEQ ID NO:7, SEQ ID NO: 10, the nucleotide sequence of the DNA insert of the plasmid deposited with ATCC as Accession Number 98634, the nucleotide sequence of the DNA insert of the plasmid deposited with ATCC as Accession Number 98633, or the nucleotide sequence of the DNA insert of the plasmid deposited with ATCC as Accession Number 98452, or of a naturally occurring mutant of SEQ ID NO: 1, SEQ ID NO:4, SEQ ID NO:7, SEQ ID NO:10, the nucleotide sequence of the DNA insert of the plasmid deposited with ATCC as Accession Number 98634, the nucleotide sequence of the DNA insert of the plasmid deposited with ATCC as Accession Number 98633, or the nucleotide sequence of the DNA insert of the plasmid deposited with ATCC as Accession Number 98452. In an exemplary embodiment, a useful nucleic acid molecule comprises a nucleotide sequence which hybridizes under stringent hybridization conditions to a nucleic acid molecule comprising nucleotides 470-2479 of SEQ ID NO: 1 or to a nucleic add molecule comprising nucleotides 1-475 of SEQ ID NO:4.

Probes based on the human CRSP nucleotide sequence can be used to detect transcripts or genomic sequences encoding the same or homologous proteins. For instance primers based on the nucleic acid represented in SEQ ID NOS:1 or 3 can be used in PCR reactions to clone CRSP homologs (e.g., CRSP-1 homologues). In a preferred embodiment of the invention, CRSP homologs are cloned by PCR amplification (e.g., RT-PCR) using primers hybridizing to a portion of the nucleotide sequence encoding the CRSP cysteine rich domain. Likewise, probes based on the subject CRSP sequences can be used to detect transcripts or genomic sequences encoding the same or homologous proteins. In preferred embodiments, the probe further comprises a label group attached thereto, e.g., the label group can be a radioisotope, a fluorescent compound, an enzyme, or an enzyme co-factor. Such probes can be used as a part of a diagnostic test kit for identifying cells or tissue which misexpress a CRSP protein, such as by measuring a level of a CRSP-encoding nucleic acid In a sample of cells from a subject e.g., detecting CRSP mRNA levels or determining whether a genomic CRSP gene has been mutated or deleted.

A nucleic acid fragment encoding a "biologically active portion of a CRSP protein" can be prepared by isolating a portion of a sequence defined in the attached claims, which encodes a polypeptide having a CRSP biological activity (the biological activities of the CRSP proteins have previously been described), expressing the encoded portion of the CRSP protein (e.g., by recombinant expression *in vitro)* and assessing the activity of the encoded portion of the CRSP protein.

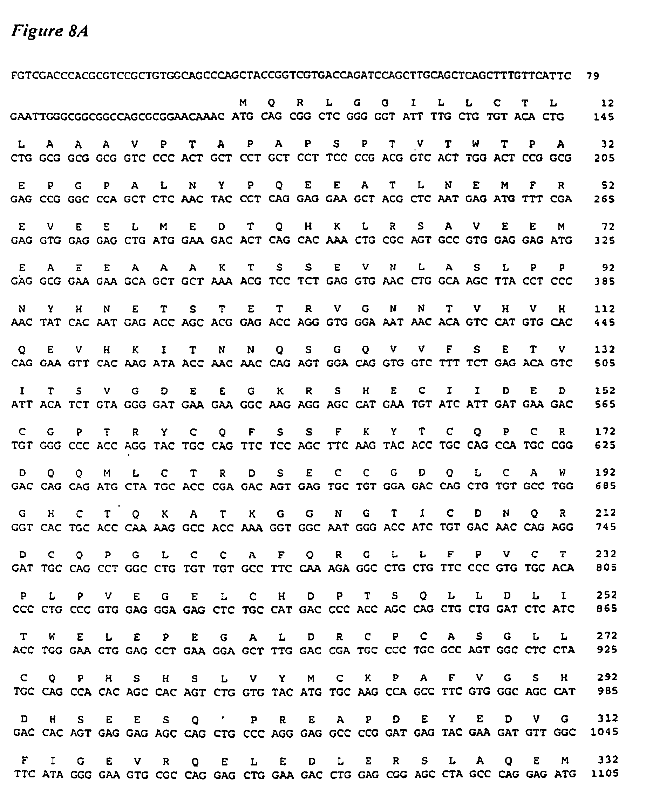

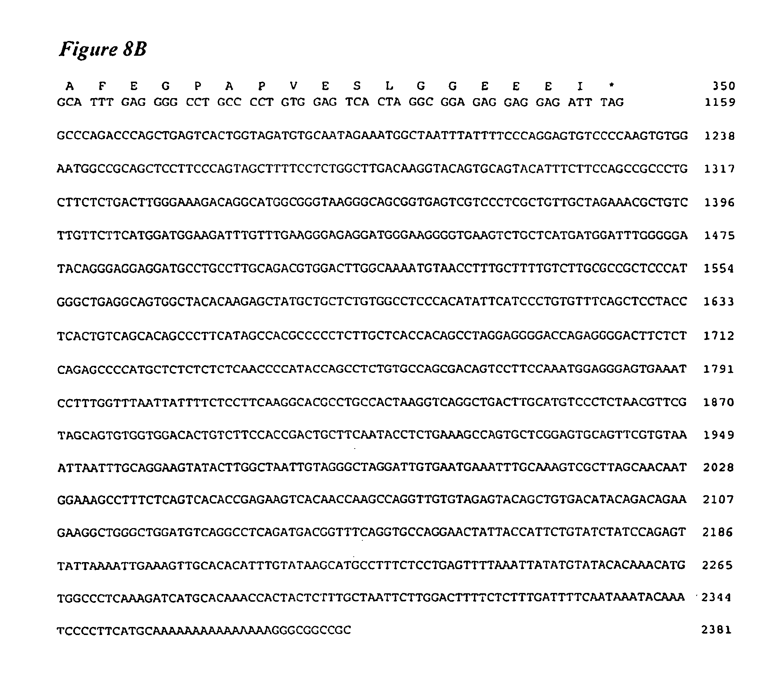

A murine CRSP-1 cDNA has been identified based of the nucleotide sequence of human CRSP-1. The nucleotide sequence of murine CRSP-1 (SEQ ID NO:16) encodes a CRSP-1 protein having 349 amino acids. The nucleotide and amino acid sequences of murine CRSP-1 are depicted in Figure 8. The coding region of murine CRSP- is represented by SEQ ID NO:18.

Nucleic acid molecules corresponding to natural allelic variants and homologues of the CRSP cDNAs of the invention can be isolated based on their homology to the human CRSP nucleic acids disclosed herein using the human cDNA, or a portion thereof, as a hybridization probe according to standard hybridization techniques under stringent hybridization conditions. Examples of tissues and/or libraries suitable for isolation of the subject nucleic acids include thymus, lymph nodes and inflammatory tissue. cDNA encoding a CRSP protein (e.g., a CRSP-) protein) can be obtained by Isolating total mRNA from a cell, e.g., a vertebrate cell, a mammalian cell, or a human cell, including embryonic cells. Double stranded cDNAs can then be prepared from the total mRNA, and subsequently inserted into a suitable plasmid or bacteriophage vector using any one of a number of known techniques. The gene encoding a CRSP-1 protein can also be cloned using established polymerase chain reaction techniques in accordance with the nucleotide sequence information provided by the invention. The nucleic acid of the invention can be DNA or RNA or analogs thereof.

Accordingly, an useful isolated nucleic acid molecule is at least 15 nucleotides in length and hybridizes under stringent conditions to the nucleic acid molecule comprising the nucleotide sequence of SEQ ID NO:1, SEQ ID NO:4, SEQ ID NO:7, SEQ ID NO:10, the nucleotide sequence of the DNA insert of the plasmid deposited with ATCC as Accession Number 98634, the nucleotide sequence of the DNA insert of the plasmid deposited with ATCC as Accession Number 98633, or the nucleotide sequence of the DNA insert of the plasmid deposited with ATCC as Accession Number 98452. In other embodiment, the nucleic acid is at least 30, 50, 100, 250 or 500 nucleotides in length. As used herein, the term "hybridizes under stringent conditions' is intended to describe conditions for hybridization and washing under which nucleotide sequences at least 60% homologous to each other typically remain hybridized to each other. Preferably, the conditions are such that sequences at least about 70%, more preferably at least about 80%, even more preferably at least about 85% or 90% homologous to each other typically remain hybridized to each other. Such stringent conditions are known to those skilled in the art and can be found in *Current Protocols in Molecular Biology,* John Wiley & Sons, N.Y. (1989), 6.3.1-6.3.6. A preferred, non-limiting example of stringent hybridization conditions are hybridization in 6X sodium chloride/sodium citrate (SSC) at about 45°C, followed by one or more washes in 0.2 X SSC, 0.1% SDS at 50-65°C. Preferably, an isolated nucleic acid molecule of the invention that hybridizes under stringent conditions to the sequence of SEQ ID NO:1 corresponds to a naturally-occurring nucleic acid molecule. As used herein, a "naturally-occurring' nucleic acid molecule refers to an RNA or DNA molecule having a nucleotide sequence that occurs In nature (e.g., encodes a natural protein).

In addition to naturally-occurring allelic variants of the CRSP sequences that may exist in the population, the skilled artisan will further appreciate that changes can be introduced by mutation into the nucleotide sequences of SEQ ID NO:1, SEQ ID NO:4, SEQ ID NO:7, SEQ ID NO:10, the nucleotide sequence of the DNA insert of the plasmid deposited with ATCC as Accession Number 98634, the nucleotide sequence of the DNA insert of the plasmid deposited with ATCC as Accession Number 98633, or the nucleotide sequence of the DNA insert of the plasmid deposited with ATCC as Accession Number 98452, thereby leading to changes in the amino acid sequence of the encoded CRSP proteins, without altering the functional ability of the CRSP proteins. For example, nucleotide substitutions leading to amino acid substitutions (particularly conservative amino acid substitutions) at "non-essential" amino acid residues can be made In the sequence of SEQ ID NO: 1, SEQ ID NO:4, SEQ ID NO:7, SEQ ID NO:10, the nucleotide sequence of the DNA Insert of the plasmid deposited with ATCC as Accession Number 98634, the nucleotide sequence of the DNA insert of the plasmid deposited with ATCC as Accession Number 98633, or the nucleotide sequence of the DNA insert of the plasmid deposited with ATCC as Accession Number 98452. A "non-essential" amino acid residue is a residue that can be altered from the wild-type sequence of CRSP (e.g., the sequence of SEQ ID NO:2, SEQ ID NO:5, SEQ ID NO:8, or SEQ ID NO:11) without altering the biological activity, whereas an "essential" amino acid residue is required for biological activity. For example, amino acid residues that are conserved among the CRSP proteins of the present invention (e.g., cysteine residues within cysteine-rich domains), are predicted to be particularly unamenable to alteration. Furthermore, amino add residues that are conserved between CRSP protein and other proteins having cysteine-rich domains are not likely to be amenable to alteration.

Accordingly, nucleic acid molecules can be generated encoding CRSP proteins that contain changes in amino acid residues that are not essential for activity. Such CRSP proteins differ in amino add sequence from SEQ ID NO:2, SEQ ID NO:5, SEQ ID NO:8, or SEQ ID NO:11 yet retain biological activity. A useful isolated nucleic acid molecule comprises a nucleotide sequence encoding a protein, wherein the protein comprises an amino acid sequence at least about 60% homologous to the amino acid sequence of SEQ ID NO:2, SEQ ID NO:5, SEQ ID NO:8, or SEQ ID NO:11. A useful protein encoded by the nucleic acid molecule is at least about 65-70% homologous to SEQ ID NO:2, SEQ ID NO:5, SEQ ID NO:8, or SEQ ID NO: 11, more preferably at least about 75-80% homologous to SEQ ID NO:2, SEQ ID NO:5, SEQ ID NO:8, or SEQ ID NO: 11, even more preferably at least about 85-90% homologous to SEQ ID NO:2, SEQ ID NO:5, SEQ ID NO:8, or SEQ ID NO:11, and most preferably at least about 95% homologous to SEQ ID NO:2, SEQ ID NO:5, SEQ ID NO:8, or SEQ ID NO:11.

An isolated nucleic acid molecule encoding a CRSP protein homologous to the protein of SEQ ID NO:2, SEQ ID NO : 5, SEQ ID NO:8, or SEQ ID NO:11 can be created by introducing one or more nucleotide substitutions, additions or deletions into the nucleotide sequence of SEQ ID NO:1, SEQ ID NO:4, SEQ ID NO:7, SEQ ID NO:10, the nucleotide sequence of the DNA insert of the plasmid deposited with ATCC as Accession Number 98634, the nucleotide sequence of the DNA insert of the plasmid deposited with ATCC as Accession Number 98633, or the nucleotide sequence of the DNA insert of the plasmid deposited with ATCC as Accession Number 98452, such that one or more amino acid substitutions, additions or deletions are introduced into the encoded protein. Mutations can be introduced into SEQ ID NO: 1, SEQ ID NO:4, SEQ ID NO:7, SEQ ID NO:10, the nucleotide sequence of the DNA insert of the plasmid deposited with ATCC as Accession Number 98634, the nucleotide sequence of the DNA insert of the plasmid deposited with ATCC as Accession Number 98633, or the nucleotide sequence of the DNA insert of the plasmid deposited with ATCC as Accession Number 98452, by standard techniques, such as site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a predicted nonessential amino acid residue in a CRSP protein (e.g., one not located in a cysteine-rich domain) is preferably replaced with another amino acid residue from the same side chain family. Alternatively, in another embodiment, mutations can be introduced randomly along all or part of a CRSP coding sequence, such as by saturation mutagenesis, and the resultant mutants can be screened for CRSP biological activity to identify mutants that retain activity. Following mutagenesis of SEQ ID NO: 1, SEQ ID NO:4, SEQ ID NO:7, SEQ ID NO:10, the nucleotide sequence of the DNA insert of the plasmid deposited with ATCC as Accession Number 98634, the nucleotide sequence of the DNA insert of the plasmid deposited with ATCC as Accession Number 98633, or the nucleotide sequence of the DNA insert of the plasmid deposited with ATCC as Accession Number 98452, the encoded protein can be expressed recombinantly and the activity of the protein can be determined.

A mutant CRSP protein can be assayed for intracellular calcium, an increase in phosphatidylinositol or other molecule, and can result, e.g., In phosphorylation of specific proteins, a modulation of gene transcription and any of the other biological activities set forth herein.

A mutant CRSP protein can also be assayed for the ability to (t) modulate cellular signal transduction, either *In vitro or in vivo;* (2) regulate communication between cells; (3) regulate expression of genes whose expression Is modulated by binding of CRSP (e.g., Crisp-1) to a receptor; (4) regulate gene transcription in a cell involved in development or differentiation, either *in vitro or in vivo;* (5) regulate cellular proliferation, either *In vitro or in vivo;* (6) form and/or maintain ordered spatial arrangements of differentiated tissues In vertebrates; (7) modulate cell death (e.g. cell survival); (8) regulate cell migration; and/or (9) modulate Immune system function.

In addition to the nucleic add molecules encoding CRSP proteins described above, one of ordinary skill can generate isolated nucleic acid molecules which are antisense thereto. An "antisense" nucleic acid comprises a nucleotide sequence which is complementary to a "sense" nucleic acid encoding a protein, e.g., complementary to the coding strand of a double-stranded cDNA molecule or complementary to an mRNA sequence. Accordingly, an antisense nucleic acid can hydrogen bond to a sense nucleic add. The antisense nucleic acid can be complementary to an entire CRSP coding strand, or to only a portion thereof. In one embodiment, an antisense nucleic acid molecule is antisense to a 'coding region' of the coding strand of a nucleotide sequence encoding CRSP. The term "coding region" refers to the region of the nucleotide sequence comprising codons which are translated into amino acid residues (e.g., the coding region of human CRSP-1 corresponds to SEQ ID NO:3, the coding region of human CRSP-2 corresponds to SEO ID NO:6, the coding region of human CRSP-3 corresponds to SEO ID NO:9, the coding region of human CRSP-4 corresponds to SEO 10 NO:12, and the coding region of human CRSP-iike-N corresponds to SEQ ID NO: 15). The antisense nucleic acid molecule is antisense to a 'noncoding region' of the coding strand of a nucleotide sequence encoding CRSP. The term "noncoding region" refers to 5' and 3' sequences which flank the coding region that are not translated into amino adds (i.e.. also referred to as 5' and 3' untranslated regions).

Given the coding strand sequences encoding CRSP disclosed herein (e.g., SEa 10 NO:3, SEO ID NO:6, SEQ ID NO:9, or SEO ID N0:12), antisense nucleic adds can be designed according to the rules of Watson and Crick base pairing. The antisense nucleic acid molecule can be complementary to the entire coding region of CRSP mRNA, but more preferably Is an oligonucleotide which is antisense to only a portion of the coding or noncoding region of CRSP mRNA. For example, the antisense oligonucleotide can be complementary to the region surrounding the translation start site of CRSP mRNA. An antisense oligonucleotide can be, for example, about 5.10, 15, 20, 25, 30, 35, 40, 45 or 50 nucleotides in length. An antisense nucleic add can be constructed using chemical synthesis and enzymatic ligation reactions using procedures known in the art For example, an antisense nucleic acid (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. Examples of modified nucleotides which can be used to generate the antisense nucleic add include 5-fluorouracil, 5-bromouracil, 5-chlarouracii, 5-lodouracil, hypoxanthine, xantine, 4-acctylcytosine, 5-(carboxyhydroxylmethyl) uracil, 5-carboxymethylaminamethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-mathykytosine, 5-methylcytasine, NB-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyledenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5- oxyacetic acid methylester, uracil-5-ox-yacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection).

The antisense nucleic acid molecules are typically administered to a subject or generated *in situ* such that they hybridize with or bind to cellular mRNA and/or genomic DNA encoding a CRSP protein to thereby inhabit expression of the protein, e.g., by inhibiting transcription and/or translation. The hybridization can be by conventional nucleotide complementarity to form a stable duplex, or, for example, In the case of an antisense nucleic acid molecule which binds to DNA duplexes, through specific interactions in the major groove of the double helix. An example of a route of administration of antisense nucleic add molecules includes direct injection at a tissue site. Alternatively, antisense nucleic add molecules can be modified to target selected cells and then administered systemically. For example, for systemic administration, antisense molecules can be modified such that they specifically bind to receptors or antigens expressed on a selected cell surface, e.g., by linking the antisense nucleic acid molecules to peptides or antibodies which bind to cell surface receptors or antigens. The antisense nucleic acid molecules can also be delivered to cells using the vectors described herein. To achieve sufficient intracellular concentrations of the antisense molecules, vector constructs in which the antisense nucleic acid molecule is placed under the control of a strong pol II or pol III promoter are preferred.

The antisense nucleic acid molecule may be an α-anomeric nucleic acid molecule. An α-anomeric nucleic acid molecule forms specific double-stranded hybrids with complementary RNA in which, contrary to the usual β-units, thestrands run parallel to each other (Gaultier et al. (1987) Nucleic Acids. Res. 15:6625-6641). The antisense nucleic acidmolecule can also comprise a 2'-o-methylribonucleotide (Inoue et al. (1987) Nucleic Acids Res. 15:6131-6148) or achimeric RNA-DNA analogue (Inoue et al. (1987) FEBS Lett. 215:327-330).

An antisense nucleic acid of the invention may also be a ribozyme. Ribozymes are catalytic RNA molecules with ribonuclease activity which are capable of cleaving a single-stranded nucleic acid, such as an mRNA, to which they have a complementary region. Thus, ribozymes (e.g., hammerhead ribozymes (described in Haselhoff and Gerlach (1988) Nature 334:585-591)) can be used to catalytically deave CRSP mRNA transcripts to thereby inhibit translation of CRSP mRNA. A ribozyme having specificity for a CRSP-encoding nucleic acid can be designed based upon the nucleotide sequence of a CRSP CDNA disclose herein (i.e., SEO ID NO:1, SEQ ID NO:4, SEO ID NO:7, SEQ ID NO:10. the nucleotide sequence of the DNA insert of the plasmid deposited with ATCC as Accession Number 98634, or the nucleotide sequence of the DNA insert of the plasmid deposited with ATCC as Accession Number 98633). For example, a derivative of a *Tetrahymena* L-19 IVS RNA can be constructed in which the nucleotide sequence of the active site is complementary to the nucleotide sequence to be cleaved in a CRSP-encoding mRNA. See, e.g, Cech et al. U.S. Patent No. 4,987,071; and Cech et al. U.S. Patent No. 5,116,742. Alternatively, CRSP mRNA can be used to elect a catalytic RNA having a specific ribonuclease activity from a pool of RNA molecules. See, e.g., Bartel, D. and Szostak, J.W. (1993) Science 261:1411-1418.

AftemaWety, CRSP gene expression can be Inhibited by targeting nucleotide sequences complementary to the regulatory region of the CRSP (e.g., the CRSP promoter and/or enhancers) to form triple helical structures thatprevent transcription of the CRSP gene in target cells. See generally, Helene, C. (1991) Anticancer Drug Des. 6(6):568-84; Helene, C. et at. (1992) Ann N. Y. Acad. Sci. 660:27-36; and Maher, L.J. (1992) Bioassays 14(12):807-15.

The CRSP nucleic acid molecules of the present invention can be modified at the base moiety, sugar moiety or phosphate backbone to improve, e.g., the stability, hybridization, or solubility of the molecule. For example, the deoxyribose phosphate backbone of the nucleic acid molecules can be modified to generate peptide nucleic acids (see Hyrup B. et al. (1996) Bioorganic & Medicinal Chemistry 4(1): 5-23). As used herein, the terms 'peptide nucleic acids' or'PNAs' refer to nucleic acid mimics, e.g., DNA mimics, In which the deoxyribose phosphate backbone is replaced by a pseudopeptide backbone and only the four natural nucleobases are retained. The neutral backbone of PNAs has been shown to allow for specific hybridization to DNA and RNA under conditions of low ionic strength. The synthesis of PNA oligomers can be performed using standard solid phase peptide synthesis protocols as described in Hyrup B. *et al.* (1996) *supra;* Perry-O'Keefe et al. PNAS 93: 14670-675.

PNAs of CRSP nucleic acid molecules can be used In therapeutic and diagnostic applications. For example, PNAs can be used as antisense or antigene agents for sequence-specific modulation of gene expression by, for example, inducing transcription or translation arrest or inhibiting replication. PNAs of CRSP nucleic acid molecules can also be used in the analysis of single base pair mutations in a gene, (e.g., by PNA-directed PCR damping); as 'artificial restriction enzymes' when used in combination with other enzyme, (e.g., S1 nucleases (Hyrup B. (1996) *supra));* or as probes or primers for DNA sequencing or hybridization (Hyrup B. *et al.* (1996) *supra;* Perry-O'Keefe *supra).*

PNAs of CRSP can be modified, (e.g., to enhance their stability or cellular uptake), by attaching lipophilic or other helper groups to PNA, by the formation of PNA-DNA chimeras, or by the use of liposomes or other techniques of drug delivery known in the art. For example, PNA-DNA chimeras of CRSP nucleic acid molecules can be generated which may combine the advantageous properties of PNA and DNA Such chimeras allow DNA recognition enzymes, (e.g., RNAse H and DNA polymerases), to interact with the DNA portion while the PNA portion would provide high binding affinity and specificity. PNA-DNA chimeras can be linked using linkers of appropriate lengths selected in terms of base stacking, number of bonds between the nucleobases, and orientation (Hyrup B. (1998) *supra).* The synthesis of PNA-DNA chimeras can be performed as described in Hyrup B. (1996) *supra* and Finn P.J, et al. (1996) Nucleic Acids Res.24 (17): 3357-63. For example, a DNA chain can be synthesized on a solid support using standard phosphoramidite coupling chemistry and modified nucleoside analogs, e.g., 5'-(4-methoxytrityl)amino-5'-deoxy-thymidine phosphoramidite, can be used as a between the PNA and the 5' end of DNA (Mag, M. et al. (1989) Nucleic Acid Res. 17: 5973-88). PNA monomers are then coupled in a stepwise manner to produce a chimeric molecule with a 5' PNA segment and a 3' DNA segment (Finn P.J. *et al.* (1996) *supra).* Alternatively, chimeric moleclues can be synthesized with a 5' DNAsegment and a 3' PNA segment (Peterser, K.H. et al. (1975) Bioorganic Med. Chem. Lett 5:1119-11124).

The oligonucleotide may include other appended groups such as peptides (e.g., for targeting host cell receptorsin vivo), or agents facilitating transport across the cell membrane (see, e.g., Letsinger et al. (1989) Proc. Nad. Acad.Sci. US. 66:6553-6556; Lemaitre et al. (1987) Proc. Natl. Acad Sci. USA 84:648-652; PCT Publication No. WO88/09810,published December 15, 1988) or the blood-brain barrier (see, e.g., PCT Publication No: WO89/10134, published April 25, 1988). In addition, oligonucleotides can be modified with hybridization-triggered cleavage agents (See, e.g., Krol et al. (1988) BioTechniques 6:958-976) or intercalating agents. (See, e.g., Zon (1988) Pharm. Res. 5:539-549). To this end, the oligonucleotide may be conjugated to another molecule, (e.g., a peptide, hybridization triggered cross-linking agent, transport agent, or hybridization-triggered cleavage agent).

### II. Isolated CRSP Proteins and Anti-CRSP Antibodies

One aspect of the invention pertains to isolated CRSP proteins, and biologically active portions thereof, as well as polypeptide fragments suitable for use as immunogens to raise anti-CRSP antibodies. In one embodiment, native CRSP proteins can be isolated from cells or tissue sources by an appropriate purification scheme using standard protein purification techniques. In another embodiment, CRSP proteins are produced by recombinant DNA techniques. Alternative to recombinant expression, a CRSP protein or polypeptide can be synthesized chemically using standard peptide synthesis techniques.

An "isolated" or "purified" protein or biologically active portion thereof is substantially free of cellular material or other contaminating proteins from the cell or tissue source from which the CRSP protein is derived, or substantially free from chemical precursors or other chemicals when chemically synthesized. The language "substantially free of cellular material" includes preparations of CRSP protein in which the protein is separated from cellular components of the cells from which it is isolated or recombinantly produced. In one embodiment, the language "substantially free of cellular material" includes preparations of CRSP protein having less than about 30% (by dry weight) of non-CRSP protein (also referred to herein as a "contaminating protein"), more preferably less than about 20% of non-CRSP protein, still more preferably less than about 10% of non-CRSP protein, and most preferably less than about 5% non-CRSP protein. When the CRSP protein or biologically active portion thereof is recombinantly produced, it is also preferably substantially free of culture medium, i.e., culture medium represents less than about 20%, more preferably less than about 10%, and most preferably less than about 5% of the volume of the protein preparation.

The language "substantially free of chemical precursors or other chemicals" includes preparations of CRSP protein in which the protein is separated from chemical precursors or other chemicals which are involved in the synthesis of the protein. In one embodiment, the language "substantially free of chemical precursors or other chemicals" includes preparations of CRSP protein having less than about 30% (by dry weight) of chemical precursors or non-CRSP chemicals, more preferably less than about 20% chemical precursors or non-CRSP chemicals, still more preferably less than about 10% chemical precursors or non-CRSP chemicals, and most preferably less than about 5% chemical precursors or non-CRSP chemicals.

Biologically active portions of a CRSP protein include peptides comprising amino acid sequences sufficiently homologous to or derived from the amino acid sequence of the CRSP protein, e.g., the amino acid sequence shown in SEQ ID NO:2, SEQ ID NO:5, SEQ ID NO: 8, or SEQ ID NO: 11, which include less amino acids than the full length CRSP proteins, and exhibit at least one activity of a CRSP protein. Typically, biologically active portions comprise a domain or motif with at least one activity of the CRSP protein. A biologically active portion of a CRSP protein can be a polypeptide which is, for example, 10, 25, 50, 100 or more amino acids in length.

In one embodiment, a biologically active portion of a CRSP protein comprises at least a cysteine-rich region. In another embodiment, a biologically active portion of a CRSP protein comprises at least a cysteine-rich region, wherein the cysteine-rich region includes at least one cysteine-rich domain. In yet another embodiment, a biologically active portion of a CRSP protein comprises at least a signal sequence.

In an alternative embodiment, a biologically active portion of a CRSP protein comprises a CRSP amino acid sequence lacking a signal sequence.

It is to be understood that a preferred biologically active portion of a CRSP protein of the present invention may contain at least one of the above-identified structural domains. A more preferred biologically active portion of a CRSP protein may contain at least two of the above-identified structural domains. An even more preferred biologically active portion of a CRSP protein may contain at least three of the above-identified structural domains. A particularly preferred biologically active portion of a CRSP protein of the present invention may contain at least four of the above-identified structural domains.

Moreover, other biologically active portions, in which other regions of the protein are deleted, can be prepared by recombinant techniques and evaluated for one or more of the functional activities of a native CRSP protein.

The CRSP protein of the invention has an amino acid sequence as shown in the attached claims.

To determine the percent homology of two amino acid sequences or of two nucleic acids, the sequences are aligned for optimal comparison purposes (e.g., gaps can be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are homologous at that position (i.e., as used herein amino acid or nucleic acid "homology" is equivalent to amino acid or nucleic acid "identity"). The percent homology between the two sequences is a function of the number of identical positions shared by the sequences (i.e., % homology = # of identical positions/total # of positions x 100). The determination of percent homology between two sequences can be accomplished using a mathematical algorithim. A preferred, non-limiting example of a mathematical algorithim utilized for the comparison of two sequences is the algorithm of Karlin and Altschul (1990) Proc. Natl. Acad. Sci. USA 87:2264-68, modified as in Karlin and Altschul (1993) Proc. Natl. Acad. Sci. USA 90:5873-77. Such an algorithm is incorporated into the NBLAST and XBLAST programs of Altschul, et al. (1990) J. Mol. Biol. 215:403-10. BLAST nucleotide searches can be performed performed with the NBLAST program, score = 100, wordlength = 12 to obtain nucleotide sequences homologous to CRSP nucleic acid molecules of the invention. BLAST protein searches can be performed with the XBLAST program, score = 50, wordlength = 3 to obtain amino acid sequences homologous to CRSP protein molecules of the invention. To obtain gapped alignments for comparison purposes, Gapped BLAST can be utilized as described in Altschul et al., (1997) Nucleic Acids Research 25(17):3389-3402. When utilizing BLAST and Gapped BLAST programs, the default parameters of the respective programs (e.g., XBLAST and NBLAST) can be used. See http://www.ncbi.nlm.nih.gov. Another.preferred, non-limiting example of a mathematical algorithim utilized for the comparison of sequences is the algorithm of Myers and Miller, CABIOS (1989). Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used.

The invention can be used to provide CRSP chimeric or fusion proteins. As used herein, a CRSP "chimeric protein" or "fusion protein" comprises a CRSP polypeptide operatively linked to a non-CRSP polypeptide. A "CRSP polypeptide" refers to a polypeptide having an amino acid sequence corresponding to CRSP, whereas a "non-CRSP polypeptide" refers to a polypeptide having an amino acid sequence corresponding to a protein which is not substantially homologous to the CRSP protein, e.g., a protein which is different from the CRSP protein and which is derived from the same or a different organism. Within a CRSP fusion protein the CRSP polypeptide can correspond to all or a portion of a CRSP protein. One CRSP fusion protein comprises at least one biologically active portion of a CRSP protein. Another CRSP fusion protein comprises at least two biologically active portions of a CRSP protein. Another CRSP fusion protein comprises at least three biologically active portions of a CRSP protein, Within the fusion protein, the term "operatively linked" is intended to indicate that the CRSP polypeptide and the non-CRSP polypeptide are fused in-frame to each other. The non-CRSP polypeptide can be fused to the N-terminus or C-terminus of the CRSP polypeptide.

For example, one fusion protein is a GST-CRSP fusion protein in which the CRSP sequences are fused to the C-terminus of the GST sequences. Such fusion proteins can facilitate the purification of recombinant CRSP.

Another fusion protein is a CRSP protein containing a heterologous signal sequence at its N-terminus. For example, the native CRSP signal sequence (i.e, about amino acids 1 to 23 of SEQ ID NO:2) can be removed and replaced with a signal sequence from another protein. In certain host cells (e.g., mammalian host cells), expression and/or secretion of CRSP can be increased through use of a heterologous signal sequence.

Yet another fusion protein is a CRSP-immunoglobulin fusion protein in which the CRSP sequences comprising primarily the CRSP cysteine-rich regions are fused to sequences derived from a member of the immunoglobulin protein family. Soluble derivatives have also been made of cell surface glycoproteins in the immunoglobulin gene superfamily consisting of an extracellular domain of the cell surface glycoprotein fused to an immunoglobulin constant (Fc) region (see e.g., Capon, D.J. et al. (1989) Nature 337:525-531 and Capon U.S. Patents 5,116,964 and 5,428,130 [CD4-IgGI constructs]; Linsley, P.S. et al. (1991) J. Exp. Med. 173:721-730 [a CD28-IgGI construct and a B7-I-IgGI construct]; and Linsley, P.S. et al. (1991) J. Exp. Med. 174:561-569 and U.S. Patent 5,434,131[a CTLA4-IgG1]). Such fusion proteins have proven useful for modulating reeeptor-ligand interactions. Soluble derivatives of cell surface proteins of the tumor necrosis factor receptor (TNPR) superfamily proteins have been made consisting of an extracellular domain of the cell surface receptor fused to an immunoglobulin constant (Fc) region (See for example Moreland et al. (1997) N. Engl. J. Med. 337(3):141-147; van der Poll et al. (1997) Blood 89(10):3727-3734; and Ammann et al. (1997) J. Clin. Invest. 99(7):1699-1703.)

CRSP-immunoglobulin fusion proteins can be incorporated into pharmaceutical compositions and administered to a subject to inhibit an interaction between a CRSP ligand and a CRSP receptor on the surface of a cell, to thereby suppress CRSP-mediated signal transduction *in vivo*. The CRSP-immunoglobulin fusion proteins can be used to affect the bioavailability of a CRSP cognate receptor. Inhibition of the CRSP ligand/CRSP interaction may be useful therapeutically for both the treatment of differentiative or proliferative disorders, as well as modulating (e.g., promoting or inhibiting) developmental responses, cell adhesion, and/or cell fate. Moreover, the CRSP-immunoglobulin fusion proteins of the invention can be used as immunogens to produce anti-CRSP antibodies in a subject, to purify CRSP ligands and in screening assays to identify molecules which inhibit the interaction of CRSP with a CRSP ligand.

Preferably, a CRSP chimeric or fusion protein is produced by standard recombinant DNA techniques. For example, DNA fragments coding for the different polypeptide sequences are ligated together in-frame in accordance with conventional techniques, for example by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. The fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and reamplified to generate a chimeric gene sequence (see, for example, Current Protocols in Molecular Biology, eds. Ausubel et al. John Wiley & Sons: 1992). Moreover, many expression vectors are commercially available that already encode a fusion moiety (e.g., a GST polypeptide). A CRSP-encoding nucleic acid can be cloned into such an expression vector such that the fusion moiety is linked in-frame to the CRSP protein.

The present invention also pertains to variants of the CRSP proteins which function as either CRSP agonists (mimetics) or as CRSP antagonists. Variants of the CRSP proteins can be generated by mutagenesis, e.g., discrete point mutation or truncation of a CRSP protein. An agonist of the CRSP proteins can retain substantially the same, or a subset, of the biological activities of the naturally occurring form of a CRSP protein. An antagonist of a CRSP protein can inhibit one.or more of the activities of the naturally occurring form of the CRSP protein by, for example, competitively binding to a downstream or upstream member of a cellular signaling cascade which includes the CRSP protein. Thus, specific biological effects can be elicited by treatment with a variant of limited function. In one embodiment, treatment of a subject with a variant having a subset of the biological activities of the naturally occurring form of the protein has fewer side effects in a subject relative to treatment with the naturally occurring form of the CRSP protein.

Variants of a CRSP protein which function as either CRSP agonists (mimetics) or as CRSP antagonists can be identified by screening combinatorial libraries of mutants, e.g., truncation mutants, of a CRSP protein for CRSP protein agonist or antagonist activity. In one embodiment, a variegated library of CRSP variants is generated by combinatorial mutagenesis at the nucleic acid level and is encoded by a variegated gene library. A variegated library of CRSP variants can be produced by, for example, enzymatically ligating a mixture of synthetic oligonucleotides into gene sequences such that a degenerate set of potential CRSP sequences is expressible as individual polypeptides, or alternatively, as a set of larger fusion proteins (e.g., for phage display) containing the set of CRSP sequences therein. There are a variety of methods which can be used to produce libraries of potential CRSP variants from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential CRSP sequences. Methods for synthesizing degenerate oligonucleotides are known in the art (see, e.g., Narang, S.A. (1983) Tetrahedron 39:3; Itakura et al. (1984) Annu. Rev. Biochem 53:323; Itakura et al. (1984) Science 198:1056; Ike et al. (1983) Nucleic Acid Res. 11:477.

In addition, libraries of fragments of a CRSP protein coding sequence can be used to generate a variegated population of CRSP fragments for screening and subsequent selection of variants of a CRSP protein. In one embodiment, a library of coding sequence fragments can be generated by treating a double stranded PCR fragment of a CRSP coding sequence with a nuclease under conditions wherein nicking occurs only about once per molecule, denaturing the double stranded DNA, renaturing the DNA to form double stranded DNA which can include sense/antisense pairs from different nicked products, removing single stranded portions from reformed duplexes by treatment with S I nuclease, and ligating the resulting fragment library into an expression vector. By this method, an expression library can be derived which encodes N-terminal, C-terminal and internal fragments of various sizes of the CRSP protein.

Several techniques are known in the art for screening gene products of combinatorial libraries made by point mutations or truncation, and for screening cDNA libraries for gene products having a selected property. Such techniques are adaptable for rapid screening of the gene libraries generated by the combinatorial mutagenesis of CRSP proteins. The most widely used techniques, which are amenable to high throughput analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recrusive ensemble mutagenesis (REM), a new technique which enhances the frequency of functional mutants in the libraries, can be used in combination with the screening assays to identify CRSP variants (Arkin and Yourvan (1992) PNAS 89:7811-7815; Delgrave et al. (1993) Protein Engineering 6(3):327-331).

Cell based assays can be exploited to analyze a variegated CRSP library. For example, a library of expression vectors can be transfected into a cell line which ordinarily responds to a particular ligand in a CRSP-dependent manner. The transfected cells are then contacted with the ligand and the effect of expression of the mutant on signaling by the ligand can be detected, e.g., by measuring any of a number of immune cell responses. Plasmid DNA can then be recovered from the cells which score for inhibition, or alternatively, potentiation of ligand induction, and the individual clones further characterized.

An isolated CRSP protein, or a portion or fragment thereof, can be used as an immunogen to generate antibodies that bind CRSP using standard techniques for polyclonal and monoclonal antibody preparation. A full-length CRSP protein can be used or, alternatively, the invention provides antigenic peptide fragments of CRSP for use as immunogens. The antigenic peptide of CRSP comprises at least 8 amino acid residues and encompasses an epitope of CRSP such that an antibody raised against the peptide forms a specific immune complex with CRSP. Preferably, the antigenic peptide comprises at least 10 amino acid residues, more preferably at least 15 amino acid residues, even more preferably at least 20 amino acid residues, and most preferably at least 30 amino acid residues.

Preferred epitopes encompassed by the antigenic peptide are regions of CRSP that are located on the surface of the protein, e.g., hydrophilic regions.

A CRSP immunogen typically is used to prepare antibodies by immunizing a suitable subject, (e.g., rabbit, goat, mouse or other mammal) with the immunogen. An appropriate immunogenic preparation can contain, for example, recombinantly expressed CRSP protein or a chemically synthesized CRSP polypeptide. The preparation can further include an adjuvant, such as Freund's complete or incomplete adjuvant, or similar immunostimulatory agent. Immunization of a suitable subject with an immunogenic CRSP preparation induces a polyclonal anti-CRSP antibody response.

Accordingly, another aspect of the invention pertains to anti-CRSP antibodies. The term "antibody" as used herein refers to immunoglobulin molecules and immunologically active portions of immunoglobulin molecules, i.e., molecules that contain an antigen binding site which specifically binds (immunoreacts with) an antigen, such as CRSP. Examples of immunologically active portions of immunoglobulin molecules include F(ab) and F(ab')₂ fragments which can be generated by treating the antibody with an enzyme such as pepsin. The invention provides polyclonal and monoclonal antibodies that bind CRSP. The term "monoclonal antibody" or "monoclonal antibody composition", as used herein, refers to a population of antibody molecules that contain only one species of an antigen binding site capable of immunoreacting with a particular epitope of CRSP. A monoclonal antibody composition thus typically displays a single binding affinity for a particular CRSP protein with which it immunoreacts.

Polyclonal anti-CRSP antibodies can be prepared as described above by immunizing a suitable subject with a CRSP immunogen. The anti-CRSP antibody titer in the immunized subject can be monitored over time by standard techniques, such as with an enzyme linked immunosorbent assay (ELISA) using immobilized CRSP. If desired, the antibody molecules directed against CRSP can be isolated from the mammal (e.g., from the blood) and further purified by well known techniques, such as protein A chromatography to obtain the IgG fraction. At an appropriate time after immunization, e.g., when the anti-CRSP antibody titers are highest, antibody-producing cells can be obtained from the subject and used to prepare monoclonal antibodies by standard techniques, such as the hybridoma technique originally described by Kohler and Milstein (1975) Nature 256:495-497) (see also, Brown et al. (1981) J. Immunol. 127:539-46; Brown et al. (1980) J. Biol. Chem. .255:4980-83; Yeh et al. (1976) PNAS 76:2927-31; and Yeh et al. (1982) Int. J. Cancer 29:269-75), the more recent human B cell hybridoma technique (Kozbor et al. (1983) Immunol Today 4:72), the EBV-hybridoma technique (Cole et al. (1985), Monoclonal Antibodies and Cancer Therapy, Alan R. Liss, Inc., pp. 77-96) or trioma techniques. The technology for producing monoclonal antibody hybridomas is well known (see generally R. H. Kenneth, in Monoclonal Antibodies: A New Dimension In Biological Analyses, Plenum Publishing Corp., New York, New York (1980); E. A. Lerner (1981) Yale J. Biol. Med., 54:387-402; M. L. Gefter et al. (1977) Somatic Cell Genet. 3:231-36). Briefly, an immortal cell line (typically a myeloma) is fused to lymphocytes (typically splenocytes) from a mammal immunized with a CRSP immunogen as described above, and the culture supernatants of the resulting hybridoma cells are screened to identify a hybridoma producing a monoclonal antibody that binds CRSP.

Any of the many well known protocols used for fusing lymphocytes and immortalized cell lines can be applied for the purpose of generating an anti-CRSP monoclonal antibody (see, e.g., G. Galfre et al. (1977) Nature 266:55052; Gefter et al. Somatic Cell Genet., cited *supra;* Lerner, *Yale J Biol. Med.,* cited *supra;* Kenneth, *Monoclonal Antibodies,* cited *supra*). Moreover, the ordinarily skilled worker will appreciate that there are many variations of such methods which also would be useful. Typically, the immortal cell line (e.g., a myeloma cell line) is derived from the same mammalian species as the lymphocytes. For example, murine hybridomas can be made by fusing lymphocytes from a mouse immunized with an immunogenic preparation of the present invention with an immortalized mouse cell line. Preferred immortal cell lines are mouse myeloma cell lines that are sensitive to culture medium containing hypoxanthine, aminopterin and thymidine ("HAT medium"). Any of a number of myeloma cell lines can be used as a fusion partner according to standard techniques, e.g., the P3-NS1/1-Ag4-1, P3-x63-Ag8.653 or Sp2/O-Ag14 myeloma lines. These myeloma lines are available from ATCC. Typically, HAT-sensitive mouse myeloma cells are fused to mouse splenocytes using polyethylene glycol ("PEG"). Hybridoma cells resulting from the fusion are then selected using HAT medium, which kills unfused and unproductively fused myeloma cells (unfused splenocytes die after several days because they are not transformed). Hybridoma cells producing a monoclonal antibody of the invention are detected by screening the hybridoma culture supernatants for antibodies that bind CRSP, e.g., using a standard ELISA assay.

Alternative to preparing monoclonal antibody-secreting hybridomas, a monoclonal anti-CRSP antibody can be identified and isolated by screening a recombinant combinatorial immunoglobulin library (e.g., an antibody phage display library) with CRSP to thereby isolate immunoglobulin library members that bind CRSP. Kits for generating and screening phage display libraries are commercially available (e.g., the Pharmacia *Recombinant Phage Antibody System,* Catalog No. 27-9400-0 1; and the Stratagene *SurfZAP^{™} Phage Display Kit,* Catalog No. 240612). Additionally, examples of methods and reagents particularly amenable for use in generating and screening antibody display library can be found in, for example, Ladner et al. U.S. Patent No. 5,223,409; Kang et al. PCT International Publication No. WO 92/18619; Dower et al. PCT International Publication No. WO 91/17271; Winter et al. PCT International Publication WO 92/20791; Markland et al. PCT International Publication No. WO 92/15679; Breitling et al. PCT International Publication WO 93/01288; McCafferty et al. PCT International Publication No. WO 92/01047; Garrard et al. PCT International Publication No. WO 92/09690; Ladner et al. PCT International Publication No. WO 90/02809; Fuchs et al. (1991) Bio/Technology 9:1370-1372; Hay et al. (1992) Hum. Antibod. Hybridomas 3:81-85; Huse et al. (1989) Science 246:1275-1281; Griffiths et al. (1993) EMBO J 12:725-734; Hawkins et al. (1992) J. Mol. Biol. 226:889-896; Clarkson et al. (1991) Nature 352:624-628; Gram et al. (1992) PNAS 89:3576-3580; Garrad et al. (1991) Bio/Technology 9:1373-1377; Hoogenboom et al. (1991) Nuc. Acid Res. 19:4133-4137; Barbas et al. (1991) PNAS 88:7978-7982; and McCafferty et al. Nature (1990) 348:552-554.

Additionally, recombinant anti-CRSP antibodies, such as chimeric and humanized monoclonal antibodies, comprising both human and non-human portions, which can be made using standard recombinant DNA techniques, are within the scope of the invention. Such chimeric and humanized monoclonal antibodies can be produced by recombinant DNA techniques known in the art, for example using methods described in Robinson et al. International Application No. PCT/US86/02269; Akira, et al. European Patent Application 184,187; Taniguchi, M., European Patent Application 171,496; Morrison et al. European Patent Application 173,494; Neuberger et al. PCT International Publication No. WO 86/01533; Cabilly et al. U.S. Patent No. 4,816,567; Cabilly et al. European Patent Application 125,023; Better et al. (1988) Science 240:1041-1043; Liu et al. (1987) PNAS 84:3439-3443; Liu et al. (1987) J. Immunol. 139:3521-3526; Sun et al. (1987) PNAS 84:214-218; Nishimura et al. (1987) Canc. Res. 47:999-1005; Wood et al. (1985) Nature 314:446-449; and Shaw et al. (1988) J Natl. Cancer Inst. 80:1553-1559); Morrison, S. L. (1985) Science 229:1202-1207; Oi et al. (1986) BioTechniques 4:214; Winter U.S. Patent 5,225,539; Jones et al. (1986) Nature 321:552-525; Verhoeyan et al. (1988) Science 239:1534; and Beidler et al. (1988) J Immunol. 141:4053-4060.

An anti-CRSP antibody (e.g., monoclonal antibody) can be used to isolate CRSP by standard techniques, such as affinity chromatography or immunoprecipitation. An anti-CRSP antibody can facilitate the purification of natural CRSP from cells and of recombinantly produced CRSP expressed in host cells. Moreover, an anti-CRSP antibody can be used to detect CRSP protein (e.g., in a cellular lysate or cell supernatant) in order to evaluate the abundance and pattern of expression of the CRSP protein. Anti-CRSP antibodies can be used diagnostically to monitor protein levels in tissue as part of a clinical testing procedure, e.g., to, for example, determine the efficacy of a given treatment regimen. Detection can be facilitated by coupling (i.e., physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

### III. Recombinant Expression Vectors and Host Cells

Another aspect of the invention pertains to vectors, preferably expression vectors, containing a nucleic acid encoding CRSP (or a portion thereof). As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (e.g., replication defective retroviruses, adenoviruses and adeno-associated viruses), which serve equivalent functions.

The recombinant expression vectors of the invention comprise a nucleic acid of the invention in a form suitable for expression of the nucleic acid in a host cell, which means that the recombinant expression vectors include one or more regulatory sequences, selected on the basis of the host cells to be used for expression, which is operatively linked to the nucleic acid sequence to be expressed. Within a recombinant expression vector, "operably linked" is intended to mean that the nucleotide sequence of interest is linked to the regulatory sequence(s) in a manner which allows for expression of the nucleotide sequence (e.g., in an *in vitro* transcription/translation system or in a host cell when the vector is introduced into the host cell). The term "regulatory sequence" is intended to includes promoters, enhancers and other expression control elements (e.g., polyadenylation signals). Such regulatory sequences are described, for example, in Goeddel; Gene Expression Technology: Methods in Enzymology 185, Academic Press, San Diego, CA (1990). Regulatory sequences include those which direct constitutive expression of a nucleotide sequence in many types of host cell and those which direct expression of the nucleotide sequence only in certain host cells (e.g., tissue-specific regulatory sequences). It will be appreciated by those skilled in the art that the design of the expression vector can depend on such factors as the choice of the host cell to be transformed, the level of expression of protein desired, etc. The expression vectors of the invention can be introduced into host cells to thereby produce proteins or peptides, including fusion proteins or peptides, encoded by nucleic acids as described herein (e.g., CRSP proteins, mutant forms of CRSP, fusion proteins, etc.).

The recombinant expression vectors of the invention can be designed for expression of CRSP in prokaryotic or eukaryotic cells. For example, CRSP can be expressed in bacterial cells such as *E. coli,* insect cells (using baculovirus expression vectors) yeast cells or mammalian cells. Suitable host cells are discussed further in Goeddel, Gene Expression Technology: Methods in Enzymology 185, Academic Press, San Diego, CA (1990). Alternatively, the recombinant expression vector can be transcribed and translated *in vitro,* for example using T7 promoter regulatory sequences and T7 polymerase.

Expression of proteins in prokaryotes is most often carried out in *E*. *coli* with vectors containing constitutive or inducible promotors directing the expression of either fusion or non-fusion proteins. Fusion vectors add a number of amino acids to a protein encoded therein, usually to the amino terminus of the recombinant protein. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith, D.B. and Johnson, K.S. (1988) Gene 67:31-40), pMAL (New England Biolabs, Beverly, MA) and pRIT5 (Pharmacia, Piscataway, NJ) which fuse glutathione S-transferase (GST), maltose E binding protein, or protein A, respectively, to the target recombinant protein.

Purified fusion proteins can be utilized in CRSP activity assays, in CRSP ligand binding (e.g., direct assays or competitive assays described in detail below), to generate antibodies specific for CRSP proteins, as examples. In a preferred embodiment, a CRSP fusion expressed in a retroviral expression vector of the present invention can be utilized to infect bone marrow cells which are subsequently transplanted into irradiated recipients. The pathology of the subject recipient is then examined after sufficient time has passed (e.g six (6) weeks).

Examples of suitable inducible non-fusion *E. coli* expression vectors include pTrc (Amann et al., (1988) Gene 69:301-315) and pET 11d (Studier et al., Gene Expression Technology: Methods in Enzymology 185, Academic Press, San Diego, California (1990) 60-89). Target gene expression from the pTrc vector relies on host RNA polymerase transcription from a hybrid trp-lac fusion promoter. Target gene expression from the pET 11d vector relies on transcription from a T7 gn10-lac fusion promoter mediated by a coexpressed viral RNA polymerase (T7 gn1). This viral polymerase is supplied by host strains BL21(DE3) or HMS174(DE3) from a resident λ prophage harboring a T7 gnl gene under the transcriptional control of the lacUV 5 promoter.

One strategy to maximize recombinant protein expression in *E. coli* is to express the protein in a host bacteria with an impaired capacity to proteolytically cleave the recombinant protein (Gottesman, S., Gene Expression Technology: Methods in Enzymology 185, Academic Press, San Diego, California (1990) 119-128). Another strategy is to alter the nucleic acid sequence of the nucleic acid to be inserted into an expression vector so that the individual codons for each amino acid are those preferentially utilized in *E*. *coli* (Wada et al., (1992) Nucleic Acids Res. 20:2111-2118). Such alteration of nucleic acid sequences of the invention can be carried out by standard DNA synthesis techniques.

In another embodiment, the CRSP expression vector is a yeast expression vector. Examples of vectors for expression in yeast *S*. *cerivisae* include pYepSec1 (Baldari, et al., (1987) Embo J 6:229-234), pMFa (Kurjan and Herskowitz, (1982) Cell 30:933-943), pJRY88 (Schultz et al., (1987) Gene 54:113-123), pYES2 (Invitrogen Corporation, San Diego, CA), and picZ (InVitrogen Corp, San Diego, CA).

Alternatively, CRSP can be expressed in insect cells using baculovirus expression vectors. Baculovirus vectors available for expression of proteins in cultured insect cells (e.g., Sf 9 cells) include the pAc series (Smith et al. (1983) Mol. Cell Biol. 3:2156-2165) and the pVL series (Lucklow and Summers (1989) Virology 170:31-39).

In yet another embodiment, a nucleic acid of the invention is expressed in mammalian cells using a mammalian expression vector. Examples of mammalian expression vectors include pCDM8 (Seed, B. (1987) Nature 329:840) and pMT2PC (Kaufman et al. (1987) EMBO J. 6:187-195). When used in mammalian cells, the expression vector's control functions are often provided by viral regulatory elements. For example, commonly used promoters are derived from polyoma, Adenovirus 2, cytomegalovirus and Simian Virus 40. For other suitable expression systems for both prokaryotic and eukaryotic cells see chapters 16 and 17 of Sambrook, J., Fritsh, E. F., and Maniatis, T. Molecular Cloning: A Laboratory Manual. 2nd, ed., Cold Spring Harbor Laboratory, Cold Spring Harbor Laboratory Press, Cold Spring Harbor, NY, 1989.

In another embodiment, the recombinant mammalian expression vector is capable of directing expression of the nucleic acid preferentially in a particular cell type (e.g., tissue-specific regulatory elements are used to express the nucleic acid). Tissue-specific regulatory elements are known in the art. Non-limiting examples of suitable tissue-specific promoters include the albumin promoter (liver-specific; Pinkert et al. (1987) Genes Dev. 1:268-277), lymphoid-specific promoters (Calame and Eaton (1988) Adv. Immunol. 43:235-275), in particular promoters of T cell receptors (Winoto and Baltimore (1989) EMBO J. 8:729-733) and immunoglobulins (Banerji et al. (1983) Cell 33:729-740; Queen and Baltimore (1983) Cell 33:741-748), neuron-specific promoters (e.g., the neurofilament promoter; Byrne and Ruddle (1989) PNAS 86:5473-5477), pancreas-specific promoters (Edlund et al. (1985) Science 230:912-916), and mammary gland-specific promoters (e.g., milk whey promoter; U.S. Patent No. 4,873,316 and European Application Publication No. 264,166). Developmentally-regulated promoters are also encompassed, for example the murine hox promoters (Kessel and Gruss (1990) Science 249:374-379) and the α-fetoprotein promoter (Campes and Tilghman (1989) Genes Dev. 3:537-546).

The invention further provides a recombinant expression vector comprising a DNA molecule of the invention cloned into the expression vector in an antisense orientation. That is, the DNA molecule is operatively linked to a regulatory sequence in a manner which allows for expression (by transcription of the DNA molecule) of an RNA molecule which is antisense to CRSP mRNA. Regulatory sequences operatively linked to a nucleic acid cloned in the antisense orientation can be chosen which direct the continuous expression of the antisense RNA molecule in a variety of cell types, for instance viral promoters and/or enhancers, or regulatory sequences can be chosen which direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activity of which can be determined by the cell type into which the vector is introduced. For a discussion of the regulation of gene expression using antisense genes see Weintraub, H. et al., Antisense RNA as a molecular tool for genetic analysis, Reviews - Trends in Genetics, Vol. 1 (1) 1986.

Another aspect of the invention pertains to host cells into which a recombinant expression vector of the invention has been introduced. The terms "host cell" and "recombinant host cell" are used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, CRSP protein can be expressed in bacterial cells such as *E*. *coli,* insect cells, yeast or mammalian cells (such as Chinese hamster ovary cells (CHO) or COS cells). Other suitable host cells are known to those skilled in the art.

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid (e.g., DNA) into a host cell, including calcium phosphate or calcium chloride co-precipitation, DEAE-dextran-mediated transfection, lipofection, or electroporation. Suitable methods for transforming or transfecting host cells can be found in Sambrook, et al. (Molecular Cloning: A Laboratory Manual. 2nd, ed., Cold Spring Harbor Laboratory, Cold Spring Harbor Laboratory Press, Cold Spring Harbor, NY, 1989), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (e.g., resistance to antibiotics) is generally introduced into the host cells along with the gene of interest. Preferred selectable markers include those which confer resistance to drugs, such as G418, hygromycin and methotrexate. Nucleic acid encoding a selectable marker can be introduced into a host cell on the same vector as that encoding CRSP or can be introduced on a separate vector. Cells stably transfected with the introduced nucleic acid can be identified by drug selection (e.g., cells that have incorporated the selectable marker gene will survive, while the other cells die).

A host cell of the invention, such as a prokaryotic or eukaryotic host cell in culture, can be used to produce (i.e., express) CRSP protein. Accordingly, the invention further provides methods for producing CRSP protein using the host cells of the invention. In one embodiment, the method comprises culturing the host cell of invention (into which a recombinant expression vector encoding CRSP has been introduced) in a suitable medium such that CRSP protein is produced. In another embodiment, the method further comprises isolating CRSP from the medium or the host cell.

The host cells of the invention can also be used to produce nonhuman transgenic animals. For example, in one embodiment, a host cell of the invention is a non-human fertilized oocyte or a non-human embryonic stem cell into which CRSP-coding sequences have been introduced. Such host cells can then be used to create non-human transgenic animals in which exogenous CRSP sequences have been introduced into their genome or homologous recombinant animals in which endogenous CRSP sequences have been altered. Such animals are useful for studying the function and/or activity of CRSP and for identifying and/or evaluating modulators of CRSP activity. As used herein, a "transgenic animal" is a non-human animal, preferably a mammal, more preferably a rodent such as a rat or mouse, in which one or more of the cells of the animal includes a transgene. Other examples of transgenic animals include non-human primates, sheep, dogs, cows, goats, chickens, amphibians, etc. A transgene is exogenous DNA which is integrated into the genome of a cell from which a transgenic animal develops and which remains in the genome of the mature animal, thereby directing the expression of an encoded gene product in one or more cell types or tissues of the transgenic animal. As used herein, a "homologous recombinant animal" is a non-human animal, preferably a mammal, more preferably a mouse, in which an endogenous CRSP gene has been altered by homologous recombination between the endogenous gene and an exogenous DNA molecule introduced into a cell of the animal, e.g., an embryonic cell of the animal, prior to developments of the animal.

A non-human transgenic animal of the invention can be created by introducing CRSP-encoding nucleic acid into the male pronuclei of a fertilized oocyte, e.g., by microinjection, retroviral infection, and allowing the oocyte to develop in a pseudopregnant female foster animal. The human CRSP cDNA sequence of the invention can be introduced as a transgene into the genome of a non-human animal. Alternatively, a nonhuman homologue of a human CRSP gene, such as a mouse CRSP gene, can be isolated based on hybridization to the human CRSP cDNA (described further in subsection I above) and used as a transgene. Intronic sequences and polyadenylation signals can also be included in the transgene to increase the efficiency of expression of the transgene. A tissue-specific regulator sequence(s) can be operably linked to the CRSP transgene to direct expression of CRSP protein to particular cells. methods for generating non-human transgenic animals via embryo manipulation and microinjection, particularly animals such as mice, have become conventional in the art and are described, for example, in U.S. Patent Nos. 4,736,866 and 4,870,009, both by Leder et al., U.S. Patent No. 4,873,191 by Wagner et al., and in Hogan, B., Manipulating the Mouse Embryo, (Cold Spring Harbor Laboratory Press, Cold Spring Harbor, N.Y., 1986). Similar methods are used for production of other non-human transgenic animals. A non-human transgenic founder animal can be identified based upon the presence of the CRSP transgene in its genome and/or expression of CRSP mRNA in tissues or cells of the animals. A non-human transgenic founder animal can then be used to breed additional animals carrying the transgene. Moreover, the non-human transgenic animals carrying a transgene encoding CRSP can further be bred to other non-human transgenic animals carrying other transgenes.

To create a homologous recombinant non-human animal, a vector is prepared which contains at least a portion of a CRSP gene into which a deletion, addition or substitution has been introduced to thereby alter, e.g., functionally disrupt, the CRSP gene. The CRSP gene can be a human gene (e.g., the cDNA of SEQ ID NO:3, SEQ ID NO:6, SEQ ID NO:9 or SEQ ID NO:12), but more preferably, is a non-human homologue of a human CRSP gene. For example, a mouse CRSP gene of SEQ ID NO:16 can be used to construct a homologous recombination vector suitable for altering an endogenous CRSP gene in the mouse genome. In a preferred embodiment, the vector is designed such that, upon homologous recombination, the endogenous CRSP gene is functionally disrupted (i.e., no longer encodes a functional protein; also referred to as a "knock out" vector). Alternatively, the vector can be designed such that, upon homologous recombination, the endogenous CRSP gene is mutated or otherwise altered but still encodes functional protein (e.g., the upstream regulatory region can be altered to thereby alter the expression of the endogenous CRSP protein). In the homologous recombination vector, the altered portion of the CRSP gene is flanked at its 5' and 3' ends by additional nucleic acid of the CRSP gene to allow for homologous recombination to occur between the exogenous CRSP gene carried by the vector and an endogenous CRSP gene in a non-human embryonic stem cell. The additional flanking CRSP nucleic acid is of sufficient length for successful homologous recombination with the endogenous gene. Typically, several kilobases of flanking DNA (both at the 5' and 3' ends) are included in the vector (see e.g., Thomas, K.R. and Capecchi, M.R. (1987) Cell 51:503 for a description of homologous recombination vectors). The vector is introduced into a non-human embryonic stem cell line (e.g., by electroporation) and cells in which the introduced CRSP gene has homologously recombined with the endogenous CRSP gene are selected (see e.g., Li, E. et al., (1992) Cell 69:915). The selected cells are then injected into a blastocyst of a non-human animal (e.g., a mouse) to form aggregation chimeras (see e.g., Bradley, A. in Teratocarcinomas and Embryonic Stem Cells A Practical Approach, E.J. Robertson, ed. (IRL, Oxford, 1987) pp. 113-152. A non-human chimeric embryo can then be implanted into a suitable pseudopregnant female non-human foster animal and the embryo brought to term. Progeny harboring the homologously recombined DNA in their germ cells can be used to breed animals in which all cells of the animal contain the homologously recombined DNA by germline transmission of the transgene. Methods for constructing homologous recombination vectors and homologous recombinant animals are described further in Bradley, A. (1991) Current Opinion in Biotechnology 2:823-829 and in PCT International Publication Nos.: WO 90/11354 by Le Mouellec et al.; WO 91/01140 by Smithies et al; WO 92/0968 by Zijlstra et al.; and WO 93/04169 by Berns et al.

In another embodiment, transgenic non-human animals can be produced which contain selected systems which allow for regulated expression of the transgene. One example of such a system is the *cre*/*loxP* recombinase system of bacteriophage P1. For a description of the *cre*/*loxP* recombinase system, see, e.g., Lakso et al. (1992) PNAS 89:6232-6236. Another example of a recombinase system is the FLP recombinase system of *Saccharomyces cerevisiae* (O'Gorman et al. (1991) Science 251:1351-1355. If a *cre*/*loxP* recombinase system is used to regulate expression of the transgene, animals containing transgenes encoding both the *Cre* recombinase and a selected protein are required. Such animals can be provided through the construction of non-human "double" transgenic animals, e.g., by mating two non-human transgenic animals, one containing a transgene encoding a selected protein and the other containing a transgene encoding a recombinase.

Clones of the non-human transgenic animals described herein can also be produced according to the methods described in Wilmut, I. et al. (1997) Nature 385:810-813. In brief, a cell, e.g., a somatic cell, from the transgenic animal can be isolated and induced to exit the growth cycle and enter Go phase. The quiescent cell can then be fused, e.g., through the use of electrical pulses, to a non-human enucleated oocyte from an animal of the same species from which the quiescent cell is isolated. The reconstructed non-human oocyte is then cultured such that it develops to morula or blastocyte and then transferred to pseudopregnant female foster animal. The offspring borne of this female foster animal will be a clone of the animal from which the cell, e.g., the somatic cell, is isolated.

### IV. Pharmaceutical Compositions

The CRSP nucleic acid molecules, CRSP proteins, and anti-CRSP antibodies (also referred to herein as "active compounds") of the invention can be incorporated into pharmaceutical compositions suitable for administration. Such compositions typically comprise the nucleic acid molecule, protein, or antibody and a pharmaceutically acceptable carrier. As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

A pharmaceutical composition of the invention is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL^{™} (BASF, Parsippany, NJ) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (e.g., a CRSP protein or anti-CRSP antibody) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

The nucleic acid molecules of the invention can be inserted into vectors and used as gene therapy vectors. Gene therapy vectors can be delivered to a subject by, for example, intravenous injection, local administration (see U.S. Patent 5,328,470) or by stereotactic injection (see e.g., Chen et al. (1994) PNAS 91:3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells which produce the gene delivery system.

The pharmaceutical compositions can be included in a container, pack, or dispenser together with instructions for administration.

### V. Uses and Methods of the Invention

The molecules of the present invention (e.g., nucleic acid molecules, proteins, protein homologues, and antibodies described herein) can be used in one or more of the following methods: a) screening assays; b) predictive medicine (e.g., diagnostic assays, prognostic assays, monitoring clinical trials, and pharmacogenetics); and c) methods of treatment (e.g., therapeutic and prophylactic). As described herein, a CRSP protein of the invention has one or more of the following activities: intracellular calcium, an increase in phosphatidylinositol or other molecule, and can result, e.g., in phosphorylation of specific proteins, a modulation of gene transcription and any of the other biological activities set forth herein.

In a preferred embodiment, a CRSP activity is at least one or more of the following activities: (i) interaction of a CRSP protein with and/or binding to a second molecule, (e.g., a protein, such as a CRSP (e.g., CRSP-1) receptor, a soluble form of a CRSP receptor, a receptor for a member of the *wnt* family of signaling proteins, or a non-CRSP signaling molecule); (ii) interaction of a CRSP protein with an intracellular protein via a membrane-bound CRSP receptor; (iii) complex formation between a soluble CRSP protein and a second soluble CRSP binding partner (e.g., a non-CRSP protein molecule or a second CRSP protein molecule); (iv) interaction with other extracellular proteins (e.g., regulation of wnt-dependent cellular adhesion to extracellular matrix components); (v) binding to and eliminating an undesirable molecule (e.g., a detoxifying activity or defense function); and/or (vi) an enzymatic activity, and can can thus be used in, for example, (1) modulation of cellular signal transduction, either *in vitro or in vivo* (e.g., antagonism of the activity of members of the *wnt* family of secreted proteins or supression of *wnt*-dependent signal transduction); (2) regulation of communication between cells (e.g., regulation of *wnt*-dependent cell-cell interactions); (3) regulation of expression of genes whose expression is modulated by binding of CRSP (e.g., CRSP-1) to a receptor; (4) regulation of gene transcription in a cell involved in development or differentiation, either *in vitro or in vivo* (e.g., induction of cellular differentiation); (5) regulation of gene transcription in a cell involved in development or differentiation, wherein at least one gene encodes a differentiation-specific protein; (6) regulation of gene transcription in a cell involved in development or differentaition, wherein at least one gene encodes a second secreted protein; (7) regulation of gene transcription in a cell involved in development or differentiation, wherein at least one gene encodes a signal transduction molecule; (8) regulation of cellular proliferation, either *in vitro or in vivo* (e.g., induction of cellular proliferation or inhibition of proliferation as in the case of supression of tumorogenesis (e.g., supression of glioblastoma formation)); (9) formation and maintenance of ordered spatial arrangements of differentiated tissues in vertebrates, both adult and embryonic (e.g., induction of head formation during vertebrate development or maintenance of hematopoietic progenitor cells); (10) modulation of cell death, such as stimulation of cell survival; (11) regulating cell migration; and/or (12) immune modulation.

Accordingly one embodiment of the present invention involves a method of use (e.g., a diagnostic assay, prognostic assay, or a prophylactic/therapeutic method of treatment) wherein a molecule of the present invention (e.g., a CRSP protein, CRSP nucleic acid, or a CRSP modulator) is used, for example, to diagnose, prognose and/or treat a disease and/or condition in which any of the aforementioned activities (i.e., activities (i) - (vi) and (1) - (12) in the above paragraph) is indicated. In another embodiment, the present invention involves a method of use (e.g., a diagnostic assay, prognostic assay, or manufacture of a medicament for a prophylactic/therapeutic method of treatment) wherein a molecule of the present invention (e.g. a CRSP protein, CRSP nucleic acid, or a CRSP modulator) is used, for example, in the manufacture of a medicament for the diagnosis, prognosis, and/or treatment of subjects, preferably a human subject, in which any of the aforementioned activities is pathologically perturbed. In a preferred embodiment, the methods of use (e.g., diagnostic assays, prognostic assays, or manufacture of medicaments for prophylactic/therapeutic methods of treatment) involve production of a medicament for administering to a subject, preferably a human subject, a molecule of the present invention (e.g., a CRSP protein, CRSP nucleic acid, or a CRSP modulator) for the diagnosis, prognosis, and/or therapeutic treatment. In another embodiment, the methods of use (e.g., diagnostic assays, prognostic assays, or manufacture of medicaments for prophylactic/therapeutic methods of treatment) involve production of a medicament for administering to a human subject a molecule of the present invention (e.g., a CRSP protein, CRSP nucleic acid, or a CRSP modulator).

Other embodiments of the invention pertain to the use of isolated nucleic acid molecules of the invention which can be used, for example, to express CRSP protein (e.g., via a recombinant expression vector in a host cell in gene therapy applications), to detect CRSP mRNA (e.g., in a biological sample) or a genetic alteration in a CRSP gene, and to modulate CRSP activity, as described further below. In addition, the CRSP proteins can be used to screen drugs or compounds which modulate the CRSP activity as well as to treat disorders characterized by insufficient or excessive production of CRSP protein or production of CRSP protein forms which have decreased or aberrant activity compared to CRSP wild type protein (e.g., developmental disorders or proliferative diseases such as cancer as well as diseases, conditions or disorders characterized by abnormal cell differentiation and/or survival, an abnormal extracellular structure, or an abnormality in a defense mechanism). Moreover, the anti-CRSP antibodies of the invention can be used to detect and isolate CRSP proteins, regulate the bioavailability of CRSP proteins, and modulate CRSP activity. The term "an aberrant activity", as applied to an activity of a protein such as CRSP (e.g., CRSP-1), refers to an activity which differs from the activity of the wild-type or native protein or which differs from the activity of the protein in a healthy subject. An activity of a protein can be aberrant because it is stronger than the activity of its native counterpart. Alternatively, an activity can be aberrant because it is weaker or absent related to the activity of its native counterpart. An aberrant activity can also be a change in an activity. For example an aberrant protein can interact with a different protein relative to its native counterpart. A cell can have an aberrant CRSP (e.g., CRSP-1) activity due to overexpression or underexpression of the gene encoding CRSP.

### A. Screening Assays:

The invention provides a method (also referred to herein as a "screening assay") for identifying modulators, i.e., candidate or test compounds or agents (e.g., peptides, peptidomimetics, small molecules or other drugs) which bind to CRSP proteins or have a stimulatory or inhibitory effect on, for example, CRSP expression or CRSP activity. Modulators can include, for example, CRSP agonist and/or CRSP antagonists. The term "agonist", as used herein, is meant to refer to an agent that mimics or upregulates (e.g. potentiates or supplements) a CRSP (e.g., CRSP-1) bioactivity. A CRSP agonist can be a compound which mimics a bioactivity of a CRSP protein, such as transduction of a signal from a CRSP receptor, by, e.g., interacting with a CRSP-1 receptor. A CRSP agonist can also be a compound that upregulates expression of a CRSP gene. A CRSP agonist can also be a compound which modulates the expression or activity of a protein which is located downstream of a CRSP receptor, thereby mimicking or enhancing the effect of binding of CRSP to a CRSP receptor.

"Antagonist" as used herein is meant to refer to an agent that inhibits, decreases or suppresses a CRSP (e.g., CRSP-1) bioactivity. An antagonist can be a compound which decreases signalling from a CRSP protein, e.g., a compound that is capable of binding to CRSP-1 or to a CRSP-1 receptor. A preferred CRSP antagonist inhibits the interaction between a CRSP protein and another molecule, such as a CRSP receptor. Alternatively, a CRSP antagonist can be a compound that down regulates expression of a CRSP gene. A CRSP antagonist can also be a compound which modulates the expression or activity of a protein which is located downstream of a CRSP receptor, thereby antagonizing the effect of binding of CRSP to a CRSP receptor.

In one embodiment, the invention provides assays for screening candidate or test compounds which bind to or modulate the activity of a CRSP protein or polypeptide or biologically active portion thereof. In another embodiment, the invention provides assays for screening candidate or test compounds which bind to or modulate the activity of a CRSP receptor. The test compounds of the present invention can be obtained using any of the numerous approaches in combinatorial library methods known in the art, including: biological libraries; spatially addressable parallel solid phase or solution phase libraries; synthetic library methods requiring deconvolution; the 'one-bead one-compound' library method; and synthetic library methods using affinity chromatography selection. The biological library approach is limited to peptide libraries, while the other four approaches are applicable to peptide, non-peptide oligomer or small molecule libraries of compounds (Lam, K.S. (1997) *Anticancer Drug Des.* 12:145).

Examples of methods for the synthesis of molecular libraries can be found in the art, for example in: DeWitt et al. (1993) Proc. Natl. Acad. Sci. U.S.A. 90:6909; Erb et al. (1994) Proc. Natl. Acad. Sci. USA 91:11422; Zuckermann et al. (1994). J Med. Chem. 37:2678; Cho et al. (1993) Science 261:1303; Carrell et al. (1994) Angew. Chem. Int. Ed. Engl. 33:2059; Carell et al. (1994) Angew. Chem. Int. Ed. Engl. 33:2061; and in Gallop et al. (1994) J Med. Chem. 37:1233.

Libraries of compounds may be presented in solution (e.g., Houghten (1992) Biotechniques 13:412-421), or on beads (Lam (1991) Nature 354:82-84), chips (Fodor (1993) Nature 364:555-556), bacteria (Ladner USP 5,223,409), spores (Ladner USP '409), plasmids (Cull et al. (1992) Proc Natl Acad Sci USA 89:1865-1869) or on phage (Scott and Smith (1990) Science 249:386-390); (Devlin (1990) Science 249:404-406); (Cwirla et al. (1990) Proc. Natl. Acad Sci. 87:6378-6382); (Felici (1991) J Mol. Biol. 222:301-310); (Ladner *supra.).*

In one embodiment, an assay is a cell-based assay in which a cell which expresses a CRSP receptor on the cell surface is contacted with a test compound and the ability of the test compound to bind to a CRSP receptor determined. The cell, for example, can be of mammalian origin or a yeast cell. Determining the ability of the test compound to bind to a CRSP receptor can be accomplished, for example, by coupling the test compound with a radioisotope or enzymatic label such that binding of the test compound to the CRSP receptor can be determined by detecting the labeled compound in a complex. For example, test compounds can be labeled with ¹²⁵I, ³⁵S, ¹⁴C, or ³H, either directly or indirectly, and the radioisotope detected by direct counting of radioemmission or by scintillation counting. Alternatively, test compounds can be enzymatically labeled with, for example, horseradish peroxidase, alkaline phosphatase, or luciferase, and the enzymatic label detected by determination of conversion of an appropriate substrate to product.

It is also within the scope of this invention to determine the ability of a test compound to interact with a CRSP receptor without the labeling of any of the interactants. For example, a microphysiometer can be used to detect the interaction of a test compound with a CRSP receptor without the labeling of either the test compound or the receptor. McConnell, H. M. et al. (1992) Science 257:1906-1912. As used herein, a "microphysiometer" (e.g., Cytosensor^{™}) is an analytical instrument that measures the rate at which a cell acidifies its environment using a light-addressable potentiometric sensor (LAPS). Changes in this acidification rate can be used as an indicator of the interaction between ligand and receptor.

In a preferred embodiment, the assay comprises contacting a cell which expresses a CRSP receptor on the cell surface with a CRSP protein or biologically-active portion thereof, to form an assay mixture, contacting the assay mixture with a test compound, and determining the ability of the test compound to interact with a CRSP receptor, wherein determining the ability of the test compound to interact with a CRSP receptor comprises determining the ability of the test compound to preferentially bind to the CRSP receptor as compared to the ability of CRSP, or a biologically active portion thereof, to bind to the receptor.

In another embodiment, an assay is a cell-based assay comprising contacting a cell expressing a CRSP target molecule with a test compound and determining the ability of the test compound to modulate (e.g. stimulate or inhibit) the activity of the CRSP target molecule. Determining the ability of the test compound to modulate the activity of a CRSP target molecule can be accomplished, for example, by determining the ability of the CRSP protein to bind to or interact with the CRSP target molecule.

Determining the ability of the CRSP protein to bind to or interact with a CRSP target molecule can be accomplished by one of the methods described above for determining direct binding. In a preferred embodiment, determining the ability of the CRSP protein to bind to or interact with a CRSP target molecule can be accomplished by determining the activity of the target molecule. For example, the activity of the target molecule can be determined by detecting induction of a cellular second messenger of the target (i.e. intracellular Ca²⁺, diacylglycerol, IP₃, etc.), detecting catalytic/enzymatic activity of the target an appropriate substrate, detecting the induction of a reporter gene (comprising a CRSP-responsive regulatory element operatively linked to a nucleic acid encoding a detectable marker, e.g., luciferase), or detecting a cellular response, for example, development, differentiation or rate of proliferation.

In yet another embodiment, an assay of the present invention is a cell-free assay in which a CRSP protein or biologically active portion thereof is contacted with a test compound and the ability of the test compound to bind to the CRSP protein or biologically active portion thereof is determined. Binding of the test compound to the CRSP protein can be determined either directly or indirectly as described above. In a preferred embodiment, the assay includes contacting the CRSP protein or biologically active portion thereof with a known compound which binds CRSP to form an assay mixture, contacting the assay mixture with a test compound, and determining the ability of the test compound to interact with a CRSP protein, wherein determining the ability of the test compound to interact with a CRSP protein comprises determining the ability of the test compound to preferentially bind to CRSP or biologically active portion thereof as compared to the known compound.

In another embodiment, the assay is a cell-free assay in which a CRSP protein or biologically active portion thereof is contacted with a test compound and the ability of the test compound to modulate (e.g., stimulate or inhibit) the activity of the CRSP protein or biologically active portion thereof is determined. Determining the ability of the test compound to modulate the activity of a CRSP protein can be accomplished, for example, by determining the ability of the CRSP protein to bind to a CRSP target molecule by one of the methods described above for determining direct binding. Determining the ability of the CRSP protein to bind to a CRSP target molecule can also be accomplished using a technology such as real-time Biomolocular Interaction Analysis (BIA). Sjolander, S. and Urbaniczky, C. (1991) Anal. Chem. 63:2338-2345 and Szabo et al. (1995) Curr. Opin. Struct. Biol. 5:699-705. As used herein, "BIA" is a technology for studying biospecific interactions in real time, without labeling any of the interactants (e.g., BIAcore^{™}). Changes in the optical phenomenon surface plasmon resonance (SPR) can be used as an indication of real-time reactions between biological molecules.

In an alternative embodiment, determining the ability of the test compound to modulate the activity of a CRSP protein can be accomplished by determining the ability of the CRSP protein to further modulate the activity of a CRSP target molecule. For example, the catalytic/enzymatic activity of the target molecule on an appropriate substrate can be determined as previously described.

In yet another embodiment, the cell-free assay involves contacting a CRSP protein or biologically active portion thereof with a known compound which binds the CRSP protein to form an assay mixture, contacting the assay mixture with a test compound, and determining the ability of the test compound to interact with the CRSP protein, wherein determining the ability of the test compound to interact with the CRSP protein comprises determining the ability of the CRSP protein to preferentially bind to or modulate the activity of a CRSP target molecule.

In many drug screening programs which test libraries of compounds and natural extracts, high throughput assays are desirable in order to maximize the number of compounds surveyed in a given period of time. Assays which are performed in cell-free systems, such as may be derived with purified or semi-purified proteins, are often preferred as "primary" screens in that they can be generated to permit rapid development and relatively easy detection of an alteration in a molecular target which is mediated by a test compound. Moreover, the effects of cellular toxicity and/or bioavailability of the test compound can be generally ignored in the *in vitro* system, the assay instead being focused primarily on the effect of the drug on the molecular target as may be manifest in an alteration of binding affinity with upstream or downstream elements. Accordingly, in an exemplary screening assay of the present invention, the compound of interest is contacted with a CRSP (e.g., CRSP-1) protein or a CRSP (e.g., CRSP-1) binding partner, e.g., a receptor. The receptor can be soluble or the receptor can be present on a cell surface. To the mixture of the compound and the CRSP protein or CRSP binding partner is then added a composition containing a CRSP binding partner or a CRSP protein, respectively. Detection and quantification of complexes of CRSP proteins and CRSP binding partners provide a means for determining a compound's efficacy at inhibiting (or potentiating) complex formation between CRSP and a binding partner. The efficacy of the compound can be assessed by generating dose response curves from data obtained using various concentrations of the test compound. Moreover, a control assay can also be performed to provide a baseline for comparison. In the control assay, isolated and purified CRSP polypeptide or binding partner is added to a composition containing the CRSP binding partner or CRSP polypeptide, and the formation of a complex is quantitated in the absence of the test compound.

The cell-free assays of the present invention are amenable to use of both soluble and/or membrane-bound forms of isolated proteins (e.g. CRSP proteins or biologically active portions thereof or CRSP target molecules). In the case of cell-free assays in which a membrane-bound form an isolated protein is used (e.g., a CRSP target molecule or receptor) it may be desirable to utilize a solubilizing agent such that the membrane-bound form of the isolated protein is maintained in solution. Examples of such solubilizing agents include non-ionic detergents such as n-octylglucoside, n-dodecylglucoside, n-dodecylmaltoside, octanoyl-N-methylglucamide, decanoyl-N-methylglucamide, Triton^{®} X-100, Triton^{®} X-114, Thesit^{®}, Isotridecypoly(ethylene glycol ether)ₙ, 3-[(3-cholamidopropyl)dimethylamminio]-1-propane sulfonate (CHAPS), 3-[(3-cholamidopropyl)dimethylamminio]-2-hydroxy-1-propane sulfonate (CHAPSO), or N-dodecyl=N,N-dimethyl-3-ammonio-1-propane sulfonate.

In more than one embodiment of the above assay methods of the present invention, it may be desirable to immobilize either CRSP or its target molecule to facilitate separation of complexed from uncomplexed forms of one or both of the proteins, as well as to accommodate automation of the assay. Binding of a test compound to a CRSP protein, or interaction of a CRSP protein with a target molecule in the presence and absence of a candidate compound, can be accomplished in any vessel suitable for containing the reactants. Examples of such vessels include microtitre plates, test tubes, and micro-centrifuge tubes. In one embodiment, a fusion protein can be provided which adds a domain that allows one or both of the proteins to be bound to a matrix. For example, glutathione-S-transferase/ CRSP fusion proteins or glutathione-S-transferase/target fusion proteins can be adsorbed onto glutathione sepharose beads (Sigma Chemical, St. Louis, MO) or glutathione derivatized microtitre plates, which are then combined with the test compound or the test compound and either the non-adsorbed target protein or CRSP protein, and the mixture incubated under conditions conducive to complex formation (e.g., at physiological conditions for salt and pH). Following incubation, the beads or microtitre plate wells are washed to remove any unbound components, the matrix immobilized in the case of beads, complex determined either directly or indirectly, for example, as described above. Alternatively, the complexes can be dissociated from the matrix, and the level of CRSP binding or activity determined using standard techniques.

Other techniques for immobilizing proteins on matrices can also be used in the screening assays of the invention. For example, either a CRSP protein or a CRSP target molecule can be immobilized utilizing conjugation of biotin and streptavidin. Biotinylated CRSP protein or target molecules can be prepared from biotin-NHS (N-hydroxy-succinimide) using techniques well known in the art (e.g., biotinylation kit, Pierce Chemicals, Rockford, IL), and immobilized in the wells of streptavidin-coated 96 well plates (Pierce Chemical). Alternatively, antibodies reactive with CRSP protein or target molecules but which do not interfere with binding of the CRSP protein to its target molecule can be derivatized to the wells of the plate, and unbound target or CRSP protein trapped in the wells by antibody conjugation. Methods for detecting such complexes, in addition to those described above for the GST-immobilized complexes, include immunodetection of complexes using antibodies reactive with the CRSP protein or target molecule, as well as enzyme-linked assays which rely on detecting an enzymatic activity associated with the CRSP protein or target molecule.

In another embodiment, modulators of CRSP expression are identified in a method wherein a cell is contacted with a candidate compound and the expression of CRSP mRNA or protein in the cell is determined. The level of expression of CRSP mRNA or protein in the presence of the candidate compound is compared to the level of expression of CRSP mRNA or protein in the absence of the candidate compound. The candidate compound can then be identified as a modulator of CRSP expression based on this comparison. For example, when expression of CRSP mRNA or protein is greater (statistically significantly greater) in the presence of the candidate compound than in its absence, the candidate compound is identified as a stimulator of CRSP mRNA or protein expression. Alternatively, when expression of CRSP mRNA or protein is less (statistically significantly less) in the presence of the candidate compound than in its absence, the candidate compound is identified as an inhibitor of CRSP mRNA or protein expression. The level of CRSP mRNA or protein expression in the cells can be determined by methods described herein for detecting CRSP mRNA or protein.

In yet another aspect of the invention, the CRSP proteins can be used as "bait proteins" in a two-hybrid assay or three-hybrid assay (see, e.g., U.S. Patent No. 5,283,317; Zervos et al. (1993) Cell 72:223-232; Madura et al. (1993) J. Biol. Chem. 268:12046-12054; Bartel et al. (1993) Biotechniques 14:920-924; Iwabuchi et al. (1993) Oncogene 8:1693-1696; and Brent WO94/10300), to identify other proteins, which bind to or interact with CRSP ("CRSP-binding proteins" or "CRSP-bp") and modulate CRSP activity. Such CRSP-binding proteins are also likely to be involved in the propagation of signals by the CRSP proteins as, for example, downstream elements of a CRSP-mediated signaling pathway. Alternatively, such CRSP-binding proteins are likely to be cell-surface molecules associated with non-CRSP expressing cells, wherein such CRSP-binding proteins are involved in signal transduction.

The two-hybrid system is based on the modular nature of most transcription factors, which consist of separable DNA-binding and activation domains. Briefly, the assay utilizes two different DNA constructs. In one construct, the gene that codes for a CRSP protein is fused to a gene encoding the DNA binding domain of a known transcription factor (e.g., GAL-4). In the other construct, a DNA sequence, from a library of DNA sequences, that encodes an unidentified protein ("prey" or "sample") is fused to a gene that codes for the activation domain of the known transcription factor. If the "bait" and the "prey" proteins are able to interact, *in vivo,* forming a CRSP-dependent complex, the DNA-binding and activation domains of the transcription factor are brought into close proximity. This proximity allows transcription of a reporter gene (e.g., LacZ) which is operably linked to a transcriptional regulatory site responsive to the transcription factor. Expression of the reporter gene can be detected and cell colonies containing the functional transcription factor can be isolated and used to obtain the cloned gene which encodes the protein which interacts with the CRSP protein.

This invention further pertains to novel agents identified by the above-described screening assays and to processes for producing such agents by use of these assays. Accordingly, in one embodiment, the present invention includes a compound or agent obtainable by a method comprising the steps of any one of the aformentioned screening assays (e.g., cell-based assays or cell-free assays). For example, in one embodiment, the invention includes a compound or agent obtainable by a method comprising contacting a cell which expresses a CRSP target molecule with a test compound and the determining the ability of the test compound to bind to, or modulate the activity of, the CRSP target molecule. In another embodiment, the invention includes a compound or agent obtainable by a method comprising contacting a cell which expresses a CRSP target molecule with a CRSP protein or biologically-active portion thereof, to form an assay mixture, contacting the assay mixture with a test compound, and determining the ability of the test compound to interact with, or modulate the activity of, the CRSP target molecule. In another embodiment, the invention includes a compound or agent obtainable by a method comprising contacting a CRSP protein or biologically active portion thereof with a test compound and determining the ability of the test compound to bind to, or modulate (e.g., stimulate or inhibit) the activity of, the CRSP protein or biologically active portion thereof. In yet another embodiment, the present invention included a compound or agent obtainable by a method comprising contacting a CRSP protein or biologically active portion thereof with a known compound which binds the CRSP protein to form an assay mixture, contacting the assay mixture with a test compound, and determining the ability of the test compound to interact with, or modulate the activity of the CRSP protein.

Accordingly, it is within the scope of this invention to further use an agent identified as described herein in an appropriate animal model. For example, an agent identified as described herein (e.g., a CRSP modulating agent, an antisense CRSP nucleic acid molecule, a CRSP-specific antibody, or a CRSP-binding partner) can be used in an animal model to determine the efficacy, toxicity, or side effects of treatment with such an agent. Alternatively, an agent identified as described herein can be used in an animal model to determine the mechanism of action of such an agent.

The present inventon also pertains to uses of novel agents identified by the above-described screening assays for diagnoses, prognoses, and treatments as described herein. Accordingly, it is within the scope of the present invention to use such agents in the design, formulation, synthesis, manufacture, and/or production of a drug or pharmaceutical composition for use in diagnosis, prognosis, or treatment, as described herein. For example, in one embodiment, the present invention includes a method of synthesizing or producing a drug or pharmaceutical composition by reference to the structure and/or properties of a compound obtainable by one of the above-described screening assays. For example, a drug or pharmaceutical composition can be synthesized based on the structure and/or properties of a compound obtained by a method in which a cell which expresses a CRSP target molecule is contacted with a test compound and the ability of the test compound to bind to, or modulate the activity of, the CRSP target molecule is determined. In another exemplary embodiment, the present invention includes a method of synthesizing or producing a drug or pharmaceutical composition based on the structure and/or properties of a compound obtainable by a method in which a CRSP protein or biologically active portion thereof is contacted with a test compound and the ability of the test compound to bind to, or modulate (e.g., stimulate or inhibit) the activity of, the CRSP protein or biologically active portion thereof is determined.

### B. Detection Assays

Portions or fragments of the cDNA sequences identified herein (and the corresponding complete gene sequences) can be used in numerous ways as polynucleotide reagents. For example, these sequences can be used to: (i) map their respective genes on a chromosome; and, thus, locate gene regions associated with genetic disease; (ii) identify an individual from a minute biological sample (tissue typing); and (iii) aid in forensic identification of a biological sample. These applications are described in the subsections below.

### 1. Chromosome Mapping

Once the sequence (or a portion of the sequence) of a gene has been isolated, this sequence can be used to map the location of the gene on a chromosome. This process is called chromosome mapping. Accordingly, portions or fragments of the CRSP nucleotide sequences, described herein, can be used to map the location of the CRSP genes on a chromosome. The mapping of the CRSP sequences to chromosomes is an important first step in correlating these sequences with genes associated with disease.

Briefly, CRSP genes can be mapped to chromosomes by preparing PCR primers (preferably 15-25 bp in length) from the CRSP nucleotide sequences. Computer analysis of the CRSP sequences can be used to predict primers that do not span more than one exon in the genomic DNA, thus complicating the amplification process. These primers can then be used for PCR screening of somatic cell hybrids containing individual human chromosomes. Only those hybrids containing the human gene corresponding to the CRSP sequences will yield an amplified fragment.

Somatic cell hybrids are prepared by fusing somatic cells from different mammals (e.g., human and mouse cells). As hybrids of human and mouse cells grow and divide, they gradually lose human chromosomes in random order, but retain the mouse chromosomes. By using media in which mouse cells cannot grow, because they lack a particular enzyme, but human cells can, the one human chromosome that contains the gene encoding the needed enzyme, will be retained. By using various media, panels of hybrid cell lines can be established. Each cell line in a panel contains either a single human chromosome or a small number of human chromosomes, and a full set of mouse chromosomes, allowing easy mapping of individual genes to specific human chromosomes. (D'Eustachio P. et al. (1983) Science 220:919-924). Somatic cell hybrids containing only fragments of human chromosomes can also be produced by using human chromosomes with translocations and deletions.

PCR mapping of somatic cell hybrids is a rapid procedure for assigning a particular sequence to a particular chromosome. Three or more sequences can be assigned per day using a single thermal cycler. Using the CRSP nucleotide sequences to design oligonucleotide primers, sublocalization can be achieved with panels of fragments from specific chromosomes. Other mapping strategies which can similarly be used to map a 9o, 1p, or 1v sequence to its chromosome include *in situ* hybridization (described in Fan, Y. et al. (1990) PNAS, 87:6223-27), pre-screening with labeled flow-sorted chromosomes, and pre-selection by hybridization to chromosome specific cDNA libraries.

Fluorescence *in situ* hybridization (FISH) of a DNA sequence to a metaphase chromosomal spread can further be used to provide a precise chromosomal location in one step. Chromosome spreads can be made using cells whose division has been blocked in metaphase by a chemical such as colcemid that disrupts the mitotic spindle. The chromosomes can be treated briefly with trypsin, and then stained with Giemsa. A pattern of light and dark bands develops on each chromosome, so that the chromosomes can be identified individually. The FISH technique can be used with a DNA sequence as short as 500 or 600 bases. However, clones larger than 1,000 bases have a higher likelihood of binding to a unique chromosomal location with sufficient signal intensity for simple detection. Preferably 1,000 bases, and more preferably 2,000 bases will suffice to get good results at a reasonable amount of time. For a review of this technique, see Verma et al., Human Chromosomes: A Manual of Basic Techniques (Pergamon Press, New York 1988).

Reagents for chromosome mapping can be used individually to mark a single chromosome or a single site on that chromosome, or panels of reagents can be used for marking multiple sites and/or multiple chromosomes. Reagents corresponding to noncoding regions of the genes actually are preferred for mapping purposes. Coding sequences are more likely to be conserved within gene families, thus increasing the chance of cross hybridizations during chromosomal mapping.

Once a sequence has been mapped to a precise chromosomal location, the physical position of the sequence on the chromosome can be correlated with genetic map data. (Such data are found, for example, in V. McKusick, Mendelian Inheritance in Man, available on-line through Johns Hopkins University Welch Medical Library). The relationship between a gene and a disease, mapped to the same chromosomal region, can then be identified through linkage analysis (co-inheritance of physically adjacent genes), described in, for example, Egeland, J. et al. (1987) Nature, 325:783-787.

Moreover, differences in the DNA sequences between individuals affected and unaffected with a disease associated with the CRSP gene, can be determined. If a mutation is observed in some or all of the affected individuals but not in any unaffected individuals, then the mutation is likely to be the causative agent of the particular disease. Comparison of affected and unaffected individuals generally involves first looking for structural alterations in the chromosomes, such as deletions or translocations that are visible from chromosome spreads or detectable using PCR based on that DNA sequence. Ultimately, complete sequencing of genes from several individuals can be performed to confirm the presence of a mutation and to distinguish mutations from polymorphisms.

### 2. Tissue Typing

The CRSP sequences of the present invention can also be used to identify individuals from minute biological samples. The United States military, for example, is considering the use of restriction fragment length polymorphism (RFLP) for identification of its personnel. In this technique, an individual's genomic DNA is digested with one or more restriction enzymes, and probed on a Southern blot to yield unique bands for identification. This method does not suffer from the current limitations of "Dog Tags" which can be lost, switched, or stolen, making positive identification difficult. The sequences of the present invention are useful as additional DNA markers for RFLP (described in U.S. Patent 5,272,057).

Furthermore, the sequences of the present invention can be used to provide an alternative technique which determines the actual base-by-base DNA sequence of selected portions of an individual's genome. Thus, the CRSP nucleotide sequences described herein can be used to prepare two PCR primers from the 5' and 3' ends of the sequences. These primers can then be used to amplify an individual's DNA and subsequently sequence it.

Panels of corresponding DNA sequences from individuals, prepared in this manner, can provide unique individual identifications, as each individual will have a unique set of such DNA sequences due to allelic differences. The sequences of the present invention can be used to obtain such identification sequences from individuals and from tissue. The CRSP nucleotide sequences of the invention uniquely represent portions of the human genome. Allelic variation occurs to some degree in the coding regions of these sequences, and to a greater degree in the noncoding regions. It is estimated that allelic variation between individual humans occurs with a frequency of about once per each 500 bases. Each of the sequences described herein can, to some degree, be used as a standard against which DNA from an individual can be compared for identification purposes. Because greater numbers of polymorphisms occur in the noncoding regions, fewer sequences are necessary to differentiate individuals. The noncoding sequences of SEQ ID NO:1, SEQ ID NO:4, SEQ ID NO:7, or SEQ ID NO:10, can comfortably provide positive individual identification with a panel of perhaps 10 to 1,000 primers which each yield a noncoding amplified sequence of 100 bases. If predicted coding sequences, such as those in SEQ ID NO:3, SEO ID NO:6, SEQ ID NO:9, or SEQ ID NO:12 are used, a more appropriate number of primers for positive individual identification would be 500-2,000.

If a panel of reagents from CRSP nucleotide sequences described herein is used to generate a unique identification database for an individual, those same reagents can later be used to identify tissue from that individual. Using the unique identification database, positive identification of the individual, living or dead, can be made from extremely small tissue samples.

### 3. Use of Partial CRSP Sequences in Forensic Biology

DNA-based identification techniques can also be used in forensic biology. Forensic biology is a scientific field employing genetic typing of biological evidence found at a crime scene as a means for positively identifying, for example, a perpetrator of a crime. To make such an identification, PCR technology can be used to amplify DNA sequences taken from very small biological samples such as tissues, e.g., hair or skin, or body fluids, e.g., blood, saliva, or semen found at a crime scene. The amplified sequence can then be compared to a standard, thereby allowing identification of the origin of the biological sample.

The sequences of the present invention can be used to provide polynucleotide reagents, e.g., PCR primers, targeted to specific loci in the human genome, which can enhance the reliability of DNA-based forensic identifications by, for example, providing another "identification marker" (i.e. another DNA sequence that is unique to a particular individual). As mentioned above, actual base sequence information can be used for identification as an accurate alternative to patterns formed by restriction enzyme generated fragments. Sequences targeted to noncoding regions of the nucleotide sequences of the invention are particularly appropriate for this use as greater numbers of polymorphisms occur in the noncoding regions, making it easier to differentiate individuals using this technique. Examples of polynucleotide reagents include the CRSP nucleotide sequences or portion thereof e.g., fragments derived from the noncoding regions of the nucleotide sequences of the invention, having a length of at least 20 bases, preferably at least 30 bases.

The CRSP nucleotide sequences described herein can further be used to provide polynucleotide reagents, e.g., labeled or labelable probes which can be used in, for example, an *in situ* hybridization technique, to identify a specific tissue, e.g., brain tissue. This can be very useful in cases where a forensic pathologist is presented with a tissue of unknown origin. Panels of such CRSP probes can be used to identify tissue by species and/or by organ type.

In a similar fashion, these reagents, e.g., CRSP primers or probes can be used to screen tissue culture for contamination (i.e. screen for the presence of a mixture of different types of cells in a culture).

### C. Predictive Medicine:

The present invention also pertains to the field of predictive medicine in which diagnostic assays, prognostic assays, and monitoring clinical trails are used for prognostic, (predictive) purposes to thereby treat an individual prophylactically. Accordingly, one aspect of the present invention relates to diagnostic assays for determining CRSP protein and/or nucleic acid expression as well as CRSP activity, in the context of a biological sample (e.g., blood, serum, cells, tissue) to thereby determine whether an individual is afflicted with a disease or disorder, or is at risk of developing a disorder, associated with aberrant CRSP expression or activity, such as aberrant cell proliferation, differentiation, and/or survival resulting for example in a a neurodegenerative disease or cancer. The invention also provides for prognostic (or predictive) assays for determining whether an individual is at risk of developing a disorder associated with CRSP protein, nucleic acid expression or activity. For example, mutations in a CRSP gene can be assayed in a biological sample. Such assays can be used for prognostic or predictive purpose to thereby phophylactically treat an individual prior to the onset of a disorder characterized by or associated with CRSP protein, nucleic acid expression or activity.

Another use of the invention pertains to monitoring the influence of agents (e.g., drugs, compounds) on the expression or activity of CRSP in clinical trials.

These and other agents are described in further detail in the following sections.

### 1. Diagnostic Assays

An exemplary method for detecting the presence or absence of CRSP protein or nucleic acid in a biological sample involves obtaining a biological sample from a test subject and contacting the biological sample with a compound or an agent capable of detecting CRSP protein or nucleic acid (e.g., mRNA, genomic DNA) that encodes CRSP protein such that the presence of CRSP protein or nucleic acid is detected in the biological sample. A preferred agent for detecting CRSP mRNA or genomic DNA is a labeled nucleic acid probe capable of hybridizing to CRSP mRNA or genomic DNA. The nucleic acid probe can be, for example, a full-length CRSP nucleic acid, such as a nucleic acid of the invention, or a portion thereof, such as an oligonucleotide of at least 15, 30, 50, 100, 250 or 500 nucleotides in length and sufficient to specifically hybridize under stringent conditions to CRSP mRNA or genomic DNA. Other suitable probes for use in the diagnostic assays of the invention are described herein.

A preferred agent for detecting CRSP protein is an antibody capable of binding to CRSP protein, preferably an antibody with a detectable label. Antibodies can be polyclonal, or more preferably, monoclonal. An intact antibody, or a fragment thereof (e.g., Fab or F(ab')₂) can be used. The term "labeled", with regard to the probe or antibody, is intended to encompass direct labeling of the probe or antibody by coupling (i.e., physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin. The term "biological sample" is intended to include tissues, cells and biological fluids isolated from a subject, as well as tissues, cells and fluids present within a subject. That is, the detection method of the invention can be used to detect CRSP mRNA, protein, or genomic DNA in a biological sample *in vitro* as well as *in vivo*. For example, *in vit*ro techniques for detection of CRSP mRNA include Northern hybridizations and *in situ* hybridizations. *In vitro* techniques for detection of CRSP protein include enzyme linked immunosorbent assays (ELISAs), Western blots, immunoprecipitations and immunofluorescence. *In vitro* techniques for detection of CRSP genomic DNA include Southern hybridizations. Furthermore, *in vivo* techniques for detection of CRSP protein include introducing into a subject a labeled anti-CRSP antibody. For example, the antibody can be labeled with a radioactive marker whose presence and location in a subject can be detected by standard imaging techniques.

In one embodiment, the biological sample contains protein molecules from the test subject. Alternatively, the biological sample can contain mRNA molecules from the test subject or genomic DNA molecules from the test subject. A preferred biological sample is a serum sample isolated by conventional means from a subject.

In another embodiment, the methods further involve obtaining a control biological sample from a control subject, contacting the control sample with a compound or agent capable of detecting CRSP protein, mRNA, or genomic DNA, such that the presence of CRSP protein, mRNA or genomic DNA is detected in the biological sample, and comparing the presence of CRSP protein, mRNA or genomic DNA in the control sample with the presence of CRSP protein, mRNA or genomic DNA in the test sample.

The invention also encompasses kits for detecting the presence of CRSP in a biological sample. For example, the kit can comprise a labeled compound or agent capable of detecting CRSP protein or mRNA in a biological sample; means for determining the amount of CRSP in the sample; and means for comparing the amount of CRSP in the sample with a standard. The compound or agent can be packaged in a suitable container. The kit can further comprise instructions for using the kit to detect CRSP protein or nucleic acid.

### 2. Prognostic Assays

The diagnostic methods described herein can furthermore be utilized to identify subjects having or at risk of developing a disease or disorder associated with aberrant CRSP expression or activity. For example, the assays described herein, such as the preceding diagnostic assays or the following assays, can be utilized to identify a subject having or at risk of developing a disorder associated with CRSP protein, nucleic acid expression or activity such as a proliferative disorder, a differentiative or developmental disorder, a hematopoietic disorder as well as diseases, conditions or disorders characterized by abnormal cell survival, abnormal extracellular structure, or an abnormality in a defense mechanism. Alternatively, the prognostic assays can be utilized to identify a subject having or at risk for developing a differentiative or proliferative disease (e.g., cancer). Thus, the present invention provides a method for identifying a disease or disorder associated with aberrant CRSP expression or activity in which a test sample is obtained from a subject and CRSP protein or nucleic acid (e.g, mRNA, genomic DNA) is detected, wherein the presence of CRSP protein or nucleic acid is diagnostic for a subject having or at risk of developing a disease or disorder associated with aberrant CRSP expression or activity. As used herein, a "test sample" refers to a biological sample obtained from a subject of interest. For example, a test sample can be a biological fluid (e.g., serum), cell sample, or tissue.

Furthermore, the prognostic assays described herein can be used to determine whether a subject can be administered an agent (e.g., an agonist, antagonist, peptidomimetic, protein, peptide, nucleic acid, small molecule, or other drug candidate) to treat a disease or disorder associated with aberrant CRSP expression or activity. For example, such methods can be used to determine whether a subject can be effectively treated with an agent for a disorder, such as a proliferative disorder, a differentiative or developmental disorder, a hematopoietic disorder, as well disorders characterized by abnormal cell survival, an abnormal extracellular structure, or an abnormality in a defense mechanism. Alternatively, such methods can be used to determine whether a subject can be effectively treated with an agent for a differentiative or proliferative disease (e.g., cancer). Thus, the present invention provides methods for determining whether a subject can be effectively treated with an agent for a disorder associated with aberrant CRSP expression or activity in which a test sample is obtained and CRSP protein or nucleic acid expression or activity is detected (e.g., wherein the abundance of CRSP protein or nucleic acid expression or activity is diagnostic for a subject that can be administered the agent to treat a disorder associated with aberrant CRSP expression or activity.)

The methods of the invention can also be used to detect genetic alterations in a CRSP gene, thereby determining if a subject with the altered gene is at risk for a disorder characterized by aberrant development, aberrant cellular differentiation, aberrant cellular proliferation or an aberrant hematopoietic response. In preferred embodiments, the methods include detecting, in a sample of cells from the subject, the presence or absence of a genetic alteration characterized by at least one of an alteration affecting the integrity of a gene encoding a CRSP-protein, or the mis-expression of the CRSP gene. For example, such genetic alterations can be detected by ascertaining the existence of at least one of 1) a deletion of one or more nucleotides from a CRSP gene; 2) an addition of one or more nucleotides to a CRSP gene; 3) a substitution of one or more nucleotides of a CRSP gene, 4) a chromosomal rearrangement of a CRSP gene; 5) an alteration in the level of a messenger RNA transcript of a CRSP gene, 6) aberrant modification of a CRSP gene, such as of the methylation pattern of the genomic DNA, 7) the presence of a non-wild type splicing pattern of a messenger RNA transcript of a CRSP gene, 8) a non-wild type level of a CRSP-protein, 9) allelic loss of a CRSP gene, and 10) inappropriate post-translational modification of a CRSP-protein. As described herein, there are a large number of assay techniques known in the art which can be used for detecting alterations in a CRSP gene. A preferred biological sample is a tissue or serum sample isolated by conventional means from a subject.

In certain embodiments, detection of the alteration involves the use of a probe/primer in a polymerase chain reaction (PCR) (see, e.g., U.S. Patent Nos. 4,683,195 and 4,683,202), such as anchor PCR or RACE PCR, or, alternatively, in a ligation chain reaction (LCR) (see, e.g., Landegran et al. (1988) Science 241:1077-1080; and Nakazawa et al. (1994) PNAS 91:360-364), the latter of which can be particularly useful for detecting point mutations in the CRSP-gene (see Abravaya et al. (1995) Nucleic Acids Res .23:675-682). This method can include the steps of collecting a sample of cells from a patient, isolating nucleic acid (e.g., genomic, mRNA or both) from the cells of the sample, contacting the nucleic acid sample with one or more primers which specifically hybridize to a CRSP gene under conditions such that hybridization and amplification of the CRSP-gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication (Guatelli, J.C. et al., 1990, Proc. Natl. Acad. Sci. USA 87:1874-1878), transcriptional amplification system (Kwoh, D.Y. et al., 1989, Proc. Natl. Acad. Sci. USA 86:1173-1177), Q-Beta Replicase (Lizardi, P.M. et all, 1988, Bio/Technology 6:1197), or any other nucleic acid amplification method, followed by the detection of the amplified molecules using techniques well known to those of skill in the art. These detection schemes are especially useful for the detection of nucleic acid molecules if such molecules are present in very low numbers.

In an alternative embodiment, mutations in a CRSP gene from a sample cell can be identified by alterations in restriction enzyme cleavage patterns. For example, sample and control DNA is isolated, amplified (optionally), digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes (see, for example, U.S. Patent No. 5,498,531) can be used to score for the presence of specific mutations by development or loss of a ribozyme cleavage site.

In other embodiments, genetic mutations in CRSP can be identified by hybridizing a sample and control nucleic acids, e.g., DNA or RNA, to high density arrays containing hundreds or thousands of oligonucleotides probes (Cronin, M.T. et al. (1996) Human Mutation 7: 244-255; Kozal, M.J. et al. (1996) Nature Medicine 2: 753-759). For example, genetic mutations in CRSP can be identified in two dimensional arrays containing light-generated DNA probes as described in Cronin, M.T. *et al. supra.* Briefly, a first hybridization array of probes can be used to scan through long stretches of DNA in a sample and control to identify base changes between the sequences by making linear arrays of sequential ovelapping probes. This step allows the identification of point mutations. This step is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the CRSP gene and detect mutations by comparing the sequence of the sample CRSP with the corresponding wild-type (control) sequence. Examples of sequencing reactions include those based on techniques developed by Maxim and Gilbert ((1977) PNAS 74:560) or Sanger ((1977) PNAS 74:5463). It is also contemplated that any of a variety of automated sequencing procedures can be utilized when performing the diagnostic assays ((1995) Biotechniques 19:448), including sequencing by mass spectrometry (see, e.g., PCT International Publication No. WO 94/16101; Cohen et al. (1996) Adv. Chromatogr. 36:127-162; and Griffin et al. (1993) Appl. Biochem. Biotechnol. 38:147-159).

Other methods for detecting mutations in the CRSP gene include methods in which protection from cleavage agents is used to detect mismatched bases in RNA/RNA or RNA/DNA heteroduplexes (Myers et al. (1985) Science 230:1242). In general, the art technique of "mismatch cleavage" starts by providing heteroduplexes of formed by hybridizing (labeled) RNA or DNA containing the wild-type CRSP sequence with potentially mutant RNA or DNA obtained from a tissue sample. The double-stranded duplexes are treated with an agent which cleaves single-stranded regions of the duplex such as which will exist due to basepair mismatches between the control and sample strands. For instance, RNA/DNA duplexes can be treated with RNase and DNA/DNA hybrids treated with S1 nuclease to enzymatically digesting the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. See, for example, Cotton et al. (1988) Proc. Natl Acad Sci USA 85:4397; Saleeba et al. (1992) Methods Enzymol. 217:286-295. In a preferred embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes) in defined systems for detecting and mapping point mutations in CRSP cDNAs obtained from samples of cells. For example, the mutY enzyme of E. coli cleaves A at G/A mismatches and the thymidine DNA glycosylase from HeLa cells cleaves T at G/T mismatches (Hsu et al. (1994) Carcinogenesis 15:1657-1662). According to an exemplary embodiment, a probe based on a CRSP sequence, e.g., a wild-type CRSP sequence, is hybridized to a cDNA or other DNA product from a test cell(s). The duplex is treated with a DNA mismatch repair enzyme, and the cleavage products, if any, can be detected from electrophoresis protocols or the like. See, for example, U.S. Patent No. 5,459,039.

In other embodiments, alterations in electrophoretic mobility will be used to identify mutations in CRSP genes. For example, single strand conformation polymorphism (SSCP) may be used to detect differences in electrophoretic mobility between mutant and wild type nucleic acids (Orita et al. (1989) Proc Natl. Acad. Sci USA: 86:2766, see also Cotton (1993) Mutat Res 285:125-144; and Hayashi (1992) Genet Anal Tech Appl 9:73-79). Single-stranded DNA fragments of sample and control CRSP nucleic acids will be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen et al. (1991) Trends Genet 7:5).

In yet another embodiment the movement of mutant or wild-type fragments in polyacrylamide gels containing a gradient of denaturant is assayed using denaturing gradient gel electrophoresis (DGGE) (Myers et al. (1985) Nature 313:495). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA (Rosenbaum and Reissner (1987) Biophys Chem 265:12753).

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions which permit hybridization only if a perfect match is found (Saiki et al. (1986) Nature 324:163); Saiki et al. (1989) Proc. Natl Acad. Sci USA 86:6230). Such allele specific oligonucleotides are hybridized to PCR amplified target DNA or a number of different mutations when the oligonucleotides are attached to the hybridizing membrane and hybridized with labeled target DNA.

Alternatively, allele specific amplification technology which depends on selective PCR amplification may be used in conjunction with the instant invention. Oligonucleotides used as primers for specific amplification may carry the mutation of interest in the center of the molecule (so that amplification depends on differential hybridization) (Gibbs et al. (1989) Nucleic Acids Res. 17:2437-2448) or at the extreme 3 end of one primer where, under appropriate conditions, mismatch can prevent, or reduce polymerase extension (Prossner (1993) Tibtech 11:238). In addition it may be desirable to introduce a novel restriction site in the region of the mutation to create cleavage-based detection (Gasparini et al. (1992) Mol. Cell Probes 6:1). It is anticipated that in certain embodiments amplification may also be performed using Taq ligase for amplification (Barany (1991) Proc. Natl. Acad. Sci USA 88:189). In such cases, ligation will occur only if there is a perfect match at the 3' end of the 5' sequence making it possible to detect the presence of a known mutation at a specific site by looking for the presence or absence of amplification.

The methods described herein may be performed, for example, by utilizing prepackaged diagnostic kits comprising at least one probe nucleic acid or antibody reagent described herein, which may be conveniently used, *e.g.,* in clinical settings to diagnose patients exhibiting symptoms or family history of a disease or illness involving a CRSP gene.

Furthermore, any cell type or tissue in which CRSP is expressed may be utilized in the prognostic assays described herein.

### 3. Monitoring of Effects During Clinical Trials

Monitoring the influence of agents (e.g., drugs, compounds) on the expression or activity of CRSP (e.g., modulation of cellular signal transduction, regulation of gene transcription in a cell involved in development or differentiation, regulation of cellular proliferation) can be applied not only in basic drug screening, but also in clinical trials. For example, the effectiveness of an agent determined by a screening assay as described herein to increase CRSP gene expression, protein levels, or upregulate CRSP activity, can be monitored in clinical trails of subjects exhibiting decreased CRSP gene expression, protein levels, or downregulated CRSP activity. Alternatively, the effectiveness of an agent determined by a screening assay to decrease CRSP gene expression, protein levels, or downregulate CRSP activity, can be monitored in clinical trails of subjects exhibiting increased CRSP gene expression, protein levels, or upregulated CRSP activity. In such clinical trials, the expression or activity of CRSP and, preferably, other genes that have been implicated in, for example, a proliferative disorder can be used as a "read out" or markers of the phenotype of a particular cell.

For example, and not by way of limitation, genes, including CRSP, that are modulated in cells by treatment with an agent (e.g., compound, drug or small molecule) which modulates CRSP activity (e.g., identified in a screening assay as described herein) can be identified. Thus, to study the effect of agents on proliferative disorders, developmental or differentiative disorder, hematopoietic disorder as well disorders characterized by abnormal cell differentiation and/or survival, an abnormal extracellular structure, or an abnormality in a defense mechanism, for example, in a clinical trial, cells can be isolated and RNA prepared and analyzed for the levels of expression of CRSP and other genes implicated in the proliferative disorder, developmental or differentiative disorder, hematopoietic disorder as well as disorders characterized by abnormal cell differentiation and/or survival, an abnormal extracellular structure, or an abnormality in a defense mechanism, respectively. The levels of gene expression (i.e., a gene expression pattern) can be quantified by Northern blot analysis or RT-PCR, as described herein, or alternatively by measuring the amount of protein produced, by one of the methods as described herein, or by measuring the levels of activity of CRSP or other genes. In this way, the gene expression pattern can serve as a marker, indicative of the physiological response of the cells to the agent. Accordingly, this response state may be determined before, and at various points during treatment of the individual with the agent.

The present invention can be used as the basis for a method for monitoring the effectiveness of treatment of a subject with an agent (e.g., an agonist, antagonist, peptidomimetic, protein, peptide, nucleic acid, small molecule, or other drug candidate identified by the screening assays described herein) comprising the steps of (i) obtaining a pre-administration sample from a subject prior to administration of the agent, (ii) detecting the level of expression of a CRSP protein, mRNA, or genomic DNA in the preadministration sample; (iii) obtaining one or more post-administration samples from the subject; (iv) detecting the level of expression or activity of the CRSP protein, mRNA, or genomic DNA in the post-administration samples; (v) comparing the level of expression or activity of the CRSP protein, mRNA, or genomic DNA in the pre-administration sample with the CRSP protein, mRNA, or genomic DNA in the post administration sample or samples; and (vi) altering the administration of the agent to the subject accordingly. For example, increased administration of the agent may be desirable to increase the expression or activity of CRSP to higher levels than detected, i.e., to increase the effectiveness of the agent. Alternatively, decreased administration of the agent may be desirable to decrease expression or activity of CRSP to lower levels than detected, i.e. to decrease the effectiveness of the agent According to such an embodiment, CRSP expression or activity may be used as an indicator of the effectiveness of an agent, even in the absence of an observable phenotypic response.

### C. Methods of Treatment:

The present invention can be used as the basis for both prophylactic and therapeutic methods of treating a subject at risk of (or susceptible to) a disorder or having a disorder associated with aberrant CRSP expression or activity. With regards to both prophylactic and therapeutic methods of treatment, such treatments may be specifically tailored or modified, based on knowledge obtained from the field of pharmacogenomics. "Pharmacogenomics", as used herein, refers to the application of genomics technologies such as gene sequencing, statistical genetics, and gene expression analysis to drugs in clinical development and on the market. More specifically, the term refers the study of how a patient's genes determine his or her response to a drug (e.g., a patient's "drug response phenotype", or "drug response genotype".) Thus, the invention provides the basis of methods for tailoring an individual's prophylactic or therapeutic treatment with either the CRSP molecules of the present invention or CRSP modulators according to that individual's drug response genotype. Pharmacogenomics allows a clinician or physician to target prophylactic or therapeutic treatments to patients who will most benefit from the treatment and to avoid treatment of patients who will experience toxic drug-related side effects.

### 1. Prophylactic Methods

In one aspect, the invention provides manufacture of a medicament for preventing in a subject, a disease or condition associated with an aberrant CRSP expression or activity. The medicament includes an agent which modulates CRSP expression or at least one CRSP activity. Subjects at risk for a disease which is caused or contributed to by aberrant CRSP expression or activity can be identified by, for example, any or a combination of diagnostic or prognostic assays as described herein. Administration of a prophylactic agent can occur prior to the manifestation of symptoms characteristic of the CRSP aberrancy, such that a disease or disorder is prevented or, alternatively, delayed in its progression. Depending on the type of CRSP aberrancy, for example, a CRSP agonist or CRSP antagonist agent can be used for treating the subject. The appropriate agent can be determined based on screening assays described herein. The prophylactic methods are further discussed in the following subsections.

### 2. Therapeutic Methods

Another aspect of the invention provides manufacture of a medicament for modulating CRSP expression or activity for therapeutic purposes. The modulatory method of the invention involves contacting a ceU with an agent that modulates one or more of the activities of CRSP protein activity associated with the cell. An agent that modulates CRSP protein activity can be an agent as described herein, such as a nucleic acid or a protein, a naturally-occurring target molecule of a CRSP protein, a peptide, a CRSP peptidomimetic, or other small molecule. In one embodiment, the agent stimulates one or more CRSP protein activity. Examples of such stimulatory agents include active CRSP protein and a nucleic acid molecule encoding CRSP that has been introduced into the cell. In another embodiment, the agent inhibits one or more CRSP protein activity. Examples of such inhibitory agents include antisense CRSP nucleic acid molecules and anti-CRSP antibodies. These modulatory methods can be performed *in vitro* (e.g., by culturing the cell with the agent) or, alternatively, in vivo, (e.g, by administering the p agent to a subject). As such, the present invention provides manufacture of medicaments for treating an individual afflicted with a disease or disorder characterized by aberrant expression or activity of a CRSP protein or nucleic acid molecule. In one embodiment, the medicament includes an agent (e.g., an agent identified by a screening assay described herein), or combination of agents that modulates (e.g., upregulates or downregulates) CRSP expression or activity. A CRSP protein or nucleic acid molecule can be administered as therapy to compensate for reduced or aberrant CRSP expression or activity.

Stimulation of CRSP activity is desirable in situations in which CRSP is abnormally downregulated and/or in which increased CRSP activity is likely to have a beneficial effect. Likewise, inhibition of CRSP activity is desirable in situations in which CRSP is abnormally upregulated and/or in which decreased CRSP activity is likely to have a beneficial effect. One example of such a situation is where a subject has a disorder characterized by aberrant development or cellular differentiation. Another example of such a situation is where the subject has a proliferative disease (e.g., cancer) or a disorder characterized by an aberrant hematopoietic response. Yet another example of such a situation is where it is desireable to acheive tissue regeneration in a subject (e.g., where a subject has undergone brain or spinal cord injury and it is desirable to regenerate neuronal tissue in a regulated manner.)

Accordingly, in one embodiment, the disease is a disease characterized by an abnormal cell proliferation, differentiation, and/or survival. For example, the disease can be a hyper-or hypoproliferative disease. The invention also provides manufacture of a medicament for treating diseases characterized by an abnormal cell proliferation, differentiation, and/or survival in a subject, which are not characterized by an abnormal CRSP activity (e.g., CRSP-1 activity). In fact, since CRSP is likely to be capable of modulating the proliferative state of a cell (i.e., state of proliferation, differentiation, and or survival of a cell), CRSP can regulate disease wherein the abnormal proliferative state of a cell results from a defect other than an abnormal CRSP activity.

Hyperproliferative diseases can be treated with CRSP (e.g., CRSP-1) therapeutics include neoplastic and hyperplastic diseases, such as various forms of cancers and leukemias, and fibroproliferative disorders. Other hyperproliferative diseases that can be treated or prevented with the subject CRSP therapeuucs (e.g. CRSP-1 therapeutics) include malignant conditions, premalignant conditions, and benign conditions. The condition to be treated or prevented can be a solid tumor, such as a tumor arising in an epithelial tissue. Accordingly, treatment of such a cancer could comprise administration to the subject of a CRSP therapeutic decreasing the interaction of CRSP with a CRSP receptor: Other cancers that can be treated or prevented with a CRSP protein include sarcomas and carcinomas, e.g., lung cancer, cancer of the colon, prostate, breast, ovary, esophagus, lung cancer, melanoma, seminoma, and squamous adenocarcinoma. Additional solid tumors within the scope of the invention include those that can be found in a medical textbook.

The condition to be treated or prevented can also be a soluble tumor, such as leukemia, either chronic or acute, including chronic or acute myelogenous leukemia, chronic or acute lymphocytic leukemia, promyelocytic leukemia, monocytic leukemia, myelomonocytic leukemia, and erythroleukemia. Yet other proliferative disorders that can be treated with a CRSP therapeutic of the invention include heavy chain disease, multiple myeloma, lymphoma, e.g., Hodgkin's lymphoma and non-Hodgkin's lymphoma, and Waldenstroem's macroglobulemia.

Diseases or conditions characterized by a solid or soluble tumor can be treated by administrating a CRSP therapeutic either locally or systemically, such that aberrant cell proliferation is inhibited or decreased. Methods for administering the compounds of the invention are further described below.

The invention also provides manufacture of a medicament for preventing the formation and/or development of tumors. For example, the development of a tumor can be preceded by the presence of a specific lesion, such as a pre-neoplastic lesion, e.g., hyperplasia, metaplasia, and dysplasia, which can be detected, e.g., by cytologic methods. Such lesions can be found; e.g., in epithelial tissue. Thus, the invention provides manufacture of a medicament for inhibiting progression of such a lesion into a neoplastic lesion. The subject having a preneoplastic lesion can be administered an amount of a CRSP-1 therapeutic sufficient to inhibit progression of the preneoplastic lesion into a neoplastic lesion.

The invention also provides manufacture of a medicament for treating or preventing diseases or conditions in which proliferation of cells is desired. For example, CRSP therapeutics can be used to stimulate tissue repair or wound healing, such as after surgery or to stimulate tissue healing from bums. Other diseases in which proliferation of cells is desired are hypoprolifetative diseases, i.e., diseases characterized by an abnormally low proliferation of certain cells.

In yet another embodiment, the invention provides manufacture of a medicament for treating or preventing diseases or conditions characterized by aberrant cell differentiation. Accordingly, methods for stimulating cellular differentiation in conditions characterized by an inhibition of normal cell differentiation which may or may not be accompanied by excessive proliferation. Alternatively, CRSP therapeutics can be used to inhibit differentiation of specific cells.

In a preferred method, the aberrantly proliferating and/or differentiating cell is a cell present in the nervous system. A role for CRSP in the nervous system is suggested at least in part from the fact that human CRSP-1 is expressed in human fetal brain. Accordingly, the invention provides manufacture of a medicament for treating diseases or conditions associated with a central or peripheral nervous system. For example, the invention provides manufacture of a medicament for treating lesions of the nervous system associated with an aberrant proliferation, differentiation or survival of any of the following cells: neurons, Schwann cells, glial cells, and other types of neural cells. Disorders of the nervous system include, but are not limited to: spinal cord injuries, brain injuries, lesions associated with surgery, ischemic lesions, malignant lesions, infectious lesions, degenerative lesions (Parkinson's disease, Alzheimer's disease, Huntington's chorea, amyotrophic lateral sclerosis), demyelating diseases (multiple sclerosis, human immunodeficiency associated myelopathy, transverse myelopathy, progressive multifocal leukoencephalopathy, pontine myelinolysis), motor neuron injuries, progressive spinal muscular atrophy, progressive bulbar palsy, primary lateral sclerosis, infantile and juvenile muscular atrophy, progressive bulbar paralysis of childhood (Fazio-Londe syndrome), poliomyelitis, and hereditary motorsensory neuropathy (Charcot-Marie-Tooth disease).

In another embodiment, the invention provides manufacture of a medicament for enhancing the survival and/or stimulating proliferation and/or differentiation of cells and tissues *in vitro*. In a preferred embodiment, CRSP therapeutics are used to promote tissue regeneration and/or repair (e.g., to treat nerve injury). For example, tissues from a subject can be obtained and grown *in vitro* in the presence of a CRSP therapeutic, such that the tissue cells are stimulated to proliferate and/or differentiate. The tissue can then be readministered to the subject.

Among the approaches which may be used to ameliorate disease symptoms involving an aberrant CRSP activity and/or an abnormal cell proliferation, differentiation, and/or survival, are, for example, antisense, ribozyme, and triple helix molecules described above. Examples of suitable compounds include the antagonists, agonists or homologues described in detail above.

Yet other CRSP therapeutics consist of a first peptide comprising a CRSP peptide capable of binding to a CRSP receptor, and a second peptide which is cytotoxic. Such therapeutics can be used to specifically target and lyse cells expressing or overexpressing a receptor for CRSP.

### 3. Pharmacogenomics

The CRSP molecules of the present invention, as well as agents, or modulators which have a stimulatory or inhibitory effect on CRSP activity (e.g., CRSP gene expression) as identified by a screening assay described herein can be administered to individuals to treat (prophylactically or therapeutically) disorders (e.g, proliferative or developmental disorders) associated with aberrant CRSP activity. In conjunction with such treatment, pharmacogenomics (i.e., the study of the relationship between an individual's genotype and that individual's response to a foreign compound or drug) may be considered. Differences in metabolism of therapeutics can lead to severe toxicity or therapeutic failure by altering the relation between dose and blood concentration of the pharmacologically active drug. Thus, a physician or clinician may consider applying knowledge obtained in relevant pharmacogenomics studies in determining whether to administer a CRSP molecule or CRSP modulator as well as tailoring the dosage and/or therapeutic regimen of treatment with a CRSP molecule or CRSP modulator.

Pharmacogenomics deals with clinically significant hereditary variations in the response to drugs due to altered drug disposition and abnormal action in affected persons. See e.g., Eichelbaum, M., Clin Exp Pharmacol Physiol, 1996, 23(10-11) :983-985 and Linder, M.W., Clin Chem, 1997, 43(2):254-266. In general, two types of pharmacogenetic conditions can be differentiated. Genetic conditions transmitted as a single factor altering the way drugs act on the body (altered drug action) or genetic conditions transmitted as single factors altering the way the body acts on drugs (altered drug metabolism). These pharmacogenetic conditions can occur either as rare genetic defects or as naturally-occurring polymorphisms. For example, glucose-6-phosphate dehydrogenase deficiency (G6PD) is a common inherited enzymopathy in which the main clinical complication is haemolysis after ingestion of oxidant drugs (anti-malarials, sulfonamides, analgesics, nitrofurans) and consumption of fava beans.

One pharmacogenomics approach to identifying genes that predict drug response, known as "a genome-wide association", relies primarily on a high-resolution map of the human genome consisting of already known gene-related markers (e.g., a "biallelic" gene marker map which consists of 60,000-100,000 polymorphic or variable sites on the human genome, each of which has two variants.) Such a high-resolution genetic map can be compared to a map of the genome of each of a statistically significant number of patients taking part in a Phase II/III drug trial to identify markers associated with a particular observed drug response or side effect. Alternatively, such a high resolution map can be generated from a combination of some ten-million known single nucleotide polymorphisms (SNPs) in the human genome. As used herein, a "SNP" is a common alteration that occurs in a single nucleotide base in a stretch of DNA. For example, a SNP may occur once per every 1000 bases of DNA. A SNP may be involved in a disease process, however, the vast majority may not be disease-associated. Given a genetic map based on the occurrence of such SNPs, individuals can be grouped into genetic categories depending on a particular pattern of SNPs in their individual genome. In such a manner, treatment regimens can be tailored to groups of genetically similar individuals, taking into account traits that may be common among such genetically similar individuals.

Alternatively, a method termed the "candidate gene approach", can be utilized to identify genes that predict drug response. According to this method, if a gene that encodes a drugs target is known (e.g., a CRSP protein or CRSP receptor of the present invention), all common variants of that gene can be fairly easily identified in the population and it can be determined if having one version of the gene versus another is associated with a particular drug response.

As an illustrative embodiment, the activity of drug metabolizing enzymes is a major determinant of both the intensity and duration of drug action. The discovery of genetic polymorphisms of drug metabolizing enzymes (e.g., N-acetyltransferase 2 (NAT 2) and cytochrome P450 enzymes CYP2D6 and CYP2C 19) has provided an explanation as to why some patients do not obtain the expected drug effects or show exaggerated drug response and serious toxicity after taking the standard and safe dose of a drug. These polymorphisms are expressed in two phenotypes in the population, the extensive metabolizer (EM) and poor metabolizer (PM). The prevalence of PM is different among different populations. For example, the gene coding for CYP2D6 is highly polymorphic and several mutations have been identified in PM, which all lead to the absence of functional CYP2D6. Poor metabolizers of CYP2D6 and CYP2C 19 quite frequently experience exaggerated drug response and side effects when they receive standard doses. If a metabolite is the active therapeutic moiety, PM show no therapeutic response, as demonstrated for the analgesic effect of codeine mediated by its CYP2D6-formed metabolite morphine. The other extreme are the so called ultra-rapid metabolizers who do not respond to standard doses. Recently, the molecular basis of ultra-rapid metabolism has been identified to be due to CYP2D6 gene amplification.

Alternatively, a method termed the "gene expression profiling", can be utilized to identify genes that predict drug response. For example, the gene expression of an animal dosed with a drug (e.g., a CRSP molecule or CRSP modulator of the present invention) can give an indication whether gene pathways related to toxicity have been turned on.

Information generated from more than one of the above pharmacogenomics approaches can be used to determine appropriate dosage and treatment regimens for prophylactic or therapeutic treatment an individual. This knowledge, when applied to dosing or drug selection, can avoid adverse reactions or therapeutic failure and thus enhance therapeutic or prophylactic efficiency when treating a subject with a CRSP molecule or CRSP modulator, such as a modulator identified by one of the exemplary screening assays described herein.

This invention is further illustrated by the following examples which should not be construed as limiting.

### EXAMPLES

### Example 1: Isolation And Characterization of Human CRSP-1 cDNA

In this example, the isolation and characterization of the gene encoding human CRSP- I (also referred to as "CRISPY-1" or "TANGO 59") is described.

### Isolation of the human CRSP-1 cDNA

The invention is based at least in part on the discovery of a human gene encoding a secreted protein, referred to herein as Cysteine Rich Secreted Protein-1 (CRSP-1). A partial cDNA was isolated using a Signal Sequence Trap method. This methodology takes advantage of the fact that molecules such as CRSP have an amino terminal signal sequence which directs certain secreted and membrane-bound proteins through the cellular secretory apparatus.

Briefly, a randomly primed cDNA library using mRNA prepared from human fetal brain tissue (Clontech, Palo Alto CA) was made by using the Stratagene-ZAP-cDNA Synthesis^{™} kit, (catalog #20041). The cDNA was ligated into the mammalian expression vector pTrap adjacent to a cDNA encoding placental alkaline phosphatase lacking a secretory signal. The plasmids were transformed into *E*. *coli* and DNA was prepared using the Wizard^{™} DNA purification kit (Promega). DNA was transfected into COS-7 cells with lipofectamine^{™} (Gibco-BRL). After 48 hours incubation the COS cell supernatants were assayed for alkaline phosphatase on a Wallac Micro-Beta scintillation counter using the Phospha-Light^{™} kit (Tropix Inc. Catalog #BP300). The individual plasmid DNAs scoring positive in the COS cell Alkaline Phosphatase secretion assay were further analyzed by DNA sequencing using standard procedures.

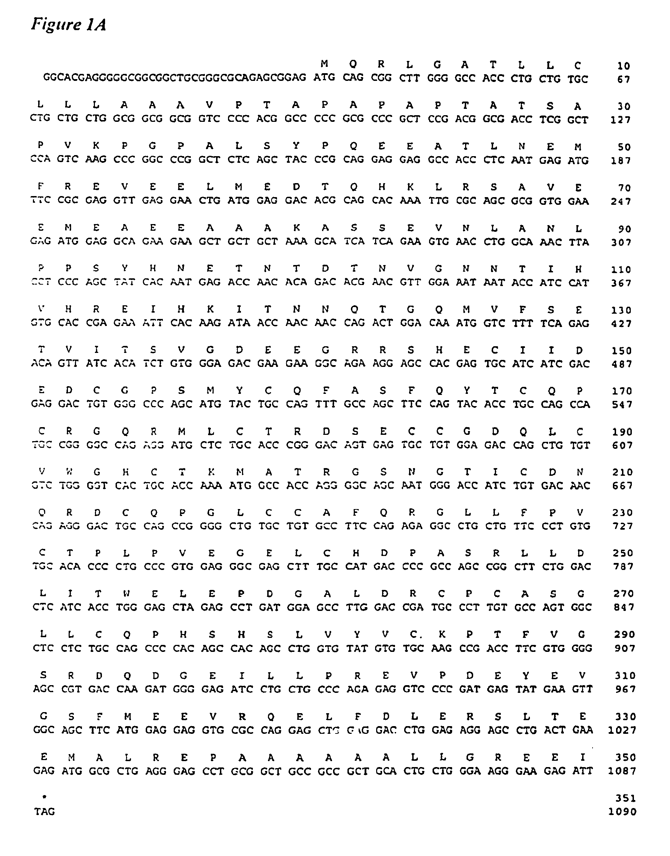

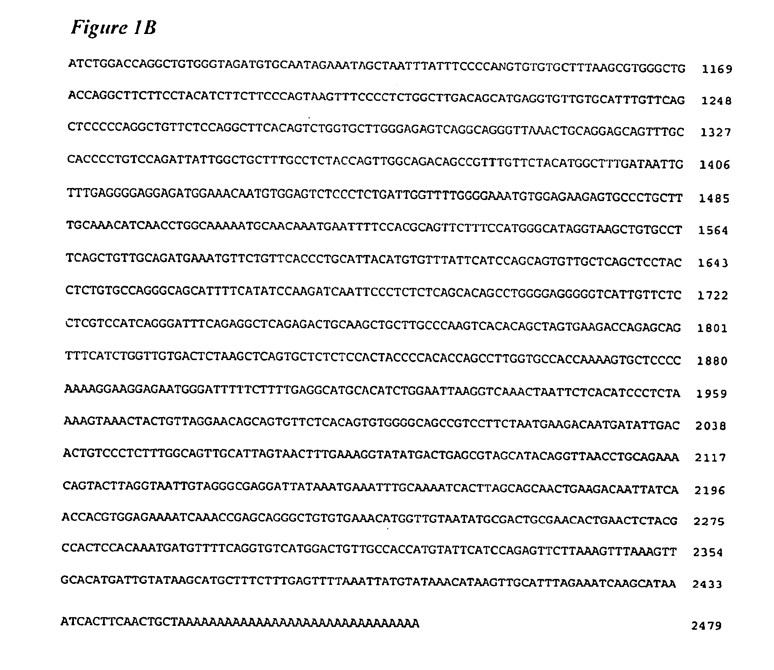

Using a partial cDNA isolated by the above-described method, a full length cDNA encoding human CRSP-1 was cloned. The nucleotide sequence encoding the full length human CRSP-1 protein is shown in Figure 1 and is set forth as SEQ ID NO: 1. The full length protein encoded by this nucleic acid is comprised of about 350 amino acids and has the amino acid sequence shown in Figure 1 and set forth as SEQ ID NO:2. The coding portion (open reading frame) of SEQ ID NO:1 is set forth as SEQ ID NO:3.

### Analysis of Human CRSP-1

Determination of the hydrophobicity profile of human CRSP-1 having the amino acid sequence set forth in SEQ ID NO:2 indicated the presence of a hydrophobic region from about amino acid 1 to about amino acid 23 of SEQ ID NO:2. Further analysis of the amino acid sequence SEQ ID NO:2 using signal peptide prediction programs predicted the presence of a signal peptide from about amino acid 1 to about amino acid 19, 21, or 23 of SEQ ID NO:2. Accordingly, the mature CRSP-1 protein includes about 327, 329, or 331 amino acids spanning from about amino acid 20, 22, or 24 to about amino acid 350 of SEQ ID NO:2. The presence of the signal sequence, in addition to the fact that CRSP-1 has been identified using a Signal Sequence Trap system, indicates that CRSP-1 is a secreted protein. Furthermore, the prediction of such a signal peptide and signal peptide cleavage site can be made, for example, utilizing the computer algorithm SIGNALP (Henrik, et al. (1997) Protein Engineering 10:1-6).

Furthermore, when the cDNA encoding human CRSP-1 was expressed in bacterial cells with a C-terminal FLAG tag, the FLAG-tagged CRSP-1 protein product was found to be secreted in to the cellular medium.

Examination of the cDNA sequence depicted in Figure 1 shows that human CRSP-1 is particularly rich in cysteine residues. As shown in Figure 1, CRSP- I contains 20 cysteine residues located between amino acid 147 and amino acid 284 of SEQ ID NO:2. These cysteine residues can possibly form 10 disulfide bridges.

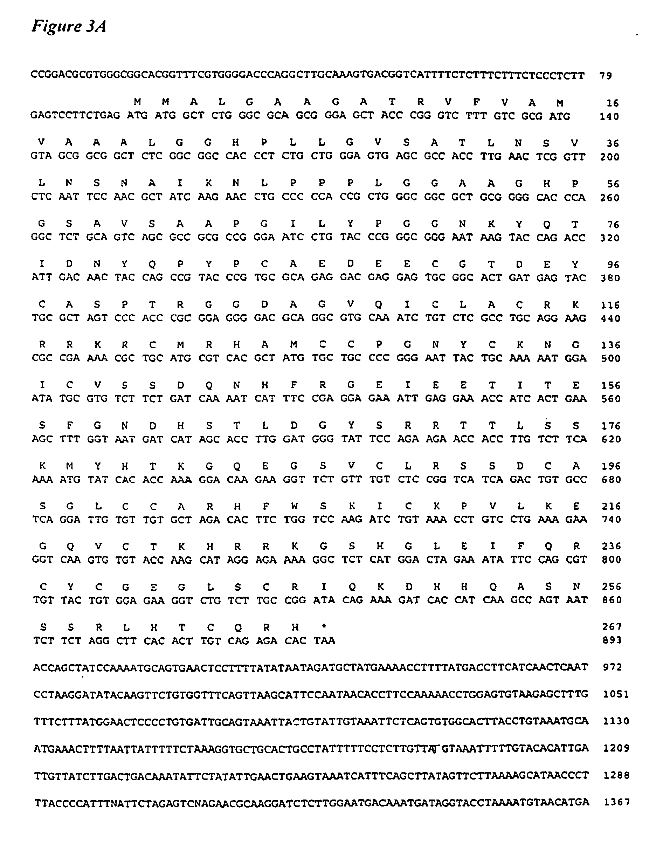

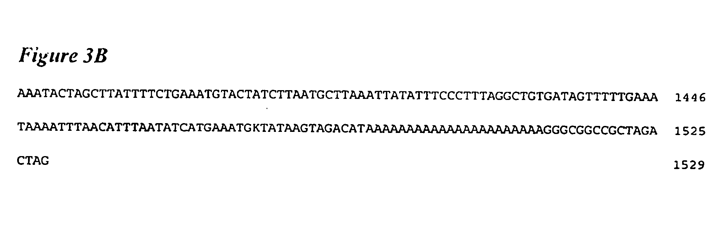

A BLAST search (Altschul et al. (1990) J. Mol. Biol. 215:403) of the nucleotide and the amino acid sequences of CRSP-1 has revealed that CRSP-1 is significantly similar to a chicken cDNA encoding a protein of unknown function having GenBank Accession No. D26311. This cDNA was isolated from a chicken lens cDNA library and was shown to be expressed in lens fibers and lens epithelium, but not in neural retina nor in liver cells. (Sawada et al. (1996) Int. J. Dev. Biol. 40:531). CRSP-1 and the chicken protein have 56% amino acid sequence identity and 72% amino acid sequence similarity. The amino acid sequence similarity between the chicken protein and human CRSP-1 is particularly high in the cysteine-rich domain of CRSP-1 which is located between amino acids 147 and 284 of SEQ ID NO: 2. In particular, the 20 cysteine residues of CRSP-1 located in this region are present in the chicken protein (see Figure 3).

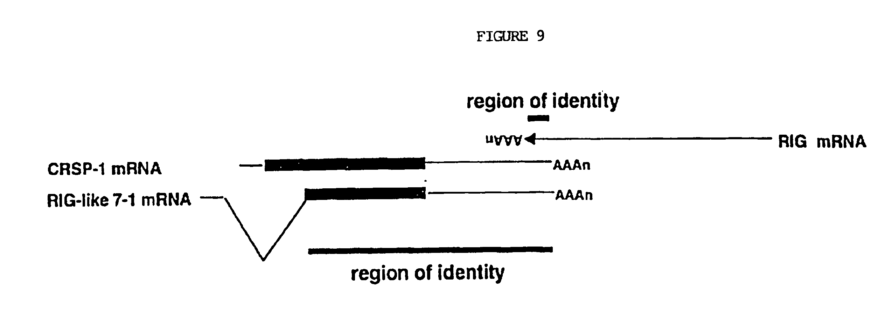

Two genes recently identified in a screen for suppressors of glioblastoma formation (Ligon et al., 1997 Oncogene 14 1075-1081) also show significant homology to hCRSP-1. These genes, RIG ("Regulated In Glioblastoma") and RIG-like 7-1 (GenBank Accession Nos. U32331 and AF034208, respectively), were identified in a differential screen for mRNAs regulated by the introduction of a normal copy of chromosome 10 into a glioblastoma cell line harboring a deletion in chromosome 10 that promotes tumorigenesis. A schematic diagram summarizing the relationship between the sequences of the CRSP-1 and the RIG genes presented as Figure 9. The indicated region of identity between CRSP-1, RIG and RIG-like 7-1 comprises a region of the 3' UTR of the human CRSP-1 mRNA. This cDNA was initially isolated in the described differential screen and subsequently used to isolate full length RIG and RIG-like 7-1. messages as depicted. RIG-like 7-1 is highly homologous (96.8% similar as determined using the Lipman-Pearson protein alignment program, Ktuple: 2; Gap Penalty: 4, Gap Length Penalty: 12; and 64.7% homologous using the Wilbur Lipman DNA alignment program, Ktuple: 3; Gap Penalty: 3; Window: 20) to CRSP-1 although the encoded protein lacks the N-terminal signal sequence and is not therefore predicted to be a secreted protein. The full length RIG mRNA only displays homology to CRSP-1 in the region of the initial cDNA. These data associate CRSP-1 with human glioblastoma and suggest that CRSP-1 may be important in the suppression of the tumorigenic phenotype. A role in glioblastoma is also consistent with the high level of CRSP-1 mRNA expression observed in human brain tissue. In addition, the co-localization of the CRSP-1, RIG and RIG-like genes to a region of chromosome 11 (11p15.1) implicated in the development of human malignant astrocytoma (Ligon et al. 1997 Oncogene 14 1075-1081) further indicates a role for these genes in tumorogenesis.

Human CRSP-1 protein has also some amino acid sequence similarity to metallothionein, particularly in the cyteine-rich domain.

### Tissue Distribution of CRSP-1 mRNA

This Example describes the tissue distribution of CRSP-1 mRNA, as determined by Northern blot hybridization.

Northern blot hybridizations with the various RNA samples were performed under standard conditions and washed under stringent conditions, i.e., 0.2xSSC at 65°C. In each sample, the probe hybridized to a single RNA of about 2.5 kb. The results of hybridization of the probe to various mRNA samples are described below.

Hybridization of a Clontech Fetal Multiple Tissue Northern (MTN) blot (Clontech, LaJolla, CA) containing RNA from fetal brain, lung, liver, and kidney indicated the presence of high levels of CRSP-1 mRNA in fetal brain, lung, and slightly lower levels of GRSP-1 mRNA in fetal kidney. However, no significant level of CRSP-1 mRNA was found in fetal liver.

Hybridization of a Clontech human Multiple Tissue Northern (MTN) blot (Clontech, LaJolla, CA) containing RNA from adult heart, brain, placenta, lung, liver, skeletal muscle, kidney, and pancreas with a human CRSP-1 probe indicated the presence of high levels of CRSP-1 mRNA in heart, slightly lower levels in brain, and much lower levels in placenta and lung. Some CRSP-1 mRNA was also found in adult skeletal muscle. However, no significant levels of CRSP-1 mRNA was observed in adult liver, kidney, or pancreas. Interestingly, the chicken gene which is homologous to CRSP-1 was not expressed at detectable levels in liver either (Sawada et al. (1996) Int. J. Dev. Biol. 40:531).

Further hybridization of a Clontech human Multiple Tissue Northern (MTN) blot (Clontech, LaJolla, CA) including RNA from adult spinal cord revealed high levels of expression of CRSP-1 in mRNA isolated from adult spinal cord.

In situ analysis further revealed the following expression patterns when tissue sections were hybridized with CRSP-1 probes. CRSP-1 mRNA was expressed in heart, lung, aorta, eye (retina and lens), and brain (cortex) in 14.5 day mouse embryos. CRSP-1 mRNA was expressed in heart, lung, eye (retina and lens), brain (cortical), and cartilage (foot, trachea, larynx, head, sternum, and spinal column) in 1.5 day post natal mouse tissues. CRSP-1 mRNA was expressed in brain (cortex, hippocampus, brainstem), heart (atria only), eye (retina and lens outer layer) in adult mouse tissues.

Thus, CRSP-1 is expressed in a tissue specific manner, with the strongest expression observed in brain, heart, and spinal cord.

### CRSP-1 protein expression data

### Constructs

Expression constructs for two forms of CRSP-1 were prepared using the mammalian expression vector pMET-stop. Form-1 comprised a cDNA incorporating the complete 350aa CRSP-1 protein coding sequence (CRSP-1 flag.long) and form-2 comprised the entire CRSP-1 protein coding sequence except for the final 18 amino acids (CRSP-1 flag.short). A C-terminal sequence encoding the FLAG epitope (DYKDDDDK) was added to both CRSP-1 forms for ease of detection and purification. CRSP-1flag cDNAs were generated by PCR from a full length CRSP-1 cDNA template and ligated into pMET-stop using EcoR1 and Sal1 restriction sites.

### Trial transfection - small scale expression

Expression constructs for CRSP-1flag.long and CRSP-1flag.short were transfected into 293T cells using 10 µl of lipofectamine (GIBCO/BRL) and 2 µg of DNA per well of a 6-well plate of cells which were 70-80% confluent. After 5 hours at 37°C, cells were fed with 1ml of 20%FCS/DMEM. After incubation overnight at 37°C, cells were conditioned in 1ml OptiMEM for 48 hours at 37°C. Samples of supernatant and cell pellets were solubilized in boiling SDS-PAGE gel buffer, run out on a 4-20% SDS-PAGE gel, transferred to a nylon membrane and probed with the anti-FLAG monoclonal antibody M2. Samples from both supernatant and pellet samples showed significant immunoreactivity within a molecular weight range of 40-65 kDa on autoradiographic film using a HRP conjugated secondary antibody and ECL detection reagents. Thus, both forms of CRSP-1 tested are secreted from 293T cells thereby confirming experimentally that CRSP-1 is a secreted protein. It should be noted that the molecular weights of both forms of CRSP-1 tested are greater than predicted from the amino acid sequence, suggesting that the CRSP-1 proteins secreted by 293T cells may be glycosylated. This is consistent with the presence of four potential sites for N-linked glycosylation in the CRSP-1 protein.

### Large scale CRSP-1 protein production

For scale-up of CRSP-1flag.long protein expression, 30 x 150mM plates of 293T cells at 70-80% confluence were transfected with 27 µg DNA, 100 µl lipofectamine in 18ml OptiMEM for 5 hours at 37°C. 18 ml of 10%FCS/DMEM was added to each plate and incubated overnight at 37°C. 24 hours after the start of transfection, transfection supernatant was aspirated and 35 mls OptiMEM was added to each plate and the plates incubated at 37°C for 72 hours. Conditioned medium was harvested, spun at 4000 rpm for 30 min. at 4°C, and filtered through a 0.45 micron filter unit. 1100 ml was passed over a 1.6 x 10 cm anti-FLAG M2 affinity column pre-equilibrated in PBS pH7.4 buffer at a flow rate of 2.0 ml per minute. After washing with 200 ml of PBS pH 7.4 buffer, bound material was eluted by a step of 200 mM Glycine pH 3.0 buffer and 0.5 ml fractions collected. Upon elution, a significant protein peak was detected by absorbance at 280nm. Samples corresponding to conditioned medium, flow through and eluted fractions were analyzed by Coomassie blue and silver stained SDS-PAGE and by western blot analysis as described above. Significant immunoreactivity within a molecular weight range of 40-65 kDa was detected in conditioned medium and eluted fractions but not in the flow through sample, indicating that the secreted CRSP-1 flag.long protein bound to the affinity column specifically and was eluted efficiently by the described conditions. Coomassie blue staining of SDS-PAGE gels suggested that the predominant immunoreactive protein constituted >90% of the protein present in the bound and eluted protein peak. Peak fractions of eluted protein were pooled and dialysed against Phosphate Buffered Saline resulting in a 4 ml volume of recombinant CRSP-1 flag.long protein at a concentration of approximately 1mg/ml.

### Example 2: Isolation And Characterization of Human CRSP-2, CRSP-3, and CRSP-4 cDNA

To identify novel proteins related to CRSP-1, the human CRSP-1 amino acid sequence was used to search the private and dbEST databases using TBLASTN (WashUversion, 2.0, BLOSUM62 search matrix). From this search, four distinct partial protein sequences were identified which displayed significant homology to human CRSP-1. These sequences were designated as partial sequences for the novel hCRSPs; hCRSP-2, h-CRSP-3, h-CRSP-4 and h-CRSP-like-n.

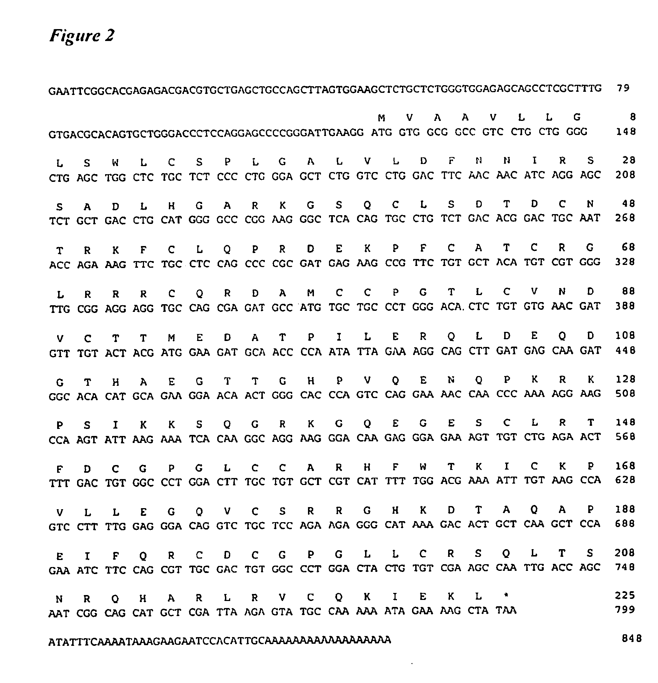

hCRSP-2 partial protein sequence was derived from a single dbEST clone with accession number AA565546. This clone was subsequently obtained from the IMAGE collection and sequenced fully to define the entire hCRSP-2 sequence depicted in Figure 2.

hCRSP-3 partial protein sequence was derived from a jthKb075a10 clone with sequence identification code jthKb075a10. This clone was sequenced further and this sequence information combined with additional sequences from dbEST using the assembly program Phrap (P. Green, U. Washington) to define the entire hCRSP-3 sequence depicted in Figure 3. DNA for the clone jthKb075a10 was deposited with the ATCC as Accession No. 98633. Northern blot analysis of various tissues using a probe specific for hCRSP-3 revealed that CRSP-3 mRNA expression was restricted to placenta. CRSP-3 mRNA expression has not yet been demonstrated in other normal adult human tissues.

A murine and *Xenopus* homologue of CRSP-3 has recently been described called dkk-1 ("dickkopf", German, thickhead) which is proposed to encode a secreted signaling molecule (Nature, 391, 357-368). dkk-1 is described as a powerful inducer of head formation during early *Xenopus* development, its mechanism of action apparently involving inhibition of the *wnt* family of secreted factors.

Given the homology between CRSP-3 and dkk-1, it is suggested that hCRSP-3 may display profound head-inducing activity during early human embryonic development. Furthermore,the mechanisms of action of dkk-1 is thought to involve antagonism of members of the *wnt* family of secreted proteins. Overexpression of *wnt* proteins has been associated with certain malignancies. For example, activation of *wnt-1* expression by proviral insertion of MMTV causes breast cancer in the mouse (see Cadigan and Nusse, Genes and Dev., 1997 11 3286-3305). Therefore, at least CRSP-3, and potentially other CRSP family members, may also play a role in the suppression of *wnt* signaling in cancer.

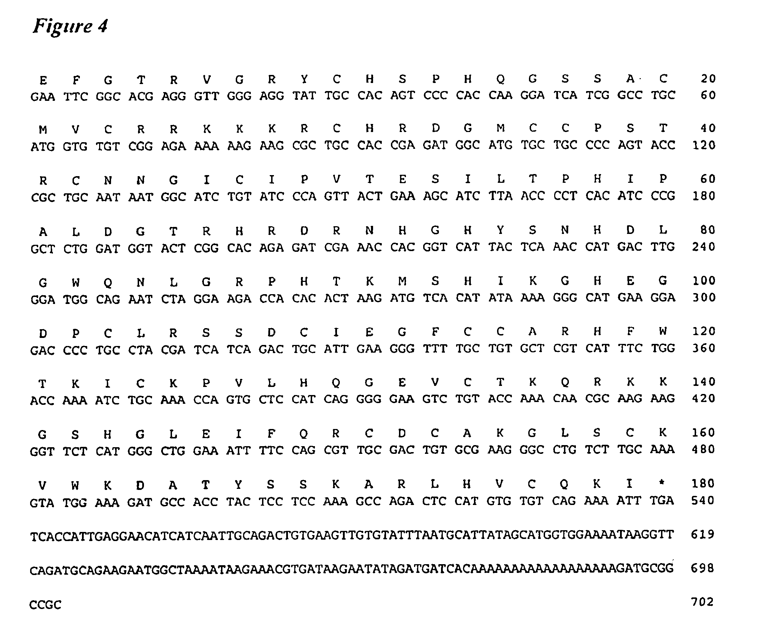

h-CRSP-4 partial protein sequence was derived from a single dbEST clone with accession number W55979. This clone was subsequently obtained from the IMAGE collection and sequenced fully to define a hCRSP-4 sequence depicted in Figure 4. Northern blot analysis of various tissues using a probe specific for hCRSP-4 revealed that CRSP-4 mRNA was expressed in several adult human tissues. CRSP-4 mRNA expression was highest in heart, brain, placenta, lung, and skeletal muscle.

h-CRSP-like-n partial protein sequence was derived from a single dbEST clone with accession number AA397836. This clone was subsequently obtained from the IMAGE collection and sequenced fully to define the entire hCRSP-like-n sequence depicted in Figure 5.

### Structure of the CRSP Family proteins

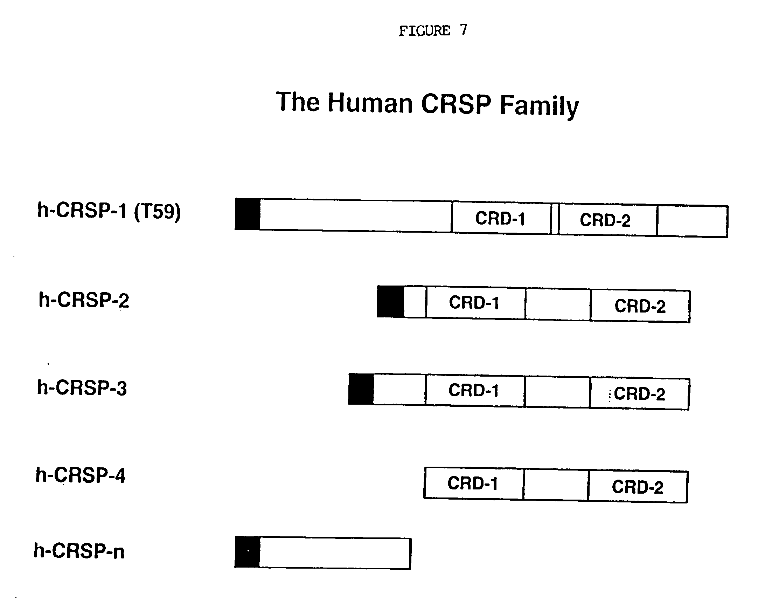

An alignment of the amino acid sequences of human CRSP-1, human CRSP-2, human CRSP-3, and human CRSP-4 is shown in Figure 6. Amino acid residues which are conserved between human CRSP family members are boxed. The 20 conserved cysteine residues are indicated by an asterix. The cysteine-rich domains are indicated as CRD-1 and CRD-2. The domain structure of the full length CRSP proteins of the present invention are depicted in Figure 7. The amino acid and nucleotide homology between CRSP family members is as follows in Tables 1 and 2.

**Table 1:**

| | **CRSP-1** | **CRSP-2** | **CRSP-3** | **CRSP-4** | **mdkk-1** | **xdkk-1** | **CLFEST** |
|---|---|---|---|---|---|---|---|
| **CRSP-1** | 100 | 16.0 | 18.6 | 15.1 | 18.5 | 16.5 | 53.0 |
| **CRSP-2** | | 100 | 33.7 | 35.2 | 32.6 | 33.7 | 16.2 |
| **CRSP-3** | | | 100 | 33.1 | 80.2 | 53.5 | 17.4 |
| **CRSP-4** | | | | 100 | 30.5 | 33.7 | 12.5 |

Amino acid percent homologies were determined using the ALIGN program, (version 2.0) (GCG software package) using a PAM120 weight residue table, a gap length penalty of 12, and a gap penalty of 4.

**Table 2:**

| | **CRSP-1** | **CRSP-2** | **CRSP-3** | **CRSP-4** | **mdkk-1** | **xdkk-1** | **CLFEST** |
|---|---|---|---|---|---|---|---|
| **CRSP-1** | 100 | 30.0 | 37.2 | 34.7 | 31.5 | 45.4 | 58.8 |
| **CRSP-2** | | 100 | 43.0 | 35.9 | 38.8 | 38.4 | 36.7 |
| **CRSP-3** | | | 100 | 59.3 | 66.4 | 53.7 | 32.1 |
| **CRSP-4** | | | | 100 | 38.8 | 38.4 | 36.7 |

Nucleotide percent homologies were determined using the using the Wilbur Lipman DNA alignment program, Ktuple: 3; Gap Penalty: 3; Window: 20.

## Claims

1. An isolated nucleic acid molecule selected from the group consisting of:
a) a nucleic acid comprising SEQ ID NO: 3 or SEQ ID NO: 7;
b) a nucleic acid which encodes a polypeptide comprising the amino acid sequence of amino acid residues 24-350 of SEQ ID NO: 2;
c) a nucleic acid which encodes a polypeptide that comprises a naturally occurring allelic variant of the amino acid sequence of amino acid residues 20-224 of SEQ ID NO: 5, SEQ ID NO: 11, or SEQ ID NO: 14, wherein said naturally occurring allelic variant modulates cellular signal transduction or cellular proliferation, and wherein (i) said nucleic acid which encodes a naturally occurring allelic variant of the amino acid sequence of amino acid residues 20-224 of SEQ ID NO: 5 hybridizes to the complement of SEQ ID NO: 6 under stringent wash conditions, (ii) said nucleic acid which encodes a naturally occurring allelic variant of the amino acid sequence of SEQ ID NO: 11 hybridizes to the complement of SEQ ID NO: 12 under stringent wash conditions, and consists of SEQ ID NO: 12, (iii) said nucleic acid which encodes a naturally occurring allelic variant of the amino acid sequence of SEQ ID NO: 14 hybridizes to the complement of SEQ ID NO: 15 under stringent wash conditions, wherein stringent wash conditions are 0.2X SSC, 0.1% SDS at 50-65°C;
d) nucleic acid comprising a nucleotide sequence that is the complement of a), b), c)(i) or c)(iii);
e) a nucleic acid consisting of SEQ ID NO: 9; and
f) a recombinant eukaryotic cell expression vector comprising the nucleic acid of a), b), c)(i), c) (iii) or d), or a nucleic acid comprising SEQ ID NO: 9.

2. The isolated nucleic acid molecule of Claim 1, wherein said isolated nucleic acid molecule comprises a nucleotide sequence selected from the group consisting of SEQ ID NO: 1, and SEQ ID NO: 3.

3. The isolated nucleic acid molecule of Claim 1, wherein said isolated nucleic acid molecule encodes a polypeptide comprising an amino acid sequence selected from the group consisting of SEQ ID NO: 2, amino acid residues 20-350 of SEQ ID NO: 2, amino acid residues 22-350 of SEQ ID NO: 2 and amino acid residues 24-350 of SEQ ID NO: 2.

4. The isolated nucleic acid molecule of Claim 1, wherein said isolated nucleic acid molecule encodes a polypeptide comprising an amino acid sequence selected from the group consisting of SEQ ID NO: 8 and amino acid residues 21-266 of SEQ ID NO: 8.

5. The isolated nucleic acid molecule of Claim 1a), 1b) or 1c)(i) or c)(iii), wherein said isolated nucleic acid molecule further comprises:
i) a vector nucleic acid sequence; and/or
ii) a nucleic acid sequence encoding a heterologous polypeptide.

6. The isolated nucleic acid molecule of Claim 1f), wherein said isolated nucleic acid molecule further comprises a nucleic acid sequence encoding a heterologous polypeptide.

7. A host cell comprising the recombinant eukaryotic cell expression vector of Claim 1f).

8. A host cell comprising a vector comprising the nucleic acid of any one of Claims 1 a), 1 b), 2 or 3, wherein said nucleic acid is a recombinant nucleic acid.

9. An isolated polypeptide selected from the group consisting of:
a) a polypeptide comprising at least 25 contiguous amino acids of SEQ ID NO: 5 or SEQ ID NO: 14, or a polypeptide comprising at least 100 contiguous amino acids of SEQ ID NO: 11, or a polypeptide comprising an amino acid sequence selected from the group consisting of SEQ ID NO:8 and amino acid residues 21-266 of SEQ ID NO: 8, which polypeptide modulates cellular signal transduction or cellular proliferation;
b) a polypeptide comprising a naturally occurring allelic variant of the amino acid sequence of amino acid residues 20-224 of SEQ ID NO: 5, amino acid residues 21-266 of SEQ ID NO: 8, or SEQ ID NO: 14, wherein said naturally occurring allelic variant modulates cellular signal transduction or cellular proliferation and wherein (i) said naturally occurring allelic variant of the amino acid sequence of amino acid residues 20-224 of SEQ ID NO: 5 is encoded by a nucleic acid molecule which hybridizes to the complement of SEQ ID NO: 6 under stringent wash conditions, (ii) said naturally occurring allelic variant of the amino acid sequence of SEQ ID NO: 8 is encoded by a nucleic acid molecule which hybridizes to the complement of SEQ ID NO: 9 under stringent wash conditions, and wherein the protein encoded by the nucleic acid is at least 85 % homologous to SEQ ID NO: 8, and (iii) said naturally occurring allelic variant of the amino acid sequence of SEQ ID NO: 14 is encoded by a nucleic acid molecule which hybridizes to the complement of SEQ ID NO: 15 under stringent wash conditions, and wherein stringent wash conditions are 0.2X SSC, 0.1% SDS at 50-65°C;
c) a polypeptide comprising amino acid residues 24-350 of SEQ ID NO: 2; and
d) a polypeptide comprising an amino acid sequence which has at least 80% amino acid sequence identity with the amino acid sequence of SEQ ID NO: 5 or SEQ ID NO: 11 over the whole length of SEQ ID NO: 5 or SEQ ID NO: 11, respectively, or a polypeptide comprising an amino acid sequence which has at least 85% amino acid sequence identity with the amino acid sequence of SEQ ID NO: 8 over the whole length of SEQ ID NO: 8, wherein the polypeptide modulates cellular signal transduction or cellular proliferation.

10. The isolated polypeptide of Claim 9, wherein said polypeptide comprises at least 50 contiguous amino acids of SEQ ID NO: 5, or SEQ ID NO: 14 and wherein said polypeptide modulates cellular signal transduction or cellular proliferation.

11. The isolated polypeptide of Claim 9, wherein said polypeptide comprises at least 100 contiguous amino acids of SEQ ID NO: 5, SEQ ID NO: 11 or SEQ ID NO: 14 and wherein said polypeptide modulates cellular signal transduction or cellular proliferation.

12. The isolated polypeptide of Claim 9, wherein said polypeptide comprises SEQ ID NO: 8 and wherein said polypeptide modulates cellular signal transduction or cellular proliferation.

13. The isolated polypeptide of Claim 9, wherein said isolated polypeptide comprises an amino acid sequence selected from the group consisting of SEQ ID NO: 8 and amino acid residues 21-266 of SEQ ID NO: 8.

14. The isolated polypeptide of Claim 9, wherein said isolated polypeptide comprises an amino acid sequence selected from the group consisting of SEQ ID NO: 2, amino acid residues 20-350 of SEQ ID NO: 2, amino acid residues 22-350 of SEQ ID NO: 2, and amino acid residues 24-350 of SEQ ID NO: 2.

15. The isolated polypeptide of Claim 9, wherein said isolated polypeptide comprises an amino acid sequence selected from the group consisting of SEQ ID NO: 5, amino acid residues 20-224 of SEQ ID NO: 5, SEQ ID NO: 11 and SEQ ID NO: 14.

16. The isolated polypeptide of any one of Claims 10-15 further comprising a heterologous polypeptide.

17. An antibody or immunologically active portion thereof which specifically binds to a polypeptide selected from the group consisting of:
a) a polypeptide consisting of 15 contiguous amino acids of SEQ ID NO: 5, SEQ ID NO: 11 or SEQ ID NO: 14 or a polypeptide consisting of 30 contiguous amino acids of SEQ ID NO: 8; and
b) a polypeptide consisting of a naturally occurring allelic variant of the amino acid sequence of amino acid residues 20-224 of SEQ ID NO: 5, amino acid residues 21-266 of SEQ ID NO: 8, SEQ ID NO: 11 or SEQ ID NO: 14, wherein said naturally occurring allelic variant modulates cellular signal transduction or cellular proliferation and wherein (i) said naturally occurring allelic variant of the amino acid sequence of amino acid residues 20-224 of SEQ ID NO: 5 is encoded by a nucleic acid molecule which hybridizes to the complement of SEQ ID NO: 6 under stringent wash conditions, (ii) said naturally occurring allelic variant of the amino acid sequence of amino acid residues 21-266 of SEQ ID NO: 8 is encoded by a nucleic acid molecule which hybridizes to the complement of SEQ ID NO: 9 under stringent wash conditions, (iii) said naturally occurring allelic variant of the amino acid sequence of SEQ ID NO: 11 is encoded by a nucleic acid molecule which hybriddizes to the complement of SEQ ID NO: 12 under stringent wash conditions, (iv) said naturally occurring allelic variant of the amino acid sequence of SEQ ID NO: 14 is encoded by a nucleic acid molecule which hybridizes to the complement of SEQ ID NO: 15 under stringent wash conditions, and wherein stringent wash conditions are 0.2X SSC, 0.1% SDS at 50-65°C.

18. The antibody or immunologically active portion thereof of Claim 17, wherein said antibody or immunologically active portion thereof specifically binds to a polypeptide consisting of 15 contiguous amino acids of SEQ ID NO: 5, SEQ ID NO: 11 or SEQ ID NO: 14.

19. The antibody or immunologically active portion thereof of Claim 17, wherein said antibody or immunologically active portion thereof specifically binds to a polypeptide consisting of 20 contiguous amino acids of SEQ ID NO: 5, SEQ ID NO: 11 or SEQ ID NO:14.

20. The antibody or immunologically active portion thereof of Claim 17, wherein said antibody or immunologically active portion thereof specifically binds to a polypeptide consisting of 30 contiguous amino acids of SEQ ID NO: 5, SEQ ID NO: 8, SEQ ID NO: 11 or SEQ ID NO: 14.

21. The antibody or immunologically active portion thereof of Claim 18, wherein said antibody or immunologically active portion thereof specifically binds a polypeptide that consists of an amino acid sequence selected from the group consisting of SEQ ID NO: 5, SEQ ID NO: 8, SEQ ID NO: 11 and SEQ ID NO: 14.

22. The antibody or immunologically active portion thereof of Claim 21, wherein said antibody or immunologically active portion thereof specifically binds a polypeptide that consists of the amino acid sequence of amino acid residues 21-266 of SEQ ID NO: 8.

23. The antibody or immunologically active portion thereof of Claim 21, wherein said antibody or immunologically active portion thereof specifically binds a polypeptide that consists of the amino acid sequence of amino acid residues 20-224 of SEQ ID NO: 5.

24. The antibody or immunologically active portion thereof of any one of Claims 17-23, wherein said antibody or immunologically active portion thereof is selected from the group consisting of a human antibody, a humanized monoclonal antibody, a chimeric monoclonal antibody and an immunologically active portion of any one of the foregoing.

25. The antibody or immunologically active portion thereof of any one of Claims 17-23 wherein said antibody or immunologically active portion thereof is a monoclonal antibody or an immunologically active portion of a monoclonal antibody.

26. The antibody or immunologically active portion thereof of any one of Claims 17-23, wherein said antibody or immunologically active portion thereof is an immunologically active portion selected from the group consisting of an Fab fragment and an F(ab')₂ fragment.

27. The antibody or immunologically active portion thereof of any one of Claims 17-26, wherein said antibody or immunologically active portion thereof comprises a detectable label.

28. The antibody or immunologically active portion thereof of Claim 27, wherein said detectable label is an enzyme, a prosthetic group, a fluorescent material, a luminescent material, a bioluminescent material or a radioactive material.

29. A method for producing a polypeptide of Claim 9 comprising culturing the host cell of Claim 7 under conditions in which the recombinant expression vector is expressed, and said polypeptide is produced.

30. A method for producing a polypeptide of Claim 13 or Claim 14 comprising culturing the host cell of Claim 8 under conditions in which the recombinant nucleic acid is expressed, and said polypeptide is produced.

31. A method for detecting the presence of a polypeptide of any one of Claims 9-16 in a sample comprising:
a) contacting the sample with an antibody or immunologically active portion thereof of any one of Claims 17-28; and
b) determining whether said antibody binds to a polypeptide in the sample to thereby detect the presence of said polypeptide of any one of Claims 9-16 in the sample.

32. A kit comprising an antibody or immunologically active portion thereof of any one of Claims 17-28 and instructions for use.

33. Use of a kit for detecting a polypeptide of any one of Claims 9-16 in a sample, said kit comprising an antibody or immunologically active portion thereof of any one of Claims 17-28 and instructions for use.

34. A method for detecting the presence of a nucleic acid molecule of any one of Claims 1-4 in a sample comprising:
a) contacting the sample with a nucleic acid probe or primer which specifically hybridizes to a nucleic acid molecule of any one of Claims 1-4; and
b) determining whether the nucleic acid probe or primer binds to a nucleic acid molecule in the sample to thereby detect the presence of a nucleic acid molecule of any one of Claims 1-4 in the sample.

35. The method of Claim 34, wherein the sample comprises mRNA molecules and is contacted with a nucleic acid probe.

36. Use of a kit for detecting a nucleic acid molecule of any one of Claims 1-4 in a sample, said kit comprising a nucleic acid probe or primer which specifically hybridizes to a nucleic acid molecule of any one of Claims 1-4 and instructions for use.

37. An in vitro method for identifying a compound which binds to a polypeptide of any one of Claims 9-16 comprising:
a) contacting a polypeptide of any one of Claims 9-16, or a cell expressing said polypeptide with a test compound; and
b) determining whether the polypeptide binds to the test compound.

38. The method of Claim 37 wherein the binding of the test compound to the polypeptide is detected by a method selected from the group consisting of:
a) detection of binding by direct detection of test compound/polypeptide binding;
b) detection of binding using a competition binding assay; and
c) detection of binding using an assay for detecting cellular signal transduction or cellular proliferation.

39. An in vitro method of modulating the cellular signal transduction or cellular proliferation activity of a polypeptide of any one of Claims 9-16 comprising contacting a polypeptide of any one of Claims 9-16 or a cell expressing said polypeptide with an antibody or immunologically active portion thereof of any one of Claims 17-28 in a sufficient concentration to modulate the cellular signal transduction or cellular proliferation activity of said polypeptide.

40. A method for identifying a compound which modulates the cellular signal transduction or cellular proliferation activity of a polypeptide of any one of Claims 9-16 comprising:
a) contacting a polypeptide of any one of Claims 9-16 with a test compound; and
b) determining the effect of the test compound on the cellular signal transduction or cellular proliferation activity of said polypeptide to thereby identify a compound which modulates the cellular signal transduction or cellular proliferation activity of said polypeptide.

41. An antibody or immunologically active portion thereof of any one of Claims 17-28 for use in therapy or diagnosis.

42. Use of an antibody or immunologically active portion thereof of any one of Claims 17-28 for the manufacture of a medicament for treatment of a developmental, differentiative, or proliferative disorder.

43. A polypeptide of any one of Claims 10-16 for use in therapy or diagnosis.

44. Use of a polypeptide according to any one of Claims 10-16 for the manufacture of a medicament for treatment of a developmental, differentiative, or proliferative disorder.

45. Use of a polypeptide according to any one of Claims 10-16 for the manufacture of a medicament or diagnostic for the treatment, prophylaxis or diagnosis of cancer.

46. A nucleic acid molecule of any one of Claims 1-6 for use in therapy or diagnosis.

47. Use of a nucleic acid molecule of any one of Claims 1-6 for the manufacture of a medicament for treatment of a developmental, differentiative, or proliferative disorder.

48. Use of an antibody or immunologically active portion thereof of any one of Claims 17-28 for the manufacture of a medicament or diagnostic for the treatment, prophylaxis or diagnosis of cancer.

49. Use of a nucleic acid molecule of any one of Claims 1-6 for the manufacture of a medicament or diagnostic for the treatment, prophylaxis or diagnosis of cancer.

50. A nonhuman host cell comprising a transgene that comprises the nucleic acid molecule of any one of Claims 1 a), 1b), 2, 3, or 4.

## Patentansprüche

1. Isoliertes Nukleinsäuremolekül ausgewählt aus der Gruppe bestehend aus:
a) einer Nukleinsäure, welche die SEQ. ID. Nr: 3 oder SEQ. ID. Nr: 7 umfasst;
b) einer Nukleinsäure, welche ein Polypeptid codiert, dass die Aminosäuresequenz der Aminosäurereste 24-350 der SEQ. ID. Nr: 2;
c) einer Nukleinsäure, welche ein Polypeptid codiert, das eine natürlich vorkommende allelische Variante der Aminosäuresequenz der Aminosäurereste 20-224 der SEQ. ID. Nr: 5, SEQ. ID. Nr: 11 oder SEQ. ID. Nr: 14 umfasst, worin die natürlich vorkommende allelische Variante die zelluläre Signalübertragung oder zelluläre Proliferation moduliert, und worin
(i) die Nukleinsäure, welche eine natürlich vorkommende allelische Variante der Aminosäuresequenz der Aminosäurereste 20-224 der SEQ. ID. Nr: 5 codiert, an das Komplement der SEQ. ID. Nr: 6 unter stringenten Waschbedingungen hybridisiert,
(ii) die Nukleinsäure, welche eine natürliche vorkommende allelische Variante der Aminosäuresequenz der SEQ. ID. Nr: 11 codiert, an das Komplement der SEQ. ID. Nr: 12 unter stringenten Waschbedingungen hybridisiert, und aus SEQ. ID. Nr: 12 besteht,
(iii) die Nukleinsäure, welche eine natürlich vorkommende allelische Variante der Aminosäuresequenz SEQ. ID. Nr: 14 codiert, an das Komplement der SEQ. ID. Nr: 15 unter stringenten Waschbedingungen hybridisiert,
wobei stringente Waschbedingungen 0,2 X SSC, 0,1% SDS bei 50-65°C sind;
d) einer Nukleinsäure, welche eine Nukleotidsequenz umfasst, die das Komplement von a), b), oder c) (i) oder c) (iii) ist;
e) einer Nukleinsäure bestehend aus SEQ. ID. Nr: 9; und
f) einem rekombinanten eukaryotischen Zellexpressions-Vektor, welcher die Nukleinsäure von a), b), c) (i), c) (iii) oder d) oder eine Nukleinsäure umfassend SEQ. ID. Nr: 9 umfasst

2. Isoliertes Nukleinsäuremolekül nach Anspruch 1, worin das isolierte Nukleinsäuremolekül eine Nukleotidsequenz umfasst, ausgewählt aus der Gruppe bestehend aus SEQ. ID. Nr: 1 und SEQ. ID. Nr: 3.

3. Isoliertes Nukleinsäuremolekül nach Anspruch 1, worin das isolierte Nukleinsäuremolekül ein Polypeptid codiert, welches eine Aminosäuresequenz umfasst, ausgewählt aus der Gruppe bestehend aus SEQ. ID. Nr: 2, den Aminosäureresten 20-350 der SEQ. ID. Nr: 2, den Aminosäureresten 22-350 der SEQ. ID. Nr: 2 und den Aminosäureresten 24-350 der SEQ. ID. Nr: 2.

4. Isoliertes Nukleinsäuremolekül nach Anspruch 1, worin das isolierte Nukleinsäuremolekül ein Polypeptid codiert, welches eine Aminosäuresequenz umfasst, ausgewählt aus der Gruppe bestehend aus SEQ. ID. Nr: 8 und den Aminosäureresten 21-266 der SEQ. ID. Nr: 8.

5. Isoliertes Nukleinsäuremolekül nach Anspruch 1a), 1b) oder 1c) (i) oder c) (iii), worin das isolierte Nukleinsäuremolekül weiter umfasst:
(i) eine Vektor-Nukleinsäuresequenz; und/oder
(ii) eine Nukleinsäuresequenz, welche ein heterologes Polypeptid codiert.

6. Isoliertes Nukleinsäuremolekül nach Anspruch 1f), worin das isolierte Nukleinsäuremolekül weiter eine Nukleinsäuresequenz umfasst, welche ein heterologes Polypeptid codiert.

7. Wirtszelle, welche den rekombinanten eukaryotischen Zellexpressions-Vektor von Anspruch 1 f) umfasst.

8. Wirtszelle, welche einen Vektor umfasst, der die Nukleinsäure nach einem der Ansprüche 1a), 1 b), 2 oder 3 umfasst, worin die Nukleinsäure eine rekombinante Nukleinsäure ist.

9. Isoliertes Polypeptid ausgewählt aus der Gruppe bestehend aus:
a) einem Polypeptid, welches mindestens 25 aufeinanderfolgende Aminosäuren der SEQ. ID. Nr: 5 oder SEQ. ID. Nr: 14 oder mindestens 100 aufeinanderfolgende Aminosäuren der SEQ. ID. Nr: 11 umfasst, oder einem Polypeptid, welches eine Aminosäuresequenz ausgewählt aus der Gruppe umfassend SEQ. ID. Nr: 8 und Aminosäureresten 21-266 der SEQ. ID. Nr: 8 umfasst, welches Polypeptid die zelluläre Signalübertragung oder zelluläre Proliferation moduliert;
b) einem Polypeptid, welches eine natürlich vorkommende allelische Variante der Aminosäuresequenz der Aminosäurereste 20-224 der SEQ. ID. Nr: 5, der Aminosäurereste 21-266 der SEQ. ID. Nr: 8, oder SEQ. ID. Nr: 14. ist, worin die natürlich vorkommende allelische Variante die zelluläre Signalübertragung oder zelluläre Proliferation moduliert und worin
(i) die natürlich vorkommende allelische Variante der Aminosäuresequenz der Aminosäurereste 20-224 der SEQ. ID. Nr: 5 von einem Nukleinsäuremolekül codiert wird, welches an das Komplement der SEQ. ID. Nr: 6 unter stringenten Waschbedingungen hybridisiert,
(ii) die natürlich vorkommende allelische Variante der Aminosäuresequenz der SEQ. ID. Nr: 8 von einem Nukleinsäuremolekül codiert wird, welches an das Komplement der SEQ. ID. Nr: 9 unter stringenten Waschbedingungen hybridisiert, und worin das durch die Nukleinsäure codierte Protein mindestens 85% homolog zu SEQ. ID. Nr: 8 ist, und
(iii) die natürlich vorkommende allelische Variante der Aminosäuresequenz der SEQ. ID. Nr: 14 von einem Nukleinsäuremolekül codiert wird, welches an das Komplement der SEQ. ID. Nr: 15 unter stringenten Waschbedingungen hybridisiert,
und wobei stringente Waschbedingungen 0,2 X SSC, 0,1% SDS bei 50-65°C sind;
c) einem Polypeptid, welches die Aminosäurereste 24-350 der SEQ. ID. Nr: 2 umfasst; und
d) einem Polypeptid, welches eine Aminosäuresequenz umfasst, die zu mindestens 80% Aminosäuresequenz-Identität mit der Aminosäuresequenz der SEQ. ID. Nr: 5 oder SEQ. ID. Nr: 11 über jeweils die gesamte Länge der SEQ. ID. Nr: 5 oder SEQ. ID. Nr: 11 aufweist, oder einem Polypeptid, welches eine Aminosäuresequenz umfasst, welche zu mindestens 85% Aminosäuresequenz-Identität mit der Aminosäuresequenz der SEQ. ID. Nr: 8 über die gesamte Länge der SEQ. ID. Nr: 8 aufweist, wobei das Polypeptide die zelluläre Signalübertragung oder zelluläre Proliferation moduliert.

10. Isoliertes Polypeptid nach Anspruch 9, worin das Polypeptid mindestens 50 aufeinanderfolgende Aminosäuren der SEQ. ID. Nr: 5 oder SEQ. ID. Nr: 11 umfasst, und worin das Polypeptid die zelluläre Signalübertragung oder zelluläre Proliferation moduliert.

11. Isoliertes Polypeptid nach Anspruch 9, worin das Polypeptid mindestens 100 aufeinanderfolgende Aminosäuren der SEQ. ID. Nr: 5, SEQ. ID. Nr: 11 oder SEQ. ID. Nr: 14 umfasst, und worin das Polypeptid die zelluläre Signalübertragung oder zelluläre Proliferation moduliert.

12. Isoliertes Polypeptid nach Anspruch 9, worin das Polypeptid SEQ. ID. Nr: 8 umfasst, und worin das Polypeptid die zelluläre Signalübertragung oder zelluläre Proliferation moduliert.

13. Isoliertes Polypeptid nach Anspruch 9, worin das isolierte Polypeptid eine Aminosäuresequenz umfasst, ausgewählt aus der Gruppe bestehend aus SEQ. ID. Nr: 8 und den Aminosäureresten 21-266 der SEQ. ID. Nr:8.

14. Isoliertes Polypeptid nach Anspruch 9, worin das isolierte Polypeptid eine Aminosäuresequenz umfasst, ausgewählt aus der Gruppe bestehend aus SEQ. ID. Nr: 2, den Aminosäureresten 20-350 der SEQ. ID. Nr: 2, den Aminosäureresten 22-350 der SEQ. ID. Nr: 2 und den Aminosäureresten 24-350 der SEQ. ID. Nr: 2.

15. Isoliertes Polypeptid nach Anspruch 9, worin das isolierte Polypeptid eine Aminosäuresequenz umfasst, ausgewählt aus der Gruppe bestehend aus SEQ. ID. Nr: 5, den Aminosäureresten 20-224 der SEQ. ID. Nr: 5, SEQ. ID. Nr: 11 und SEQ. ID. Nr: 14.

16. Isoliertes Polypeptid nach einem der Ansprüche 10 bis 15, welches weiter ein heterologes Polypeptid umfasst

17. Antikörper oder immunologisch aktiver Teil davon, welcher spezifisch an ein Polypeptid bindet, ausgewählt aus der Gruppe bestehend aus:
a) einem Polypeptid bestehend aus mindestens 15 aufeinanderfolgenden Aminosäuren der SEQ. ID. Nr: 5, SEQ. ID. Nr: 11 oder SEQ. ID. Nr: 14, oder einem Polypeptid bestehend aus mindestens 30 aufeinanderfolgenden Aminosäuren der SEQ. ID. Nr: 8;
b) einem Polypeptid, welches aus einer natürlich vorkommenden allelischen Variante der Aminosäuresequenz der Aminosäurereste 20-224 der SEQ. ID. Nr: 5, der Aminosäurereste 21-266 der SEQ. ID. Nr: 8, SEQ. ID. Nr: 11 oder SEQ. ID. Nr: 14 besteht, worin die natürlich vorkommende allelische Variante die zelluläre Signalübertragung oder zelluläre Proliferation moduliert und worin
(i) die natürlich vorkommende allelische Variante der Aminosäuresequenz der Aminosäurereste 20-224 der SEQ. ID. Nr: 5 von einem Nukleinsäuremolekül codiert wird, welches an das Komplement der SEQ.ID. Nr: 6 unter stringenten Waschbedingungen hybridisiert,
(ii) die natürlich vorkommende allelische Variante der Aminosäuresequenz der Aminosäurereste 21-266 der SEQ. ID. Nr: 8 von einem Nukleinsäuremolekül codiert wird, welches an das Komplement der SEQ. ID. Nr: 9 unter stringenten Waschbedingungen hybridisiert,
(iii) die natürlich vorkommende allelische Variante der Aminosäuresequenz der SEQ. ID. Nr: 11 von einem Nukleinsäuremolekül codiert wird, welches an das Komplement der SEQ. ID. Nr: 12 unter stringenten Waschbedingungen hybridisiert, und
(iv) die natürlich vorkommende allelische Variante der Aminosäuresequenz der SEQ. ID. Nr: 14 von einem Nukleinsäuremolekül codiert wird, welches an das Komplement der SEQ. ID. Nr: 15 unter stringenten Waschbedingungen hybridisiert,
und worin stringente Waschbedingungen 0,2 X SSC, 0,1% STS bei 50-65°C sind.

18. Antikörper oder immunologisch aktiver Teil davon nach Anspruch 17, worin der Antikörper oder der immunologisch aktive Teil davon spezifisch an ein Polypeptid bindet, welches aus 15 aufeinanderfolgenden Aminosäuren der SEQ. ID. Nr: 5, SEQ. ID. Nr: 11 oder SEQ. ID. Nr: 14 besteht

19. Antikörper oder immunologisch aktiver Teil davon nach Anspruch 17, worin der Antikörper oder der immunologisch aktive Teil davon spezifisch an ein Polypeptid bindet, das aus 20 aufeinanderfolgenden Aminosäuren der SEQ. ID. Nr: 5, der SEQ. ID. Nr: 11 oder der SEQ. ID. Nr: 14 besteht.

20. Antikörper oder immunologisch aktiver Teil davon nach Anspruch 17, worin der Antikörper oder der immunologisch aktive Teil davon spezifisch an ein Polypeptid bindet, das aus 30 aufeinanderfolgenden Aminosäuren der SEQ. ID. Nr: 5, der SEQ. ID. Nr: 8, der SEQ. ID. Nr: 11 oder der SEQ. ID. Nr: 14 besteht

21. Antikörper oder immunologisch aktiver Teil davon nach Anspruch 17, worin der Antikörper oder der immunologisch aktive Teil davon spezifisch an ein Polypeptid bindet, das aus einer Aminosäuresequenz besteht, ausgewählt aus der Gruppe bestehend aus SEQ. ID. Nr: 5, SEQ. ID. Nr: 8, SEQ. ID. Nr: 11, und SEQ. ID. Nr: 14.

22. Antikörper oder immunologisch aktiver Teil davon nach Anspruch 21, worin der Antikörper oder der immunologisch aktive Teil davon spezifisch an ein Polypeptid bindet, das aus der Aminosäuresequenz der Aminosäurereste 21-266 der SEQ. ID. Nr: 8 besteht.

23. Antikörper oder immunologisch aktiver Teil davon nach Anspruch 21, worin der Antikörper oder der immunologisch aktive Teil davon spezifisch an ein Polypeptid bindet, das aus der Aminosäuresequenz der Aminosäurereste 20-224 der SEQ. ID. Nr: 5 besteht.

24. Antikörper oder immunologisch aktiver Teil davon nach einem der Ansprüche 17-23, worin der Antikörper oder der immunologisch aktive Teil davon ausgewählt ist aus der Gruppe bestehend aus einem menschlichen Antikörper, einem humanisierten monoklonalen Antikörper, einem chimären monoklonalen Antikörper und einem immunologisch aktiven Teil von einem der vorstehenden.

25. Antikörper oder immunologisch aktiver Teil davon nach einem der Ansprüche 17-23, worin der Antikörper oder immunologisch aktive Teil davon ein monoklonaler Antikörper oder ein immunologisch aktiver Teil eines monoklonalen Antikörpers ist.

26. Antikörper oder immunologisch aktiver Teil davon nach einem der Ansprüche 17-23, worin der Antikörper oder immunologisch aktive Teil davon ein immunologisch aktiver Teil ist, ausgewählt aus der Gruppe bestehend aus einem Fab-Fragment und einem F(ab')₂-Fragment.

27. Antikörper oder immunologisch aktiver Teil davon nach einem der Ansprüche 17-26, worin der Antikörper oder immunologisch aktive Teil davon einen nachweisbaren Marker umfasst.

28. Antikörper oder immunologisch aktiver Teil davon nach Anspruch 27, worin der nachweisbare Marker ein Enzym, eine prosthetische Gruppe, ein fluoreszierendes Material, ein lumineszierendes Material, ein biolumineszierendes Material oder ein radioaktives Material ist.

29. Verfahren zur Herstellung eines Polypeptids nach Anspruch 9, welches umfasst: Züchten der Wirtszelle nach Anspruch 7 unter Bedingungen, bei denen der rekombinante Expressionsvektor exprimiert und das Polypeptid hergestellt wird.

30. Verfahren zur Herstellung eines Polypeptids nach Anspruch 13 oder Anspruch 14, welches umfasst: Züchten der Wirtszelle nach Anspruch 8 unter Bedingungen, bei denen die rekombinante Nukleinsäure exprimiert wird und das Polypeptid hergestellt wird.

31. Verfahren zum Nachweis der Anwesenheit eines Polypeptids nach einem der Ansprüche 9-16 in einer Probe, umfassend:
a) Inkontaktbringen der Probe mit einem Antikörper oder immunologisch aktiven Teil davon nach einem der Ansprüche 17-28; und
b) Bestimmen, ob der Antikörper an ein Polypeptid in der Probe bindet, um **dadurch** die Anwesenheit des Polypeptids nach einem der Ansprüche 9-16 in der Probe zu erfassen.

32. Kit, umfassend einen Antikörper oder immunologisch aktiven Teil davon nach einem der Ansprüche 17-28 und Instruktionen zur Verwendung.

33. Verwendung eines Kits zum Nachweis eines Polypeptids nach einem der Ansprüche 9-16 in einer Probe, worin der Kit einen Antikörper oder immunologisch aktiven Teil davon nach einem der Ansprüche 17-28 und Instruktionen zur Verwendung umfasst.

34. Verfahren zum Nachweis der Anwesenheit eines Nukleinsäuremoleküls nach einem der Ansprüche 1-4 in einer Probe, welches umfasst:
a) Inkontaktbringen der Probe mit einer Nukleinsäuresonde oder einem Primer, die/der spezifisch an ein Nukleinsäuremolekül nach einem der Ansprüche 1-4 hybridisiert; und
b) Bestimmen, ob die Nukleinsäuresonde oder der Primer an ein Nukleinsäuremolekül in der Probe bindet, um damit die Anwesenheit eines Nukleinsäuremoleküls nach einem der Ansprüche 1-4 in der Probe zu erfassen.

35. Verfahren nach Anspruch 34, wobei die Probe mRNA-Moleküle umfasst und mit einer Nukleinsäuresonde in Kontakt gebracht wird.

36. Verwendung eines Kits zum Nachweis eines Nukleinsäuremoleküls nach einem der Ansprüche 1-4 in einer Probe, wobei der Kit eine Nukleinsäuresonde oder einen Primer, die/der spezifisch an ein Nukleinsäuremolekül nach einem der Ansprüche 1-4 hybridisiert, und Instruktionen zur Verwendung umfasst.

37. In vitro Verfahren zur Identifizierung einer Verbindung, welche an ein Polypeptid nach einem der Ansprüche 9-16 bindet, umfassend:
a) Inkontaktbringen eines Polypeptids nach einem der Ansprüche 9-16, oder einer Zelle, welche das Polypeptid exprimiert, mit einer Testverbindung; und
b) Bestimmen, ob das Polypeptid an die Testverbindung bindet.

38. Verfahren nach Anspruch 37, wobei die Bindung der Testverbindung an das Polypeptid durch ein Verfahren erfaßt wird, das ausgewählt ist aus der Gruppe bestehend aus:
(a) Bestimmen der Bindung durch direktes Erfassen der Bindung der Testverbindung/des Polypeptids,
(b) Erfassen der Bindung unter Verwendung eines kompetetiven Bindungsassays, und
(c) Erfassen der Bindung unter Verwendung eines Assays zum Nachweis der zellulären Signalübertragung und zellulären Proliferation.

39. In vitro Verfahren zur Modulierung der Aktivität eines Polypeptids nach einem der Ansprüche 9-16 bei der zellulären Signalübertragung oder zellulären Proliferation, umfassend: Inkontaktbringen eines Polypeptids nach einem der Ansprüche 9-16 oder einer Zelle, die das Polypeptid exprimiert, mit einem Antikörper oder immunologisch aktiven Teil davon nach einem der Ansprüche 17-28 in einer ausreichenden Konzentration, um die Aktivität des Polypeptids hinsichtlich zellulärer Signalübertragung und zellulärer Proliferation zu modulieren.

40. Verfahren zum Identifizieren einer Verbindung, welche die Aktivität eines Polypeptids nach einem der Ansprüche 9-16 hinsichtlich zellulärer Signalübertragung oder zellulärer Proliferation moduliert, umfassend:
a) Inkontaktbringen eines Polypeptids nach einem der Ansprüche 9-16 mit einer Testverbindung; und
b) Bestimmen der Auswirkung der Testverbindung auf die Aktivität des Polypeptids hinsichtlich zellulärer Signalübertragung oder zellulärer Proliferation, wobei eine Verbindung identifiziert wird, welche die Aktivität des Polypeptids hinsichtlich zellulärer Signalübertragung oder zellulärer Proliferation moduliert.

41. Antikörper oder immunologisch aktiver Teil davon nach einem der Ansprüche 17-28 zur Verwendung in der Therapie oder Diagnose.

42. Verwendung eines Antikörpers oder immunologisch aktiven Teils davon nach einem der Ansprüche 17-28 zur Herstellung eines Medikaments zur Behandlung einer Störung der Entwicklung, Differentiation oder Proliferation.

43. Polypeptid nach einem der Ansprüche 10-16 zur Verwendung in der Therapie oder Diagnose.

44. Verwendung eines Polypeptids nach einem der Ansprüche 10-16 zur Herstellung eines Medikaments zur Behandlung einer Störung der Entwicklung, Differentiation oder Proliferation.

45. Verwendung eines Polypeptids nach einem der Ansprüche 10-16 zur Herstellung eines Medikaments oder diagnostischen Mittels zur Behandlung, Prophylaxe oder Diagnose von Krebs.

46. Nukleinsäuremolekül nach einem der Ansprüche 1-6 zur Verwendung in der Therapie oder Diagnose.

47. Verwendung eines Nukleinsäuremoleküls nach einem der Ansprüche 1-6 zur Herstellung eines Medikaments zur Behandlung einer Störung der Entwicklung, Differentiation oder Proliferation.

48. Verwendung eines Antikörpers oder immunologisch aktiven Teils davon nach einem der Ansprüche 17-28 zur Herstellung eines Medikaments oder diagnostischen Mittels zur Behandlung, Prophylaxe, oder Diagnose von Krebs.

49. Verwendung eines Nukleinsäuremoleküls nach einem der Ansprüche 1-6 zur Herstellung eines Medikaments oder diagnostischen Mittels zur Behandlung, Prophylaxe oder Diagnose von Krebs.

50. Nicht-menschliche Wirtszelle, welche ein Transgen umfasst, das das Nukleinsäuremolekül nach einem der Ansprüche 1a), 1b), 2, 3 oder 4 umfasst.

## Revendications

1. Molécule d'acide nucléique isolée choisie dans le groupe constitué par :
a) un acide nucléique comprenant SEQ ID NO : 3 ou SEQ ID NO : 7 ;
b) un acide nucléique qui code un polypeptide comprenant la séquence d'acides aminés des résidus d'acides aminés 24 à 350 de SEQ ID NO : 2 ;
c) un acide nucléique qui code un polypeptide qui comprend un variant allélique existant à l'état naturel de la séquence d'acides aminés des résidus d'acides aminés 20 à 224 de SEQ ID NO : 5, SEQ ID NO : 11 ou SEQ ID NO : 14, où ledit variant allélique existant à l'état naturel module la transduction des signaux cellulaires ou la prolifération cellulaire et où (i) ledit acide nucléique qui code un variant allélique existant à l'état naturel de la séquence d'acides aminés des résidus d'acides aminés 20 à 224 de SEQ ID NO : 5 s'hybride au complémentaire de SEQ ID NO : 6 dans des conditions stringentes de lavage, (ii) ledit acide nucléique qui code un variant allélique existant à l'état naturel de la séquence d'acides aminés de SEQ ID NO : 11 s'hybride au complémentaire de SEQ ID NO : 12 dans des conditions stringentes de lavage, et est constitué de SEQ ID NO : 12, (iii) ledit acide nucléique qui code un variant allélique existant à l'état naturel de la séquence d'acides aminés de SEQ ID NO : 14 s'hybride au complémentaire de SEQ ID NO : 15 dans des conditions stringentes de lavage, où les conditions stringentes de lavage sont du SSC 0,2X, 0,1% de SDS à 50 à 65°C ;
d) un acide nucléique comprenant une séquence nucléotidique complémentaire de a), b), c) (i) ou c) (iii) ;
e) un acide nucléique constitué de SEQ ID NO : 9 ; et
f) un vecteur d'expression recombinant pour cellule eucaryote comprenant l'acide nucléique de a), b), c) (i), c) (iii) ou d), ou un acide nucléique comprenant SEQ ID NO : 9.

2. Molécule d'acide nucléique isolée selon la revendication 1, où ladite molécule d'acide nucléique isolée comprend une séquence nucléotidique choisie dans le groupe constitué par SEQ ID NO : 1 et SEQ ID NO : 3.

3. Molécule d'acide nucléique isolée selon la revendication 1, où ladite molécule d'acide nucléique isolée code un polypeptide comprenant une séquence d'acides aminés choisie dans le groupe constitué par SEQ ID NO : 2, les résidus d'acides aminés 20 à 350 de SEQ ID NO : 2 et les résidus d'acides aminés 24 à 350 de SEQ ID NO : 2.

4. Molécule d'acide nucléique isolée selon la revendication 1, où ladite molécule d'acide nucléique isolée code un polypeptide comprenant une séquence d'acides aminés choisie dans le groupe constitué par SEQ ID NO : 8, et les résidus d'acides aminés 21 à 266 de SEQ ID NO : 8.

5. Molécule d'acide nucléique isolé selon la revendication 1a), 1b) ou 1c) (i) ou 1c (iii), où ladite molécule d'acide nucléique isolée comprend en outre ;
i) une séquence d'acide nucléique de vecteur ; et/ou
ii) une séquence d'acide nucléique codant un polypeptide hétérologue.

6. Molécule d'acide nucléique isolée selon la revendication 1f), où ladite molécule d'acide nucléique isolée comprend en outre une séquence d'acide nucléique codant un polypeptide hétérologue.

7. Cellule hôte comprenant le vecteur d'expression recombinant pour cellule eucaryote selon la revendication 1f).

8. Cellule hôte comprenant un vecteur comprenant l'acide nucléique selon l'une quelconque des revendications 1a), 1b), 2 ou 3, où ledit acide nucléique est un acide nucléique recombinant.

9. Polypeptide isolé choisi dans le groupe constitué par :
a) un polypeptide comprenant au moins 25 acides aminés contigus de SEQ ID NO : 5 ou SEQ ID NO : 14, ou un polypeptide comprenant au moins 100 acides aminés contigus de SEQ ID NO : 11, ou un polypeptide comprenant une séquence d'acides aminés choisie dans le groupe constitué par SEQ ID NO : 8 et les résidus d'acides aminés 21 à 266 de SEQ ID NO : 8, lequel polypeptide module la transduction des signaux cellulaires ou la prolifération cellulaire ;
b) un polypeptide comprenant un variant allélique existant à l'état naturel de la séquence d'acides aminés des résidus d'acides aminés 20 à 224 de SEQ ID NO : 5, des résidus d'acides aminés 21 à 266 de SEQ ID NO : 8, ou de SEQ ID NO : 14, où ledit variant allélique existant à l'état naturel module la transduction des signaux cellulaires ou la prolifération cellulaire et où (i) ledit variant allélique existant à l'état naturel de la séquence d'acides aminés des résidus d'acides aminés 20 à 224 de SEQ ID NO : 5 est codé par une molécule d'acide nucléique qui s'hybride au complémentaire de SEQ ID NO : 6 dans des conditions stringentes de lavage, (ii) ledit variant allélique existant à l'état naturel de la séquence d'acides aminés de SEQ ID NO : 8 est codé par une molécule d'acide nucléique qui s'hybride au complémentaire de SEQ ID NO : 9 dans des conditions stringentes de lavage, et où la protéine codée par l'acide nucléique est au moins homologue à 85 % avec SEQ ID NO : 8, et (iii) ledit variant allélique existant à l'état naturel de la séquence d'acides aminés de SEQ ID NO : 14 est codé par une molécule d'acide nucléique qui s'hybride au complémentaire de SEQ ID NO : 15 dans des conditions stringentes de lavage, et où les conditions stringentes de lavage sont du SSC 0,2X, 0,1% de SDS à 50 à 65°C ;
c) un polypeptide comprenant les résidus d'acides aminés 24 à 350 de SEQ ID NO : 2 ; et
d) un polypeptide comprenant une séquence d'acides aminés qui présente au moins 80 % d'identité de séquence d'acides aminés avec la séquence d'acides aminés de SEQ ID NO : 5 ou SEQ ID NO : 11 sur toute la longueur de SEQ ID NO : 5 ou SEQ ID NO : 11, respectivement, ou un polypeptide comprenant une séquence d'acides aminés qui présente au moins 85 % d'identité de séquence d'acides aminés avec la séquence d'acides aminés de SEQ ID NO : 8 sur toute la longueur de SEQ ID NO : 8, où le polypeptide module la transduction des signaux cellulaires ou la prolifération cellulaire.

10. Polypeptide isolé selon la revendication 9, où ledit polypeptide comprend au moins 50 acides aminés contigus de SEQ ID NO : 5 ou SEQ ID NO : 14 et où ledit polypeptide module la transduction des signaux cellulaires ou la prolifération cellulaire.

11. Polypeptide isolé selon la revendication 9, où ledit polypeptide comprend au moins 100 acides aminés contigus de SEQ ID NO : 5, SEQ ID NO : 11 ou SEQ ID NO : 14 et où ledit polypeptide module la transduction des signaux cellulaires ou la prolifération cellulaire.

12. Polypeptide isolé selon la revendication 9, où ledit polypeptide comprend SEQ ID NO : 8 et où ledit polypeptide module la transduction des signaux cellulaires ou la prolifération cellulaire.

13. Polypeptide isolé selon la revendication 9, où ledit polypeptide isolé comprend une séquence d'acides aminés choisie dans le groupe constitué par SEQ ID NO : 8 et les résidus d'acides aminés 21 à 266 de SEQ ID NO : 8.

14. Polypeptide isolé selon la revendication 9, où ledit polypeptide isolé comprend une séquence d'acides aminés choisie dans le groupe constitué par SEQ ID NO : 2, les résidus d'acides aminés 20 à 350 de SEQ ID NO : 2, les résidus d'acides aminés 22 à 350 de SEQ ID NO : 2, et les résidus d'acides aminés 24 à 350 de SEQ ID NO : 2.

15. Polypeptide isolé selon la revendication 9, où ledit polypeptide isolé comprend une séquence d'acides aminés choisie dans le groupe constitué par SEQ ID NO : 5, les résidus d'acides aminés 20 à 224 de SEQ ID NO : 5, SEQ ID NO : 11 et SEQ ID NO : 14.

16. Polypeptide isolé selon l'une quelconque des revendications 10 à 15, comprenant en outre un polypeptide hétérologue.

17. Anticorps ou partie immunologiquement active de celui-ci qui se lie spécifiquement à un polypeptide choisi dans le groupe constitué par :
a) un polypeptide constitué de 15 acides aminés contigus de SEQ ID NO : 5, SEQ ID NO : 11 ou SEQ ID NO : 14 ou un polypeptide constitués de 30 acides aminés contigus de SEQ ID NO : 8 ; et
b) un polypeptide constitué d'un variant allélique existant à l'état naturel de la séquence d'acides aminés des résidus d'acides aminés 20 à 224 de SEQ ID NO : 5, des résidus d'acides aminés 21 à 266 de SEQ ID NO : 8, SEQ ID NO : 11 ou SEQ ID NO : 14, où ledit variant allélique existant à l'état naturel module la transduction des signaux cellulaires ou la prolifération cellulaire et où (i) ledit variant allélique existant à l'état naturel de la séquence d'acides aminés des résidus d'acides aminés 20 à 224 de SEQ ID NO : 5 est codé par une molécule d'acide nucléique qui s'hybride au complémentaire de SEQ ID NO : 6 dans des conditions stringentes de lavage, (ii) ledit variant allélique existant à l'état naturel de la séquence d'acides aminés des résidus d'acides aminés 21 à 266 de SEQ ID NO : 8 est codé par une molécule d'acide nucléique qui s'hybride au complémentaire de SEQ ID NO : 9 dans des conditions stringentes de lavage, (iii) ledit variant allélique existant à l'état naturel de la séquence d'acides aminés de SEQ ID NO : 11 est codé par une molécule d'acide nucléique qui s'hybride au complémentaire de SEQ ID NO : 12 dans des conditions stringentes de lavage, (iv) ledit variant allélique existant à l'état naturel de la séquence d'acides aminés de SEQ ID NO : 14 est codé par une molécule d'acide nucléique qui s'hybride au complémentaire de SEQ ID NO : 15 dans des conditions stringentes de lavage, et où les conditions stringentes de lavage sont du SSC 0,2X, 0,1 % de SDS à 50 à 65°C.

18. Anticorps ou partie immunologiquement active de celui-ci selon la revendication 17, où ledit anticorps ou ladite partie immunologiquement active de celui-ci se lie spécifiquement à un polypeptide constitué de 15 acides aminés contigus de SEQ ID NO : 5, SEQ ID NO : 11 ou SEQ ID NO : 14.

19. Anticorps ou partie immunologiquement active de celui-ci selon la revendication 17, où ledit anticorps ou ladite partie immunologiquement active de celui-ci se lie spécifiquement à un polypeptide constitué de 20 acides aminés contigus de SEQ ID NO : 5, SEQ ID NO : 11 ou SEQ ID NO : 14.

20. Anticorps ou partie immunologiquement active de celui-ci selon la revendication 17, où ledit anticorps ou ladite partie immunologiquement active de celui-ci se lie spécifiquement à un polypeptide constitué de 30 acides aminés contigus de SEQ ID NO : 5, SEQ ID NO : 8, SEQ ID NO : 11 ou SEQ ID NO : 14.

21. Anticorps ou partie immunologiquement active de celui-ci selon la revendication 18, où ledit anticorps ou ladite partie immunologiquement active de celui-ci lie spécifiquement un polypeptide qui est constitué d'une séquence d'acides aminés choisie dans le groupe constitué par SEQ ID NO : 5, SEQ ID NO : 8, SEQ ID NO : 11 et SEQ ID NO : 14.

22. Anticorps ou partie immunologiquement active de celui-ci selon la revendication 21, où ledit anticorps ou ladite partie immunologiquement active de celui-ci lie spécifiquement un polypeptide qui est constitué de la séquence d'acides aminés des résidus d'acides aminés 21 à 266 de SEQ ID NO : 8.

23. Anticorps ou partie immunologiquement active de celui-ci selon la revendication 21, où ledit anticorps ou ladite partie immunologiquement active de celui-ci lie spécifiquement un polypeptide qui est constitué de la séquence d'acides aminés des résidus d'acides aminés 20 à 224 de SEQ ID NO : 5.

24. Anticorps ou partie immunologiquement active de celui-ci selon l'une quelconque des revendications 17 à 23, où ledit anticorps ou ladite partie immunologiquement active de celui-ci est choisi dans le groupe constitué par un anticorps humain, un anticorps monoclonal humanisé, un anticorps monoclonal chimérique et une partie immunologiquement active de l'un quelconque des précédents.

25. Anticorps ou partie immunologiquement active de celui-ci selon l'une quelconque des revendications 17 à 23, où ledit anticorps ou ladite partie immunologiquement active de celui-ci est un anticorps monoclonal ou une partie immunologiquement active d'un anticorps monoclonal.

26. Anticorps ou partie immunologiquement active de celui-ci selon l'une quelconque des revendications 17 à 23, où ledit anticorps ou ladite partie immunologiquement active de celui-ci est une partie immunologiquement active choisie dans le groupe constitué par un fragment Fab et un fragment F(ab')₂.

27. Anticorps ou partie immunologiquement active de celui-ci selon l'une quelconque des revendications 17 à 26, où ledit anticorps ou ladite partie immunologiquement active de celui-ci comprend un marqueur détectable.

28. Anticorps ou partie immunologiquement active de celui-ci selon la revendication 27, où ledit marqueur détectable est une enzyme, un groupe prothétique, un matériau fluorescent, un matériau luminescent, un matériau bioluminescent ou un matériau radioactif.

29. Procédé de production d'un polypeptide selon la revendication 9, comprenant la culture de la cellule hôte selon la revendication 7 dans des conditions dans lesquelles le vecteur d'expression recombinant est exprimé, et ledit polypeptide est produit.

30. Procédé de production d'un polypeptide selon la revendication 13 ou la revendication 14, comprenant la culture de la cellule hôte selon la revendication 8 dans des conditions dans lesquelles l'acide nucléique recombinant est exprimé, et ledit polypeptide est produit.

31. Procédé de détection de la présence d'un polypeptide selon l'une quelconque des revendications 9 à 16 dans un échantillon comprenant :
a) la mise en contact de l'échantillon avec un anticorps ou une partie immunologiquement active de celui-ci selon l'une quelconque des revendications 17 à 28 ; et
b) la détermination si ledit anticorps se lie à un polypeptide dans l'échantillon pour détecter de cette manière la présence dudit polypeptide selon l'une quelconque des revendications 9 à 16 dans l'échantillon.

32. Kit comprenant un anticorps ou une partie immunologiquement active de celui-ci selon l'une quelconque des revendications 17 à 28 et des instructions d'utilisation.

33. Utilisation d'un kit pour la détection d'un polypeptide selon l'une quelconque des revendications 9 à 16 dans un échantillon, ledit kit comprenant un anticorps ou une partie immunologiquement active de celui-ci selon l'une quelconque des revendications 17 à 28 et des instructions d'utilisation.

34. Procédé de détection de la présence d'une molécule d'acide nucléique selon l'une quelconque des revendications 1 à 4 dans un échantillon comprenant :
a) la mise en contact de l'échantillon avec une sonde ou une amorce d'acide nucléique qui s'hybride spécifiquement à une molécule d'acide nucléique selon l'une quelconque des revendications 1 à 4 ; et
b) la détermination si la sonde ou l'amorce d'acide nucléique se lie à une molécule d'acide nucléique dans l'échantillon pour détecter de cette manière la présence d'une molécule d'acide nucléique selon l'une quelconque des revendications 1 à 4 dans l'échantillon.

35. Procédé selon la revendication 34, où l'échantillon comprend des molécules d'ARNm et est mis en contact avec une sonde d'acide nucléique.

36. Utilisation d'un kit pour la détection d'une molécule d'acide nucléique selon l'une quelconque des revendications 1 à 4 dans un échantillon, ledit kit comprenant une sonde ou une amorce d'acide nucléique qui s'hybride spécifiquement à une molécule d'acide nucléique selon l'une quelconque des revendications 1 à 4 et des instructions d'utilisation.

37. Procédé *in vitro* d'identification d'un composé qui se lie à un polypeptide selon l'une quelconque des revendications 9 à 16 comprenant :
a) la mise en contact d'un polypeptide selon l'une quelconque des revendications 9 à 16, ou d'une cellule exprimant ledit polypeptide avec un composé à tester ; et
b) la détermination si le polypeptide se lie au composé à tester.

38. Procédé selon la revendication 37, dans lequel la liaison du composé à tester au polypeptide est détectée par un procédé choisi dans le groupe constitué par :
a) la détection de la liaison par détection directe de la liaison du composé à tester/polypeptide ;
b) la détection de la liaison en utilisant un test de liaison avec compétition ; et
c) la détection de la liaison en utilisant un test pour détecter la transduction des signaux cellulaires ou la prolifération cellulaire.

39. Procédé *in vitro* de modulation de l'activité de transduction des signaux cellulaires ou de prolifération cellulaire d'un polypeptide selon l'une quelconque des revendications 9 à 16 comprenant la mise en contact d'un polypeptide selon l'une quelconque des revendications 9 à 16 ou d'une cellule exprimant le polypeptide avec un anticorps ou une partie immunologiquement active de celui-ci selon l'une quelconque des revendications 17 à 28 dans une concentration suffisante pour moduler l'activité de transduction des signaux cellulaires ou de prolifération cellulaire dudit polypeptide.

40. Procédé d'identification d'un composé qui module l'activité de transduction des signaux cellulaires ou de prolifération cellulaire d'un polypeptide selon l'une quelconque des revendications 9 à 16 comprenant :
a) la mise en contact d'un polypeptide selon l'une quelconque des revendications 9 à 16 avec un composé à tester ; et
b) la détermination de l'effet du composé à tester sur l'activité de transduction des signaux cellulaires ou de prolifération cellulaire dudit polypeptide pour identifier de cette manière un composé qui module l'activité de transduction des signaux cellulaires ou de prolifération cellulaire dudit polypeptide.

41. Anticorps ou partie immunologiquement active de celui-ci selon l'une quelconque des revendications 17 à 28, pour une utilisation en thérapie ou en diagnostic.

42. Utilisation d'un anticorps ou d'une partie immunologiquement active de celui-ci selon l'une quelconque des revendications 17 à 28 pour la fabrication d'un médicament destiné au traitement d'un trouble du développement, de la différenciation ou de la prolifération.

43. Polypeptide selon l'une quelconque des revendications 10 à 16, pour une utilisation en thérapie ou en diagnostic.

44. Utilisation d'un polypeptide selon l'une quelconque des revendications 10 à 16 pour la fabrication d'un médicament destiné au traitement d'un trouble du développement, de la différenciation ou de la prolifération.

45. Utilisation d'un polypeptide selon l'une quelconque des revendications 10 à 16 pour la fabrication d'un médicament ou d'un diagnostic pour le traitement, la prophylaxie ou le diagnostic d'un cancer.

46. Molécule d'acide nucléique selon l'une quelconque des revendications 1 à 6, pour une utilisation en thérapie ou en diagnostic.

47. Utilisation d'une molécule d'acide nucléique selon l'une quelconque des revendications 1 à 6 pour la fabrication d'un médicament destiné au traitement d'un trouble du développement, de la différenciation ou de la prolifération.

48. Utilisation d'un anticorps ou d'une partie immunologiquement active de celui-ci selon l'une quelconque des revendications 17 à 28 pour la fabrication d'un médicament ou d'un diagnostic pour le traitement, la prophylaxie ou le diagnostic d'un cancer.

49. Utilisation d'une molécule d'acide nucléique selon l'une quelconque des revendications 1 à 6, pour la fabrication d'un médicament ou d'un diagnostic pour le traitement, la prophylaxie ou le diagnostic d'un cancer.

50. Cellule hôte non humaine comprenant un transgène qui comprend la molécule d'acide nucléique selon l'une quelconque des revendications 1a), 1b), 2, 3 ou 4.


### SEQUENCE LISTING

(1) GENERAL INFORMATION:
   (i) APPLICANT:
      (A) NAME: MILLENNIUM BIOTHERAPEUTICS, INC.
      (B) STREET: 620 MEMORIAL DRIVE
      (C) CITY: CAMBRIDGE
      (D) STATE: MASSACHUSETTS
      (E) COUNTRY: US
      (F) POSTAL CODE: 02319
      (G) TELEPHONE:
      (H) TELEFAX:
   (ii) TITLE OF INVENTION: NOVEL CRSP PROTEIN AND NUCLEIC ACID MOLECULES AND USES THEREFOR
   (iii) NUMBER OF SEQUENCES: 18
   (iv) CORRESPONDENCE ADDRESS:
      (A) ADDRESSEE: LAHIVE & COCKFIELD, LLP
      (B) STREET: 28 STATE STREET
      (C) CITY: BOSTON
      (D) STATE: MASSACHUSETTS
      (E) COUNTRY: US
      (F) ZIP: 02109
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: PCT/US98/
      (B) FILING DATE: 16 APRIL 1998
      (C) CLASSIFICATION:
   (vii) PRIOR APPLICATION DATA:
      (A) APPLICATION NUMBER: US 08/843,704
      (B) FILING DATE: 16 APRIL 1997
      (C) APPLICATION NUMBER: US 08/842,898
      (D) FILING DATE: 17 APRIL 1997
      (E) APPLICATION NUMBER: 60/071,589
      (F) FILING DATE: 15 JANUARY 1998
      (G) APPLICATION NUMBER: 09/009,802
      (H) FILING DATE: 20 JANUARY 1998
   (viii) ATTORNEY/AGENT INFORMATION:
      (A) NAME: MANDRAGOURAS, AMY E.
      (B) REGISTRATION NUMBER: 36,207
      (C) REFERENCE/DOCKET NUMBER: MEI-008CPPC
   (ix) TELECOMMUNICATION INFORMATION:
      (A) TELEPHONE: (617)227-7400
      (B) TELEFAX: (617)742-4214
(2) INFORMATION FOR SEQ ID NO:1:
   (i) SEQUENCE CHARACTERISTICS:
      (A) LENGTH: 2479 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 38..1087
   (xi) SEQUENCE DESCRIPTION: SEQ ID NO:1:
   GGCACGAGGG GGCGGCGGCT GCGGGCGCAG AGCGGAG ATG CAG CGG CTT GGG GCC      55
                                           Met Gln Arg Leu Gly Ala
                                             1               5
   ACC CTG CTG TGC CTG CTG CTG GCG GCG GCG GTC CCC ACG GCC CCC GCG     103
   Thr Leu Leu Cys Leu Leu Leu Ala Ala Ala Val Pro Thr Ala Pro Ala
               10                  15                  20
   CCC GCT CCG ACG GCG ACC TCG GCT CCA GTC AAG CCC GGC CCG GCT CTC     151
   Pro Ala Pro Thr Ala Thr Ser Ala Pro Val Lys Pro Gly Pro Ala Leu
           25                  30                  35
   AGC TAC CCG CAG GAG GAG GCC ACC CTC AAT GAG ATG TTC CGC GAG GTT     199
   Ser Tyr Pro Gln Glu Glu Ala Thr Leu Asn Glu Met Phe Arg Glu Val
       40                  45                  50
   GAG GAA CTG ATG GAG GAC ACG CAG CAC AAA TTG CGC AGC GCG GTG GAA     247
   Glu Glu Leu Met Glu Asp Thr Gln His Lys Leu Arg Ser Ala Val Glu
    55                  60                  65                  70
   GAG ATG GAG GCA GAA GAA GCT GCT GCT AAA GCA TCA TCA GAA GTG AAC     295
   Glu Met Glu Ala Glu Glu Ala Ala Ala Lys Ala Ser Ser Glu Val Asn
                   75                  80                  85
   CTG GCA AAC TTA CCT CCC AGC TAT CAC AAT GAG ACC AAC ACA GAC ACG     343
   Leu Ala Asn Leu Pro Pro Ser Tyr His Asn Glu Thr Asn Thr Asp Thr
               90                  95                 100
   AAC GTT GGA AAT AAT ACC ATC CAT GTG CAC CGA GAA ATT CAC AAG ATA     391
   Asn Val Gly Asn Asn Thr Ile His Val His Arg Glu Ile His Lys Ile
           105                 110                 115
   ACC AAC AAC CAG ACT GGA CAA ATG GTC TTT TCA GAG ACA GTT ATC ACA      439
   Thr Asn Asn Gln Thr Gly Gln Met Val Phe Ser Glu Thr Val Ile Thr
       120                 125                 130
   TCT GTG GGA GAC GAA GAA GGC AGA AGG AGC CAC GAG TGC ATC ATC GAC      487
   Ser Val Gly Asp Glu Glu Gly Arg Arg Ser His Glu Cys Ile Ile Asp
   135                 140                 145                 150
   GAG GAC TGT GGG CCC AGC ATG TAC TGC CAG TTT GCC AGC TTC CAG TAC      535
   Glu Asp Cys Gly Pro Ser Met Tyr Cys Gln Phe Ala Ser Phe Gln Tyr
                   155                 160                 165
   ACC TGC CAG CCA TGC CGG GGC CAG AGG ATG CTC TGC ACC CGG GAC AGT      583
   Thr Cys Gln Pro Cys Arg Gly Gln Arg Met Leu Cys Thr Arg Asp Ser
               170                 175                 180
   GAG TGC TGT GGA GAC CAG CTG TGT GTC TGG GGT CAC TGC ACC AAA ATG      631
   Glu Cys Cys Gly Asp Gln Leu Cys Val Trp Gly His Cys Thr Lys Met
           185                 190                 195
   GCC ACC AGG GGC AGC AAT GGG ACC ATC TGT GAC AAC CAG AGG GAC TGC      679
   Ala Thr Arg Gly Ser Asn Gly Thr Ile Cys Asp Asn Gln Arg Asp Cys
       200                 205                 210
   CAG CCG GGG CTG TGC TGT GCC TTC CAG AGA GGC CTG CTG TTC CCT GTG      727
   Gln Pro Gly Leu Cys Cys Ala Phe Gln Arg Gly Leu Leu Phe Pro Val
   215                 220                 225                 230
   TGC ACA CCC CTG CCC GTG GAG GGC GAG CTT TGC CAT GAC CCC GCC AGC      775
   Cys Thr Pro Leu Pro Val Glu Gly Glu Leu Cys His Asp Pro Ala Ser
                   235                 240                 245
   CGG CTT CTG GAC CTC ATC ACC TGG GAG CTA GAG CCT GAT GGA GCC TTG      823
   Arg Leu Leu Asp Leu Ile Thr Trp Glu Leu Glu Pro Asp Gly Ala Leu
               250                 255                 260
   GAC CGA TGC CCT TGT GCC AGT GGC CTC CTC TGC CAG CCC CAC AGC CAC      871
   Asp Arg Cys Pro Cys Ala Ser Gly Leu Leu Cys Gln Pro His Ser His
           265                 270                 275
   AGC CTG GTG TAT GTG TGC AAG CCG ACC TTC GTG GGG AGC CGT GAC CAA      919
   Ser Leu Val Tyr Val Cys Lys Pro Thr Phe Val Gly Ser Arg Asp Gln
       280                 285                 290
   GAT GGG GAG ATC CTG CTG CCC AGA GAG GTC CCC GAT GAG TAT GAA GTT      967
   Asp Gly Glu Ile Leu Leu Pro Arg Glu Val Pro Asp Glu Tyr Glu Val
   295                 300                 305                 310
   GGC AGC TTC ATG GAG GAG GTG CGC CAG GAG CTG GAG GAC CTG GAG AGG     1015
   Gly Ser Phe Met Glu Glu Val Arg Gln Glu Leu Glu Asp Leu Glu Arg
                   315                 320                 325
   AGC CTG ACT GAA GAG ATG GCG CTG AGG GAG CCT GCG GCT GCC GCC GCT     1063
   Ser Leu Thr Glu Glu Met Ala Leu Arg Glu Pro Ala Ala Ala Ala Ala
               330                 335                 340
   GCA CTG CTG GGA AGG GAA GAG ATT TAGATCTGGA CCAGGCTGTG GGTAGATGTG   1117
   Ala Leu Leu Gly Arg Glu Glu Ile
           345                350
   CAATAGAAAT AGCTAATTTA TTTCCCCANG TGTGTGCTTT AAGCGTGGGC TGACCAGGCT  1177
   TCTTCCTACA TCTTCTTCCC AGTAAGTTTC CCCTCTGGCT TGACAGCATG AGGTGTTGTG  1237
   CATTTGTTCA GCTCCCCCAG GCTGTTCTCC AGGCTTCACA GTCTGGTGCT TGGGAGAGTC  1297
   AGGCAGGGTT AAACTGCAGG AGCAGTTTGC CACCCCTGTC CAGATTATTG GCTGCTTTGC  1357
   CTCTACCAGT TGGCAGACAG CCGTTTGTTC TACATGGCTT TGATAATTGT TTGAGGGGAG  1417
   GAGATGGAAA CAATGTGGAG TCTCCCTCTG ATTGGTTTTG GGGAAATGTG GAGAAGAGTG  1477
   CCCTGCTTTG CAAACATCAA CCTGGCAAAA ATGCAACAAA TGAATTTTCC ACGCAGTTCT  1537
   TTCCATGGGC ATAGGTAAGC TGTGCCTTCA GCTGTTGCAG ATGAAATGTT CTGTTCACCC  1597
   TGCATTACAT GTGTTTATTC ATCCAGCAGT GTTGCTCAGC TCCTACCTCT GTGCCAGGGC  1657
   AGCATTTTCA TATCCAAGAT CAATTCCCTC TCTCAGCACA GCCTGGGGAG GGGGTCATTG  1717
   TTCTCCTCGT CCATCAGGGA TTTCAGAGGC TCAGAGACTG CAAGCTGCTT GCCCAAGTCA  1777
   CACAGCTAGT GAAGACCAGA GCAGTTTCAT CTGGTTGTGA CTCTAAGCTC AGTGCTCTCT  1837
   CCACTACCCC ACACCAGCCT TGGTGCCACC AAAAGTGCTC CCCAAAAGGA AGGAGAATGG  1897
   GATTTTTCTT TTGAGGCATG CACATCTGGA ATTAAGGTCA AACTAATTCT CACATCCCTC  1957
   TAAAAGTAAA CTACTGTTAG GAACAGCAGT GTTCTCACAG TGTGGGGCAG CCGTCCTTCT  2017
   AATGAAGACA ATGATATTGA CACTGTCCCT CTTTGGCAGT TGCATTAGTA ACTTTGAAAG  2077
   GTATATGACT GAGCGTAGCA TACAGGTTAA CCTGCAGAAA CAGTACTTAG GTAATTGTAG  2137
   GGCGAGGATT ATAAATGAAA TTTGCAAAAT CACTTAGCAG CAACTGAAGA CAATTATCAA  2197
   CCACGTGGAG AAAATCAAAC CGAGCAGGGC TGTGTGAAAC ATGGTTGTAA TATGCGACTG  2257
   CGAACACTGA ACTCTACGCC ACTCCACAAA TGATGTTTTC AGGTGTCATG GACTGTTGCC  2317
   ACCATGTATT CATCCAGAGT TCTTAAAGTT TAAAGTTGCA CATGATTGTA TAAGCATGCT  2377
   TTCTTTGAGT TTTAAATTAT GTATAAACAT AAGTTGCATT TAGAAATCAA GCATAAATCA  2437
   CTTCAACTGC TAAAAAAAAA AAAAAAAAAA AAAAAAAAAA AA                     2479
(2) INFORMATION FOR SEQ ID NO:2:
   (i) SEQUENCE CHARACTERISTICS:
      (A) LENGTH: 350 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Gln Arg Leu Gly Ala Thr Leu Leu Cys Leu Leu Leu Ala Ala Ala
   1 5 10 15
   Val Pro Thr Ala Pro Ala Pro Ala Pro Thr Ala Thr Ser Ala Pro Val
   20 25 30
   Lys Pro Gly Pro Ala Leu Ser Tyr Pro Gln Glu Glu Ala Thr Leu Asn
   35 40 45
   Glu Met Phe Arg Glu Val Glu Glu Leu Met Glu Asp Thr Gln His Lys
   50 55 60
   Leu Arg Ser Ala Val Glu Glu Met Glu Ala Glu Glu Ala Ala Ala Lys
   65 70 75 80
   Ala Ser Ser Glu Val Asn Leu Ala Asn Leu Pro Pro Ser Tyr His Asn
   85 90 95
   Glu Thr Asn Thr Asp Thr Asn Val Gly Asn Asn Thr Ile His Val His
   100 105 110
   Arg Glu Ile His Lys Ile Thr Asn Asn Gln Thr Gly Gln Met Val Phe
   115 120 125
   Ser Glu Thr Val Ile Thr Ser Val Gly Asp Glu Glu Gly Arg Arg Ser
   130 135 140
   His Glu Cys Ile Ile Asp Glu Asp Cys Gly Pro Ser Met Tyr Cys Gln
   145 150 155 160
   Phe Ala Ser Phe Gln Tyr Thr Cys Gln Pro Cys Arg Gly Gln Arg Met
   165 170 175
   Leu Cys Thr Arg Asp Ser Glu Cys Cys Gly Asp Gln Leu Cys Val Trp
   180 185 190
   Gly His Cys Thr Lys Met Ala Thr Arg Gly Ser Asn Gly Thr Ile Cys
   195 200 205
   Asp Asn Gln Arg Asp Cys Gln Pro Gly Leu Cys Cys Ala Phe Gln Arg
   210 215 220
   Gly Leu Leu Phe Pro Val Cys Thr Pro Leu Pro Val Glu Gly Glu Leu
   225 230 235 240
   Cys His Asp Pro Ala Ser Arg Leu Leu Asp Leu Ile Thr Trp Glu Leu
                   245                     250                 255
   Glu Pro Asp Gly Ala Leu Asp Arg Cys Pro Cys Ala Ser Gly Leu Leu
               260                 265                 270
   Cys Gln Pro His Ser His Ser Leu Val Tyr Val Cys Lys Pro Thr Phe
           275                 280                 285
   Val Gly Ser Arg Asp Gln Asp Gly Glu Ile Leu Leu Pro Arg Glu Val
       290                 295                 300
   Pro Asp Glu Tyr Glu Val Gly Ser Phe Met Glu Glu Val Arg Gln Glu
   305                 310                 315                     320
   Leu Glu Asp Leu Glu Arg Ser Leu Thr Glu Glu Met Ala Leu Arg Glu
                   325                     330                 335
   Pro Ala Ala Ala Ala Ala Ala Leu Leu Gly Arg Glu Glu Ile
               340                     345                 350
(2) INFORMATION FOR SEQ ID NO:3:
   (i) SEQUENCE CHARACTERISTICS:
      (A) LENGTH: 1050 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..1050
   (xi) SEQUENCE DESCRIPTION: SEQ ID NO:3:
   ATG CAG CGG CTT GGG GCC ACC CTG CTG TGC CTG CTG CTG GCG GCG GCG      48
   Met Gln Arg Leu Gly Ala Thr Leu Leu Cys Leu Leu Leu Ala Ala Ala
    1               5                  10                  15
   GTC CCC ACG GCC CCC GCG CCC GCT CCG ACG GCG ACC TCG GCT CCA GTC      96
   Val Pro Thr Ala Pro Ala Pro Ala Pro Thr Ala Thr Ser Ala Pro Val
               20                  25                  30
   AAG CCC GGC CCG GCT CTC AGC TAC CCG CAG GAG GAG GCC ACC CTC AAT     144
   Lys Pro Gly Pro Ala Leu Ser Tyr Pro Gln Glu Glu Ala Thr Leu Asn
           35                  40                  45
   GAG ATG TTC CGC GAG GTT GAG GAA CTG ATG GAG GAC ACG CAG CAC AAA     192
   Glu Met Phe Arg Glu Val Glu Glu Leu Met Glu Asp Thr Gln His Lys
       50                  55                  60
   TTG CGC AGC GCG GTG GAA GAG ATG GAG GCA GAA GAA GCT GCT GCT AAA      240
   Leu Arg Ser Ala Val Glu Glu Met Glu Ala Glu Glu Ala Ala Ala Lys
    65                 70                  75                  80
   GCA TCA TCA GAA GTG AAC CTG GCA AAC TTA CCT CCC AGC TAT CAC AAT      288
   Ala Ser Ser Glu Val Asn Leu Ala Asn Leu Pro Pro Ser Tyr His Asn
                   85                  90                  95
   GAG ACC AAC ACA GAC ACG AAC GTT GGA AAT AAT ACC ATC CAT GTG CAC      336
   Glu Thr Asn Thr Asp Thr Asn Val Gly Asn Asn Thr Ile His Val His
               100                 105                 110
   CGA GAA ATT CAC AAG ATA ACC AAC AAC CAG ACT GGA CAA ATG GTC TTT      384
   Arg Glu Ile His Lys Ile Thr Asn Asn Gln Thr Gly Gln Met Val Phe
           115                 120                 125
   TCA GAG ACA GTT ATC ACA TCT GTG GGA GAC GAA GAA GGC AGA AGG AGC      432
   Ser Glu Thr Val Ile Thr Ser Val Gly Asp Glu Glu Gly Arg Arg Ser
       130                 135                 140
   CAC GAG TGC ATC ATC GAC GAG GAC TGT GGG CCC AGC ATG TAC TGC CAG      480
   His Glu Cys Ile Ile Asp Glu Asp Cys Gly Pro Ser Met Tyr Cys Gln
   145                 150                 155                 160
   TTT GCC AGC TTC CAG TAC ACC TGC CAG CCA TGC CGG GGC CAG AGG ATG      528
   Phe Ala Ser Phe Gln Tyr Thr Cys Gln Pro Cys Arg Gly Gln Arg Met
                   165                 170                 175
   CTC TGC ACC CGG GAC AGT GAG TGC TGT GGA GAC CAG CTG TGT GTC TGG      576
   Leu Cys Thr Arg Asp Ser Glu Cys Cys Gly Asp Gln Leu Cys Val Trp
               180                 185                 190
   GGT CAC TGC ACC AAA ATG GCC ACC AGG GGC AGC AAT GGG ACC ATC TGT      624
   Gly His Cys Thr Lys Met Ala Thr Arg Gly Ser Asn Gly Thr Ile Cys
           195                 200                 205
   GAC AAC CAG AGG GAC TGC CAG CCG GGG CTG TGC TGT GCC TTC CAG AGA      672
   Asp Asn Gln Arg Asp Cys Gln Pro Gly Leu Cys Cys Ala Phe Gln Arg
       210                 215                 220
   GGC CTG CTG TTC CCT GTG TGC ACA CCC CTG CCC GTG GAG GGC GAG CTT      720
   Gly Leu Leu Phe Pro Val Cys Thr Pro Leu Pro Val Glu Gly Glu Leu
   225                 230                 235                 240
   TGC CAT GAC CCC GCC AGC CGG CTT CTG GAC CTC ATC ACC TGG GAG CTA      768
   Cys His Asp Pro Ala Ser Arg Leu Leu Asp Leu Ile Thr Trp Glu Leu
                   245                 250                 255
   GAG CCT GAT GGA GCC TTG GAC CGA TGC CCT TGT GCC AGT GGC CTC CTC      816
   Glu Pro Asp Gly Ala Leu Asp Arg Cys Pro Cys Ala Ser Gly Leu Leu
               260                 265                 270
   TGC CAG CCC CAC AGC CAC AGC CTG GTG TAT GTG TGC AAG CCG ACC TTC      864
   Cys Gln Pro His Ser His Ser Leu Val Tyr Val Cys Lys Pro Thr Phe
           275                 280                 285
   GTG GGG AGC CGT GAC CAA GAT GGG GAG ATC CTG CTG CCC AGA GAG GTC      912
   Val Gly Ser Arg Asp Gln Asp Gly Glu Ile Leu Leu Pro Arg Glu Val
       290                 295                     300
   CCC GAT GAG TAT GAA GTT GGC AGC TTC ATG GAG GAG GTG CGC CAG GAG      960
   Pro Asp Glu Tyr Glu Val Gly Ser Phe Met Glu Glu Val Arg Gln Glu
   305                 310                     315                 320
   CTG GAG GAC CTG GAG AGG AGC CTG ACT GAA GAG ATG GCG CTG AGG GAG     1008
   Leu Glu Asp Leu Glu Arg Ser Leu Thr Glu Glu Met Ala Leu Arg Glu
                   325                     330                 335
   CCT GCG GCT GCC GCC GCT GCA CTG CTG GGA AGG GAA GAG ATT             1050
   Pro Ala Ala Ala Ala Ala Ala Leu Leu Gly Arg Glu Glu Ile
               340                 345                     350
(2) INFORMATION FOR SEQ ID NO:4:
   (i) SEQUENCE CHARACTERISTICS:
      (A) LENGTH: 848 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 125..796
   (xi) SEQUENCE DESCRIPTION: SEQ ID NO:4:
   GAATTCGGCA CGAGAGACGA CGTGCTGAGC TGCCAGCTTA GTGGAAGCTC TGCTCTGGGT    60
   GGAGAGCAGC CTCGCTTTGG TGACGCACAG TGCTGGGACC CTCCAGGAGC CCCGGGATTG   120
   AAGG ATG GTG GCG GCC GTC CTG CTG GGG CTG AGC TGG CTC TGC TCT CCC    169
        Met Val Ala Ala Val Leu Leu Gly Leu Ser Trp Leu Cys Ser Pro
          1               5                  10                  15
   CTG GGA GCT CTG GTC CTG GAC TTC AAC AAC ATC AGG AGC TCT GCT GAC     217
   Leu Gly Ala Leu Val Leu Asp Phe Asn Asn Ile Arg Ser Ser Ala Asp
                    20                  25                  30
   CTG CAT GGG GCC CGG AAG GGC TCA CAG TGC CTG TCT GAC ACG GAC TGC     265
   Leu His Gly Ala Arg Lys Gly Ser Gln Cys Leu Ser Asp Thr Asp Cys
                35                  40                  45
   AAT ACC AGA AAG TTC TGC CTC CAG CCC CGC GAT GAG AAG CCG TTC TGT     313
   Asn Thr Arg Lys Phe Cys Leu Gln Pro Arg Asp Glu Lys Pro Phe Cys
            50                  55                  60
   GCT ACA TGT CGT GGG TTG CGG AGG AGG TGC CAG CGA GAT GCC ATG TGC      361
   Ala Thr Cys Arg Gly Leu Arg Arg Arg Cys Gln Arg Asp Ala Met Cys
        65                  70                  75
   TGC CCT GGG ACA CTC TGT GTG AAC GAT GTT TGT ACT ACG ATG GAA GAT      409
   Cys Pro Gly Thr Leu Cys Val Asn Asp Val Cys Thr Thr Met Glu Asp
    80                  85                  90                  95
   GCA ACC CCA ATA TTA GAA AGG CAG CTT GAT GAG CAA GAT GGC ACA CAT      457
   Ala Thr Pro Ile Leu Glu Arg Gln Leu Asp Glu Gln Asp Gly Thr His
                   100                 105                 110
   GCA GAA GGA ACA ACT GGG CAC CCA GTC CAG GAA AAC CAA CCC AAA AGG      505
   Ala Glu Gly Thr Thr Gly His Pro Val Gln Glu Asn Gln Pro Lys Arg
               115                 120                 125
   AAG CCA AGT ATT AAG AAA TCA CAA GGC AGG AAG GGA CAA GAG GGA GAA      553
   Lys Pro Ser Ile Lys Lys Ser Gln Gly Arg Lys Gly Gln Glu Gly Glu
           130                 135                 140
   AGT TGT CTG AGA ACT TTT GAC TGT GGC CCT GGA CTT TGC TGT GCT CGT      601
   Ser Cys Leu Arg Thr Phe Asp Cys Gly Pro Gly Leu Cys Cys Ala Arg
       145                 150                 155
   CAT TTT TGG ACG AAA ATT TGT AAG CCA GTC CTT TTG GAG GGA CAG GTC      649
   His Phe Trp Thr Lys Ile Cys Lys Pro Val Leu Leu Glu Gly Gln Val
   160                 165                 170                 175
   TGC TCC AGA AGA GGG CAT AAA GAC ACT GCT CAA GCT CCA GAA ATC TTC      697
   Cys Ser Arg Arg Gly His Lys Asp Thr Ala Gln Ala Pro Glu Ile Phe
                   180                 185                 190
   CAG CGT TGC GAC TGT GGC CCT GGA CTA CTG TGT CGA AGC CAA TTG ACC      745
   Gln Arg Cys Asp Cys Gly Pro Gly Leu Leu Cys Arg Ser Gln Leu Thr
               195                 200                 205
   AGC AAT CGG CAG CAT GCT CGA TTA AGA GTA TGC CAA AAA ATA GAA AAG      793
   Ser Asn Arg Gln His Ala Arg Leu Arg Val Cys Gln Lys Ile Glu Lys
           210                 215                 220
   CTA TAAATATTTC AAAATAAAGA AGAATCCACA TTGCAAAAAA AAAAAAAAAA AA        848
   Leu
(2) INFORMATION FOR SEQ ID NO:5:
   (i) SEQUENCE CHARACTERISTICS:
      (A) LENGTH: 224 amino acids
      (B) TYPE: amino acid
      (D) TOPOLOGY: linear
   (ii) MOLECULE TYPE: protein
   (xi) SEQUENCE DESCRIPTION: SEQ ID NO:5:
   Met Val Ala Ala Val Leu Leu Gly Leu Ser Trp Leu Cys Ser Pro Leu
   1               5                   10                  15
   Gly Ala Leu Val Leu Asp Phe Asn Asn Ile Arg Ser Ser Ala Asp Leu
               20                  25                  30
   His Gly Ala Arg Lys Gly Ser Gln Cys Leu Ser Asp Thr Asp Cys Asn
           35                  40                  45
   Thr Arg Lys Phe Cys Leu Gln Pro Arg Asp Glu Lys Pro Phe Cys Ala
       50                  55                  60
   Thr Cys Arg Gly Leu Arg Arg Arg Cys Gln Arg Asp Ala Met Cys Cys
   65                  70                  75                  80
   Pro Gly Thr Leu Cys Val Asn Asp Val Cys Thr Thr Met Glu Asp Ala
                   85                  90                  95
   Thr Pro Ile Leu Glu Arg Gln Leu Asp Glu Gln Asp Gly Thr His Ala
               100                 105                 110
   Glu Gly Thr Thr Gly His Pro Val Gln Glu Asn Gln Pro Lys Arg Lys
           115                 120                 125
   Pro Ser Ile Lys Lys Ser Gln Gly Arg Lys Gly Gln Glu Gly Glu Ser
       130                 135                 140
   Cys Leu Arg Thr Phe Asp Cys Gly Pro Gly Leu Cys Cys Ala Arg His
   145                 150                 155                 160
   Phe Trp Thr Lys Ile Cys Lys Pro Val Leu Leu Glu Gly Gln Val Cys
                   165                 170                 175
   Ser Arg Arg Gly His Lys Asp Thr Ala Gln Ala Pro Glu Ile Phe Gln
               180                 185                 190
   Arg Cys Asp Cys Gly Pro Gly Leu Leu Cys Arg Ser Gln Leu Thr Ser
           195                 200                 205
   Asn Arg Gln His Ala Arg Leu Arg Val Cys Gln Lys Ile Glu Lys Leu
       210                 215                 220
(2) INFORMATION FOR SEQ ID NO:6:
   (i) SEQUENCE CHARACTERISTICS:
      (A) LENGTH: 672 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..672
   (xi) SEQUENCE DESCRIPTION: SEQ ID NO:6:
   ATG GTG GCG GCC GTC CTG CTG GGG CTG AGC TGG CTC TGC TCT CCC CTG      48
   Met Val Ala Ala Val Leu Leu Gly Leu Ser Trp Leu Cys Ser Pro Leu
    1               5                  10                  15
   GGA GCT CTG GTC CTG GAC TTC AAC AAC ATC AGG AGC TCT GCT GAC CTG      96
   Gly Ala Leu Val Leu Asp Phe Asn Asn Ile Arg Ser Ser Ala Asp Leu
               20                  25                  30
   CAT GGG GCC CGG AAG GGC TCA CAG TGC CTG TCT GAC ACG GAC TGC AAT     144
   His Gly Ala Arg Lys Gly Ser Gln Cys Leu Ser Asp Thr Asp Cys Asn
           35                  40                  45
   ACC AGA AAG TTC TGC CTC CAG CCC CGC GAT GAG AAG CCG TTC TGT GCT     192
   Thr Arg Lys Phe Cys Leu Gln Pro Arg Asp Glu Lys Pro Phe Cys Ala
       50                  55                  60
   ACA TGT CGT GGG TTG CGG AGG AGG TGC CAG CGA GAT GCC ATG TGC TGC     240
   Thr Cys Arg Gly Leu Arg Arg Arg Cys Gln Arg Asp Ala Met Cys Cys
    65                  70                  75                  80
   CCT GGG ACA CTC TGT GTG AAC GAT GTT TGT ACT ACG ATG GAA GAT GCA     288
   Pro Gly Thr Leu Cys Val Asn Asp Val Cys Thr Thr Met Glu Asp Ala
                   85                  90                  95
   ACC CCA ATA TTA GAA AGG CAG CTT GAT GAG CAA GAT GGC ACA CAT GCA     336
   Thr Pro Ile Leu Glu Arg Gln Leu Asp Glu Gln Asp Gly Thr His Ala
               100                 105                 110
   GAA GGA ACA ACT GGG CAC CCA GTC CAG GAA AAC CAA CCC AAA AGG AAG     384
   Glu Gly Thr Thr Gly His Pro Val Gln Glu Asn Gln Pro Lys Arg Lys
           115                 120                 125
   CCA AGT ATT AAG AAA TCA CAA GGC AGG AAG GGA CAA GAG GGA GAA AGT     432
   Pro Ser Ile Lys Lys Ser Gln Gly Arg Lys Gly Gln Glu Gly Glu Ser
       130                 135                 140
   TGT CTG AGA ACT TTT GAC TGT GGC CCT GGA CTT TGC TGT GCT CGT CAT     480
   Cys Leu Arg Thr Phe Asp Cys Gly Pro Gly Leu Cys Cys Ala Arg His
   145                 150                 155                 160
   TTT TGG ACG AAA ATT TGT AAG CCA GTC CTT TTG GAG GGA CAG GTC TGC     528
   Phe Trp Thr Lys Ile Cys Lys Pro Val Leu Leu Glu Gly Gln Val Cys
                   165                 170                 175
   TCC AGA AGA GGG CAT AAA GAC ACT GCT CAA GCT CCA GAA ATC TTC CAG     576
   Ser Arg Arg Gly His Lys Asp Thr Ala Gln Ala Pro Glu Ile Phe Gln
               180                 185                 190
   CGT TGC GAC TGT GGC CCT GGA CTA CTG TGT CGA AGC CAA TTG ACC AGC      624
   Arg Cys Asp Cys Gly Pro Gly Leu Leu Cys Arg Ser Gln Leu Thr Ser
           195                 200                     205
   AAT CGG CAG CAT GCT CGA TTA AGA GTA TGC CAA AAA ATA GAA AAG CTA      672
   Asn Arg Gln His Ala Arg Leu Arg Val Cys Gln Lys Ile Glu Lys Leu
       210                 215                     220
(2) INFORMATION FOR SEQ ID NO:7:
   (i) SEQUENCE CHARACTERISTICS:
      (A) LENGTH: 1529 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 93..890
   (xi) SEQUENCE DESCRIPTION: SEQ ID NO:7:
   CCGGACGCGT GGGCGGCACG GTTTCGTGGG GACCCAGGCT TGCAAAGTGA CGGTCATTTT    60
   CTCTTTCTTT CTCCCTCTTG AGTCCTTCTG AG ATG ATG GCT CTG GGC GCA GCG     113
                                       Met Met Ala Leu Gly Ala Ala
                                        1               5
   GGA GCT ACC CGG GTC TTT GTC GCG ATG GTA GCG GCG GCT CTC GGC GGC     161
   Gly Ala Thr Arg Val Phe Val Ala Met Val Ala Ala Ala Leu Gly Gly
               10                  15                  20
   CAC CCT CTG CTG GGA GTG AGC GCC ACC TTG AAC TCG GTT CTC AAT TCC     209
   His Pro Leu Leu Gly Val Ser Ala Thr Leu Asn Ser Val Leu Asn Ser
           25                  30                  35
   AAC GCT ATC AAG AAC CTG CCC CCA CCG CTG GGC GGC GCT GCG GGG CAC     257
   Asn Ala Ile Lys Asn Leu Pro Pro Pro Leu Gly Gly Ala Ala Gly His
    40                  45                  50                  55
   CCA GGC TCT GCA GTC AGC GCC GCG CCG GGA ATC CTG TAC CCG GGC GGG     305
   Pro Gly Ser Ala Val Ser Ala Ala Pro Gly Ile Leu Tyr Pro Gly Gly
                   60                  65                  70
   AAT AAG TAC CAG ACC ATT GAC AAC TAC CAG CCG TAC CCG TGC GCA GAG     353
   Asn Lys Tyr Gln Thr Ile Asp Asn Tyr Gln Pro Tyr Pro Cys Ala Glu
               75                  80                  85
   GAC GAG GAG TGC GGC ACT GAT GAG TAC TGC GCT AGT CCC ACC CGC GGA     401
   Asp Glu Glu Cys Gly Thr Asp Glu Tyr Cys Ala Ser Pro Thr Arg Gly
           90                  95                 100
   GGG GAC GCA GGC GTG CAA ATC TGT CTC GCC TGC AGG AAG CGC CGA AAA       449
   Gly Asp Ala Gly Val Gln Ile Cys Leu Ala Cys Arg Lys Arg Arg Lys
       105                 110                 115
   CGC TGC ATG CGT CAC GCT ATG TGC TGC CCC GGG AAT TAC TGC AAA AAT       497
   Arg Cys Met Arg His Ala Met Cys Cys Pro Gly Asn Tyr Cys Lys Asn
   120                 125                 130                 135
   GGA ATA TGC GTG TCT TCT GAT CAA AAT CAT TTC CGA GGA GAA ATT GAG       545
   Gly Ile Cys Val Ser Ser Asp Gln Asn His Phe Arg Gly Glu Ile Glu
                   140                 145                 150
   GAA ACC ATC ACT GAA AGC TTT GGT AAT GAT CAT AGC ACC TTG GAT GGG       593
   Glu Thr Ile Thr Glu Ser Phe Gly Asn Asp His Ser Thr Leu Asp Gly
               155                 160                 165
   TAT TCC AGA AGA ACC ACC TTG TCT TCA AAA ATG TAT CAC ACC AAA GGA       641
   Tyr Ser Arg Arg Thr Thr Leu Ser Ser Lys Met Tyr His Thr Lys Gly
           170                 175                 180
   CAA GAA GGT TCT GTT TGT CTC CGG TCA TCA GAC TGT GCC TCA GGA TTG       689
   Gln Glu Gly Ser Val Cys Leu Arg Ser Ser Asp Cys Ala Ser Gly Leu
       185                 190                 195
   TGT TGT GCT AGA CAC TTC TGG TCC AAG ATC TGT AAA CCT GTC CTG AAA       737
   Cys Cys Ala Arg His Phe Trp Ser Lys Ile Cys Lys Pro Val Leu Lys
   200                 205                 210                 215
   GAA GGT CAA GTG TGT ACC AAG CAT AGG AGA AAA GGC TCT CAT GGA CTA       785
   Glu Gly Gln Val Cys Thr Lys His Arg Arg Lys Gly Ser His Gly Leu
                   220                 225                 230
   GAA ATA TTC CAG CGT TGT TAC TGT GGA GAA GGT CTG TCT TGC CGG ATA       833
   Glu Ile Phe Gln Arg Cys Tyr Cys Gly Glu Gly Leu Ser Cys Arg Ile
               235                 240                 245
   CAG AAA GAT CAC CAT CAA GCC AGT AAT TCT TCT AGG CTT CAC ACT TGT       881
   Gln Lys Asp His His Gln Ala Ser Asn Ser Ser Arg Leu His Thr Cys
           250                 255                 260
   CAG AGA CAC TAAACCAGCT ATCCAAAATG CAGTGAACTC CTTTTATATA               930
   Gln Arg His
       265
   ATAGATGCTA TGAAAACCTT TTATGACCTT CATCAACTCA ATCCTAAGGA TATACAAGTT     990
   CTGTGGTTTC AGTTAAGCAT TCCAATAACA CCTTCCAAAA ACCTGGAGTG TAAGAGCTTT    1050
   GTTTCTTTAT GGAACTCCCC TGTGATTGCA GTAAATTACT GTATTGTAAA TTCTCAGTGT    1110
   GGCACTTACC TGTAAATGCA ATGAAACTTT TAATTATTTT TCTAAAGGTG CTGCACTGCC    1170
   TATTTTTCCT CTTGTTATGT AAATTTTTGT ACACATTGAT TGTTATCTTG ACTGACAAAT    1230
   ATTCTATATT GAACTGAAGT AAATCATTTC AGCTTATAGT TCTTAAAAGC ATAACCCTTT    1290
   ACCCCATTTN ATTCTAGAGT CNAGAACGCA AGGATCTCTT GGAATGACAA ATGATAGGTA 1350
   CCTAAAATGT AACATGAAAA TACTAGCTTA TTTTCTGAAA TGTACTATCT TAATGCTTAA 1410
   ATTATATTTC CCTTTAGGCT GTGATAGTTT TTGAAATAAA ATTTAACATT TAATATCATG 1470
   AAATGKTATA AGTAGACATA AAAAAAAAAA AAAAAAAAAA AGGGCGGCCG CTAGACTAG 1529
(2) INFORMATION FOR SEQ ID NO:8:
   (i) SEQUENCE CHARACTERISTICS:
      (A) LENGTH: 266 amino acids
      (B) TYPE: amino acid
      (D) TOPOLOGY: linear
   (ii) MOLECULE TYPE: protein
   (xi) SEQUENCE DESCRIPTION: SEQ ID NO:8:
   Met Met Ala Leu Gly Ala Ala Gly Ala Thr Arg Val Phe Val Ala Met
   1 5 10 15
   Val Ala Ala Ala Leu Gly Gly His Pro Leu Leu Gly Val Ser Ala Thr
   20 25 30
   Leu Asn Ser Val Leu Asn Ser Asn Ala Ile Lys Asn Leu Pro Pro Pro
   35 40 45
   Leu Gly Gly Ala Ala Gly His Pro Gly Ser Ala Val Ser Ala Ala Pro
   50 55 60
   Gly Ile Leu Tyr Pro Gly Gly Asn Lys Tyr Gln Thr Ile Asp Asn Tyr
   65 70 75 80
   Gln Pro Tyr Pro Cys Ala Glu Asp Glu Glu Cys Gly Thr Asp Glu Tyr
   85 90 95
   Cys Ala Ser Pro Thr Arg Gly Gly Asp Ala Gly Val Gln Ile Cys Leu
   100 105 110
   Ala Cys Arg Lys Arg Arg Lys Arg Cys Met Arg His Ala Met Cys Cys
   115 120 125
   Pro Gly Asn Tyr Cys Lys Asn Gly Ile Cys Val Ser Ser Asp Gln Asn
   130 135 140
   His Phe Arg Gly Glu Ile Glu Glu Thr Ile Thr Glu Ser Phe Gly Asn
   145 150 155 160
   Asp His Ser Thr Leu Asp Gly Tyr Ser Arg Arg Thr Thr Leu Ser Ser
   165 170 175
   Lys Met Tyr His Thr Lys Gly Gln Glu Gly Ser Val Cys Leu Arg Ser
   180 185 190
   Ser Asp Cys Ala Ser Gly Leu Cys Cys Ala Arg His Phe Trp Ser Lys
   195 200 205
   Ile Cys Lys Pro Val Leu Lys Glu Gly Gln Val Cys Thr Lys His Arg
   210 215 220
   Arg Lys Gly Ser His Gly Leu Glu Ile Phe Gln Arg Cys Tyr Cys Gly
   225 230 235 240
   Glu Gly Leu Ser Cys Arg Ile Gln Lys Asp His His Gln Ala Ser Asn
   245 250 255
   Ser Ser Arg Leu His Thr Cys Gln Arg His
   260 265
(2) INFORMATION FOR SEQ ID NO:9:
   (i) SEQUENCE CHARACTERISTICS:
      (A) LENGTH: 798 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..798
   (xi) SEQUENCE DESCRIPTION: SEQ ID NO:9:
   ATG ATG GCT CTG GGC GCA GCG GGA GCT ACC CGG GTC TTT GTC GCG ATG       48
   Met Met Ala Leu Gly Ala Ala Gly Ala Thr Arg Val Phe Val Ala Met
     1               5                  10                  15
   GTA GCG GCG GCT CTC GGC GGC CAC CCT CTG CTG GGA GTG AGC GCC ACC       96
   Val Ala Ala Ala Leu Gly Gly His Pro Leu Leu Gly Val Ser Ala Thr
                20                  25                  30
   TTG AAC TCG GTT CTC AAT TCC AAC GCT ATC AAG AAC CTG CCC CCA CCG      144
   Leu Asn Ser Val Leu Asn Ser Asn Ala Ile Lys Asn Leu Pro Pro Pro
            35                  40                  45
   CTG GGC GGC GCT GCG GGG CAC CCA GGC TCT GCA GTC AGC GCC GCG CCG      192
   Leu Gly Gly Ala Ala Gly His Pro Gly Ser Ala Val Ser Ala Ala Pro
        50                  55                  60
   GGA ATC CTG TAC CCG GGC GGG AAT AAG TAC CAG ACC ATT GAC AAC TAC      240
   Gly Ile Leu Tyr Pro Gly Gly Asn Lys Tyr Gln Thr Ile Asp Asn Tyr
    65                  70                  75                  80
   CAG CCG TAC CCG TGC GCA GAG GAC GAG GAG TGC GGC ACT GAT GAG TAC      288
   Gln Pro Tyr Pro Cys Ala Glu Asp Glu Glu Cys Gly Thr Asp Glu Tyr
                    85                  90                  95
   TGC GCT AGT CCC ACC CGC GGA GGG GAC GCA GGC GTG CAA ATC TGT CTC      336
   Cys Ala Ser Pro Thr Arg Gly Gly Asp Ala Gly Val Gln Ile Cys Leu
               100                 105                 110
   GCC TGC AGG AAG CGC CGA AAA CGC TGC ATG CGT CAC GCT ATG TGC TGC      384
   Ala Cys Arg Lys Arg Arg Lys Arg Cys Met Arg His Ala Met Cys Cys
           115                 120                 125
   CCC GGG AAT TAC TGC AAA AAT GGA ATA TGC GTG TCT TCT GAT CAA AAT      432
   Pro Gly Asn Tyr Cys Lys Asn Gly Ile Cys Val Ser Ser Asp Gln Asn
       130                 135                 140
   CAT TTC CGA GGA GAA ATT GAG GAA ACC ATC ACT GAA AGC TTT GGT AAT      480
   His Phe Arg Gly Glu Ile Glu Glu Thr Ile Thr Glu Ser Phe Gly Asn
   145                 150                 155                 160
   GAT CAT AGC ACC TTG GAT GGG TAT TCC AGA AGA ACC ACC TTG TCT TCA      528
   Asp His Ser Thr Leu Asp Gly Tyr Ser Arg Arg Thr Thr Leu Ser Ser
                   165                 170                 175
   AAA ATG TAT CAC ACC AAA GGA CAA GAA GGT TCT GTT TGT CTC CGG TCA      576
   Lys Met Tyr His Thr Lys Gly Gln Glu Gly Ser Val Cys Leu Arg Ser
               180                 185                 190
   TCA GAC TGT GCC TCA GGA TTG TGT TGT GCT AGA CAC TTC TGG TCC AAG      624
   Ser Asp Cys Ala Ser Gly Leu Cys Cys Ala Arg His Phe Trp Ser Lys
           195                 200                 205
   ATC TGT AAA CCT GTC CTG AAA GAA GGT CAA GTG TGT ACC AAG CAT AGG      672
   Ile Cys Lys Pro Val Leu Lys Glu Gly Gln Val Cys Thr Lys His Arg
       210                 215                 220
   AGA AAA GGC TCT CAT GGA CTA GAA ATA TTC CAG CGT TGT TAC TGT GGA      720
   Arg Lys Gly Ser His Gly Leu Glu Ile Phe Gln Arg Cys Tyr Cys Gly
   225                 230                 235                 240
   GAA GGT CTG TCT TGC CGG ATA CAG AAA GAT CAC CAT CAA GCC AGT AAT      768
   Glu Gly Leu Ser Cys Arg Ile Gln Lys Asp His His Gln Ala Ser Asn
                   245                 250                 255
   TCT TCT AGG CTT CAC ACT TGT CAG AGA CAC                              798
   Ser Ser Arg Leu His Thr Cys Gln Arg His
               260                 265
(2) INFORMATION FOR SEQ ID NO:10:
   (i) SEQUENCE CHARACTERISTICS:
      (A) LENGTH: 702 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..537
   (xi) SEQUENCE DESCRIPTION: SEQ ID NO:10:
   GAA TTC GGC ACG AGG GTT GGG AGG TAT TGC CAC AGT CCC CAC CAA GGA      48
   Glu Phe Gly Thr Arg Val Gly Arg Tyr Cys His Ser Pro His Gln Gly
    1               5                  10                  15
   TCA TCG GCC TGC ATG GTG TGT CGG AGA AAA AAG AAG CGC TGC CAC CGA      96
   Ser Ser Ala Cys Met Val Cys Arg Arg Lys Lys Lys Arg Cys His Arg
               20                  25                  30
   GAT GGC ATG TGC TGC CCC AGT ACC CGC TGC AAT AAT GGC ATC TGT ATC     144
   Asp Gly Met Cys Cys Pro Ser Thr Arg Cys Asn Asn Gly Ile Cys Ile
           35                  40                  45
   CCA GTT ACT GAA AGC ATC TTA ACC CCT CAC ATC CCG GCT CTG GAT GGT     192
   Pro Val Thr Glu Ser Ile Leu Thr Pro His Ile Pro Ala Leu Asp Gly
       50                  55                  60
   ACT CGG CAC AGA GAT CGA AAC CAC GGT CAT TAC TCA AAC CAT GAC TTG     240
   Thr Arg His Arg Asp Arg Asn His Gly His Tyr Ser Asn His Asp Leu
   65                  70                  75                  80
   GGA TGG CAG AAT CTA GGA AGA CCA CAC ACT AAG ATG TCA CAT ATA AAA     288
   Gly Trp Gln Asn Leu Gly Arg Pro His Thr Lys Met Ser His Ile Lys
                   85                  90                  95
   GGG CAT GAA GGA GAC CCC TGC CTA CGA TCA TCA GAC TGC ATT GAA GGG     336
   Gly His Glu Gly Asp Pro Cys Leu Arg Ser Ser Asp Cys Ile Glu Gly
               100                 105                 110
   TTT TGC TGT GCT CGT CAT TTC TGG ACC AAA ATC TGC AAA CCA GTG CTC     384
   Phe Cys Cys Ala Arg His Phe Trp Thr Lys Ile Cys Lys Pro Val Leu
           115                 120                 125
   CAT CAG GGG GAA GTC TGT ACC AAA CAA CGC AAG AAG GGT TCT CAT GGG     432
   His Gln Gly Glu Val Cys Thr Lys Gln Arg Lys Lys Gly Ser His Gly
       130                 135                 140
   CTG GAA ATT TTC CAG CGT TGC GAC TGT GCG AAG GGC CTG TCT TGC AAA     480
   Leu Glu Ile Phe Gln Arg Cys Asp Cys Ala Lys Gly Leu Ser Cys Lys
   145                 150                 155                 160
   GTA TGG AAA GAT GCC ACC TAC TCC TCC AAA GCC AGA CTC CAT GTG TGT     528
   Val Trp Lys Asp Ala Thr Tyr Ser Ser Lys Ala Arg Leu His Val Cys
                   165                 170                 175
   CAG AAA ATT TGATCACCAT TGAGGAACAT CATCAATTGC AGACTGTGAA               577
   Gln Lys Ile
   GTTGTGTATT TAATGCATTA TAGCATGGTG GAAAATAAGG TTCAGATGCA GAAGAATGGC     637
   TAAAATAAGA AACGTGATAA GAATATAGAT GATCACAAAA AAAAAAAAAA AAAAGATGCG     697
   GCCGC                                                                 702
(2) INFORMATION FOR SEQ ID NO:11:
   (i) SEQUENCE CHARACTERISTICS:
      (A) LENGTH: 179 amino acids
      (B) TYPE: amino acid
      (D) TOPOLOGY: linear
   (ii) MOLECULE TYPE: protein
   (xi) SEQUENCE DESCRIPTION: SEQ ID NO:11:
   Glu Phe Gly Thr Arg Val Gly Arg Tyr Cys His Ser Pro His Gln Gly
    1               5                  10                  15
   Ser Ser Ala Cys Met Val Cys Arg Arg Lys Lys Lys Arg Cys His Arg
               20                  25                  30
   Asp Gly Met Cys Cys Pro Ser Thr Arg Cys Asn Asn Gly Ile Cys Ile
           35                  40                  45
   Pro Val Thr Glu Ser Ile Leu Thr Pro His Ile Pro Ala Leu Asp Gly
       50                  55                  60
   Thr Arg His Arg Asp Arg Asn His Gly His Tyr Ser Asn His Asp Leu
   65                  70                  75                  80
   Gly Trp Gln Asn Leu Gly Arg Pro His Thr Lys Met Ser His Ile Lys
                   85                  90                  95
   Gly His Glu Gly Asp Pro Cys Leu Arg Ser Ser Asp Cys Ile Glu Gly
               100                 105                 110
   Phe Cys Cys Ala Arg His Phe Trp Thr Lys Ile Cys Lys Pro Val Leu
           115                 120                 125
   His Gln Gly Glu Val Cys Thr Lys Gln Arg Lys Lys Gly Ser His Gly
       130                 135                 140
   Leu Glu Ile Phe Gln Arg Cys Asp Cys Ala Lys Gly Leu Ser Cys Lys
   145                 150                 155                 160
   Val Trp Lys Asp Ala Thr Tyr Ser Ser Lys Ala Arg Leu His Val Cys
                   165                 170                 175
   Gln Lys Ile
(2) INFORMATION FOR SEQ ID NO:12:
   (i) SEQUENCE CHARACTERISTICS:
      (A) LENGTH: 537 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..537
   (xi) SEQUENCE DESCRIPTION: SEQ ID NO:12:
   GAA TTC GGC ACG AGG GTT GGG AGG TAT TGC CAC AGT CCC CAC CAA GGA      48
   Glu Phe Gly Thr Arg Val Gly Arg Tyr Cys His Ser Pro His Gln Gly
    1               5                  10                  15
   TCA TCG GCC TGC ATG GTG TGT CGG AGA AAA AAG AAG CGC TGC CAC CGA      96
   Ser Ser Ala Cys Met Val Cys Arg Arg Lys Lys Lys Arg Cys His Arg
               20                  25                  30
   GAT GGC ATG TGC TGC CCC AGT ACC CGC TGC AAT AAT GGC ATC TGT ATC     144
   Asp Gly Met Cys Cys Pro Ser Thr Arg Cys Asn Asn Gly Ile Cys Ile
           35                  40                  45
   CCA GTT ACT GAA AGC ATC TTA ACC CCT CAC ATC CCG GCT CTG GAT GGT     192
   Pro Val Thr Glu Ser Ile Leu Thr Pro His Ile Pro Ala Leu Asp Gly
       50                  55                  60
   ACT CGG CAC AGA GAT CGA AAC CAC GGT CAT TAC TCA AAC CAT GAC TTG     240
   Thr Arg His Arg Asp Arg Asn His Gly His Tyr Ser Asn His Asp Leu
   65                  70                  75                  80
   GGA TGG CAG AAT CTA GGA AGA CCA CAC ACT AAG ATG TCA CAT ATA AAA     288
   Gly Trp Gln Asn Leu Gly Arg Pro His Thr Lys Met Ser His Ile Lys
                   85                  90                  95
   GGG CAT GAA GGA GAC CCC TGC CTA CGA TCA TCA GAC TGC ATT GAA GGG     336
   Gly His Glu Gly Asp Pro Cys Leu Arg Ser Ser Asp Cys Ile Glu Gly
               100                 105                 110
   TTT TGC TGT GCT CGT CAT TTC TGG ACC AAA ATC TGC AAA CCA GTG CTC     384
   Phe Cys Cys Ala Arg His Phe Trp Thr Lys Ile Cys Lys Pro Val Leu
           115                 120                 125
   CAT CAG GGG GAA GTC TGT ACC AAA CAA CGC AAG AAG GGT TCT CAT GGG     432
   His Gln Gly Glu Val Cys Thr Lys Gln Arg Lys Lys Gly Ser His Gly
       130                 135                 140
   CTG GAA ATT TTC CAG CGT TGC GAC TGT GCG AAG GGC CTG TCT TGC AAA     480
   Leu Glu Ile Phe Gln Arg Cys Asp Cys Ala Lys Gly Leu Ser Cys Lys
   145                 150                 155                 160
   GTA TGG AAA GAT GCC ACC TAC TCC TCC AAA GCC AGA CTC CAT GTG TGT     528
   Val Trp Lys Asp Ala Thr Tyr Ser Ser Lys Ala Arg Leu His Val Cys
                   165                 170                 175
   CAG AAA ATT                                                         537
   Gln Lys Ile
(2) INFORMATION FOR SEQ ID NO:13:
   (i) SEQUENCE CHARACTERISTICS:
      (A) LENGTH: 928 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 75..800
   (xi) SEQUENCE DESCRIPTION: SEQ ID NO:13:
   CTCGAGGCCA AAATTCGGCA CGAGGCCGGG CTGTGGTCTA GCATAAAGGC GGAGCCCAGA    60
   AGAAGGGGCG GGGT ATG GGA GAA GCC TCC CCA CCT GCC CCC GCA AGG CGG     110
                   Met Gly Glu Ala Ser Pro Pro Ala Pro Ala Arg Arg
                     1               5                  10
   CAT CTG CTG GTC CTG CTG CTG CTC CTC TCT ACC CTG GTG ATC CCC TCC     158
   His Leu Leu Val Leu Leu Leu Leu Leu Ser Thr Leu Val Ile Pro Ser
           15                  20                  25
   GCT GCA GCT CCT ATC CAT GAT GCT GAC GCC CAA GAG AGC TCC TTG GGT     206
   Ala Ala Ala Pro Ile His Asp Ala Asp Ala Gln Glu Ser Ser Leu Gly
       30                  35                  40
   CTC ACA GGC CTC CAG AGC CTA CTC CAA GGC TTC AGC CGA CTT TTC CTG     254
   Leu Thr Gly Leu Gln Ser Leu Leu Gln Gly Phe Ser Arg Leu Phe Leu
    45                  50                  55                  60
   AAA GGT AAC CTG CTT CGG GGC ATA GAC AGC TTA TTC TCT GCC CCC ATG     302
   Lys Gly Asn Leu Leu Arg Gly Ile Asp Ser Leu Phe Ser Ala Pro Met
                    65                  70                  75
   GAC TTC CGG GGC CTC CCT GGG AAC TAC CAC AAA GAG GAG AAC CAG GAG     350
   Asp Phe Arg Gly Leu Pro Gly Asn Tyr His Lys Glu Glu Asn Gln Glu
                80                  85                  90
   CAC CAG CTG GGG AAC AAC ACC CTC TCC AGC CAC CTC CAG ATC GAC AAG      398
   His Gln Leu Gly Asn Asn Thr Leu Ser Ser His Leu Gln Ile Asp Lys
           95                  100                 105
   ATG ACC GAC AAC AAG ACA GGA GAG GTG CTG ATC TCC GAG AAT GTG GTG      446
   Met Thr Asp Asn Lys Thr Gly Glu Val Leu Ile Ser Glu Asn Val Val
       110                 115                 120
   GCA TCC ATT CAA CCA GCG GAG GGG AGC TTC GAG GGT GAT TTG AAG GTA      494
   Ala Ser Ile Gln Pro Ala Glu Gly Ser Phe Glu Gly Asp Leu Lys Val
   125                 130                 135                 140
   CCC AGG ATG GAG GAG AAG GAG GCC CTG GTA CCC ATC CAG AAG GCC ACG      542
   Pro Arg Met Glu Glu Lys Glu Ala Leu Val Pro Ile Gln Lys Ala Thr
                   145                 150                 155
   GAC AGC TTC CAC ACA GAA CTC CAT CCC CGG GTG GCC TTC TGG ATC ATT      590
   Asp Ser Phe His Thr Glu Leu His Pro Arg Val Ala Phe Trp Ile Ile
               160                 165                 170
   AAG CTG CCA CGG CGG AGG TCC CAC CAG GAT GCC CTG GAG GGC GGC CAC      638
   Lys Leu Pro Arg Arg Arg Ser His Gln Asp Ala Leu Glu Gly Gly His
           175                 180                 185
   TGG CTC AGC GAG AAG CGA CAC CGC CTG CAG GCC ATC CGG GAT GGA CTC      686
   Trp Leu Ser Glu Lys Arg His Arg Leu Gln Ala Ile Arg Asp Gly Leu
       190                 195                 200
   CGC AAG GGG ACC CAC AAG GAC GTC CTA GAA GAG GGG ACC GAG AGC TCC      734
   Arg Lys Gly Thr His Lys Asp Val Leu Glu Glu Gly Thr Glu Ser Ser
   205                 210                 215                 220
   TCC CAC TCC AGG CTG TCC CCC CGA AAG ACC CAC TTA CTG TAC ATC CTC      782
   Ser His Ser Arg Leu Ser Pro Arg Lys Thr His Leu Leu Tyr Ile Leu
                   225                 230                 235
   AGG CCC TCT CGG CAG CTG TAGGGGTGGG GACCGGGGAG CACCTGCCTG             830
   Arg Pro Ser Arg Gln Leu
               240
   TAGCCCCCAT CAGACCCTGC CCCAAGCACC ATATGGAAAT AAAGTTCTTT CTTACATCTA    890
   AAAAAAAAAA AAAAAAAAAA AAAAAAATTG GCGGCCGC                            928
(2) INFORMATION FOR SEQ ID NO:14:
   (i) SEQUENCE CHARACTERISTICS:
      (A) LENGTH: 242 amino acids
      (B) TYPE: amino acid
      (D) TOPOLOGY: linear
   (ii) MOLECULE TYPE: protein
   (xi) SEQUENCE DESCRIPTION: SEQ ID NO:14:
   Met Gly Glu Ala Ser Pro Pro Ala Pro Ala Arg Arg His Leu Leu Val
    1               5                  10                  15
   Leu Leu Leu Leu Leu Ser Thr Leu Val Ile Pro Ser Ala Ala Ala Pro
               20                  25                  30
   Ile His Asp Ala Asp Ala Gln Glu Ser Ser Leu Gly Leu Thr Gly Leu
           35                  40                  45
   Gln Ser Leu Leu Gln Gly Phe Ser Arg Leu Phe Leu Lys Gly Asn Leu
       50                  55                  60
   Leu Arg Gly Ile Asp Ser Leu Phe Ser Ala Pro Met Asp Phe Arg Gly
   65                  70                  75                  80
   Leu Pro Gly Asn Tyr His Lys Glu Glu Asn Gln Glu His Gln Leu Gly
                   85                  90                  95
   Asn Asn Thr Leu Ser Ser His Leu Gln Ile Asp Lys Met Thr Asp Asn
               100                 105                 110
   Lys Thr Gly Glu Val Leu Ile Ser Glu Asn Val Val Ala Ser Ile Gln
           115                 120                 125
   Pro Ala Glu Gly Ser Phe Glu Gly Asp Leu Lys Val Pro Arg Met Glu
       130                 135                 140
   Glu Lys Glu Ala Leu Val Pro Ile Gln Lys Ala Thr Asp Ser Phe His
   145                 150                 155                 160
   Thr Glu Leu His Pro Arg Val Ala Phe Trp Ile Ile Lys Leu Pro Arg
                   165                 170                 175
   Arg Arg Ser His Gln Asp Ala Leu Glu Gly Gly His Trp Leu Ser Glu
               180                 185                 190
   Lys Arg His Arg Leu Gln Ala Ile Arg Asp Gly Leu Arg Lys Gly Thr
           195                 200                 205
   His Lys Asp Val Leu Glu Glu Gly Thr Glu Ser Ser Ser His Ser Arg
       210                 215                 220
   Leu Ser Pro Arg Lys Thr His Leu Leu Tyr Ile Leu Arg Pro Ser Arg
   225                 230                 235                 240
   Gln Leu
(2) INFORMATION FOR SEQ ID NO:15:
   (i) SEQUENCE CHARACTERISTICS:
      (A) LENGTH: 726 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..726
   (xi) SEQUENCE DESCRIPTION: SEQ ID NO:15:
   ATG GGA GAA GCC TCC CCA CCT GCC CCC GCA AGG CGG CAT CTG CTG GTC       48
   Met Gly Glu Ala Ser Pro Pro Ala Pro Ala Arg Arg His Leu Leu Val
     1               5                  10                  15
   CTG CTG CTG CTC CTC TCT ACC CTG GTG ATC CCC TCC GCT GCA GCT CCT       96
   Leu Leu Leu Leu Leu Ser Thr Leu Val Ile Pro Ser Ala Ala Ala Pro
                    20                  25                  30
   ATC CAT GAT GCT GAC GCC CAA GAG AGC TCC TTG GGT CTC ACA GGC CTC      144
   Ile His Asp Ala Asp Ala Gln Glu Ser Ser Leu Gly Leu Thr Gly Leu
                35                  40                  45
   CAG AGC CTA CTC CAA GGC TTC AGC CGA CTT TTC CTG AAA GGT AAC CTG      192
   Gln Ser Leu Leu Gln Gly Phe Ser Arg Leu Phe Leu Lys Gly Asn Leu
            50                  55                  60
   CTT CGG GGC ATA GAC AGC TTA TTC TCT GCC CCC ATG GAC TTC CGG GGC      240
   Leu Arg Gly Ile Asp Ser Leu Phe Ser Ala Pro Met Asp Phe Arg Gly
    65                  70                  75                  80
   CTC CCT GGG AAC TAC CAC AAA GAG GAG AAC CAG GAG CAC CAG CTG GGG      288
   Leu Pro Gly Asn Tyr His Lys Glu Glu Asn Gln Glu His Gln Leu Gly
                    85                  90                  95
   AAC AAC ACC CTC TCC AGC CAC CTC CAG ATC GAC AAG ATG ACC GAC AAC      336
   Asn Asn Thr Leu Ser Ser His Leu Gln Ile Asp Lys Met Thr Asp Asn
               100                 105                 110
   AAG ACA GGA GAG GTG CTG ATC TCC GAG AAT GTG GTG GCA TCC ATT CAA      384
   Lys Thr Gly Glu Val Leu Ile Ser Glu Asn Val Val Ala Ser Ile Gln
           115                 120                 125
   CCA GCG GAG GGG AGC TTC GAG GGT GAT TTG AAG GTA CCC AGG ATG GAG      432
   Pro Ala Glu Gly Ser Phe Glu Gly Asp Leu Lys Val Pro Arg Met Glu
       130                 135                 140
   GAG AAG GAG GCC CTG GTA CCC ATC CAG AAG GCC ACG GAC AGC TTC CAC      480
   Glu Lys Glu Ala Leu Val Pro Ile Gln Lys Ala Thr Asp Ser Phe His
   145                 150                 155                 160
   ACA GAA CTC CAT CCC CGG GTG GCC TTC TGG ATC ATT AAG CTG CCA CGG      528
   Thr Glu Leu His Pro Arg Val Ala Phe Trp Ile Ile Lys Leu Pro Arg
                   165                 170                 175
   CGG AGG TCC CAC CAG GAT GCC CTG GAG GGC GGC CAC TGG CTC AGC GAG      576
   Arg Arg Ser His Gln Asp Ala Leu Glu Gly Gly His Trp Leu Ser Glu
               180                 185                 190
   AAG CGA CAC CGC CTG CAG GCC ATC CGG GAT GGA CTC CGC AAG GGG ACC      624
   Lys Arg His Arg Leu Gln Ala Ile Arg Asp Gly Leu Arg Lys Gly Thr
           195                 200                 205
   CAC AAG GAC GTC CTA GAA GAG GGG ACC GAG AGC TCC TCC CAC TCC AGG      672
   His Lys Asp Val Leu Glu Glu Gly Thr Glu Ser Ser Ser His Ser Arg
       210                 215                 220
   CTG TCC CCC CGA AAG ACC CAC TTA CTG TAC ATC CTC AGG CCC TCT CGG      720
   Leu Ser Pro Arg Lys Thr His Leu Leu Tyr Ile Leu Arg Pro Ser Arg
   225                 230                 235                     240
   CAG CTG                                                              726
   Gln Leu
(2) INFORMATION FOR SEQ ID NO:16:
   (i) SEQUENCE CHARACTERISTICS:
      (A) LENGTH: 2381 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 110..1156
   (xi) SEQUENCE DESCRIPTION: SEQ ID NO:16:
(2) INFORMATION FOR SEQ ID NO:17:
   (i) SEQUENCE CHARACTERISTICS:
      (A) LENGTH: 349 amino acids
      (B) TYPE: amino acid
      (D) TOPOLOGY: linear
   (ii) MOLECULE TYPE: protein
   (xi) SEQUENCE DESCRIPTION: SEQ ID NO:17:
   Met Gln Arg Leu Gly Gly Ile Leu Leu Cys Thr Leu Leu Ala Ala Ala
    1               5                  10                  15
   Val Pro Thr Ala Pro Ala Pro Ser Pro Thr Val Thr Trp Thr Pro Ala
               20                  25                  30
   Glu Pro Gly Pro Ala Leu Asn Tyr Pro Gln Glu Glu Ala Thr Leu Asn
           35                  40                  45
   Glu Met Phe Arg Glu Val Glu Glu Leu Met Glu Asp Thr Gln His Lys
       50                  55                  60
   Leu Arg Ser Ala Val Glu Glu Met Glu Ala Glu Glu Ala Ala Ala Lys
   65                  70                  75                  80
   Thr Ser Ser Glu Val Asn Leu Ala Ser Leu Pro Pro Asn Tyr His Asn
                   85                  90                  95
   Glu Thr Ser Thr Glu Thr Arg Val Gly Asn Asn Thr Val His Val His
               100                 105                 110
   Gln Glu Val His Lys Ile Thr Asn Asn Gln Ser Gly Gln Val Val Phe
           115                 120                 125
   Ser Glu Thr Val Ile Thr Ser Val Gly Asp Glu Glu Gly Lys Arg Ser
       130                 135                 140
   His Glu Cys Ile Ile Asp Glu Asp Cys Gly Pro Thr Arg Tyr Cys Gln
   145                 150                 155                 160
   Phe Ser Ser Phe Lys Tyr Thr Cys Gln Pro Cys Arg Asp Gln Gln Met
                   165                 170                 175
   Leu Cys Thr Arg Asp Ser Glu Cys Cys Gly Asp Gln Leu Cys Ala Trp
               180                 185                 190
   Gly His Cys Thr Gln Lys Ala Thr Lys Gly Gly Asn Gly Thr Ile Cys
           195                 200                 205
   Asp Asn Gln Arg Asp Cys Gln Pro Gly Leu Cys Cys Ala Phe Gln Arg
       210                 215                 220
   Gly Leu Leu Phe Pro Val Cys Thr Pro Leu Pro Val Glu Gly Glu Leu
   225                 230                 235                 240
   Cys His Asp Pro Thr Ser Gln Leu Leu Asp Leu Ile Thr Trp Glu Leu
                   245                 250                 255
   Glu Pro Glu Gly Ala Leu Asp Arg Cys Pro Cys Ala Ser Gly Leu Leu
               260                 265                 270
   Cys Gln Pro His Ser His Ser Leu Val Tyr Met Cys Lys Pro Ala Phe
           275                 280                 285
   Val Gly Ser His Asp His Ser Glu Glu Ser Gln Leu Pro Arg Glu Ala
       290                 295                 300
   Pro Asp Glu Tyr Glu Asp Val Gly Phe Ile Gly Glu Val Arg Gln Glu
   305                 310                 315                 320
   Leu Glu Asp Leu Glu Arg Ser Leu Ala Gln Glu Met Ala Phe Glu Gly
                   325                 330                 335
   Pro Ala Pro Val Glu Ser Leu Gly Gly Glu Glu Glu Ile
               340                 345
(2) INFORMATION FOR SEQ ID NO:18:
   (i) SEQUENCE CHARACTERISTICS:
      (A) LENGTH: 1047 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..1047
   (xi) SEQUENCE DESCRIPTION: SEQ ID NO:18:
   ATG CAG CGG CTC GGG GGT ATT TTG CTG TGT ACA CTG CTG GCG GCG GCG      48
   Met Gln Arg Leu Gly Gly Ile Leu Leu Cys Thr Leu Leu Ala Ala Ala
     1               5                  10                  15
   GTC CCC ACT GCT CCT GCT CCT TCC CCG ACG GTC ACT TGG ACT CCG GCG      96
   Val Pro Thr Ala Pro Ala Pro Ser Pro Thr Val Thr Trp Thr Pro Ala
                20                  25                  30
   GAG CCG GGC CCA GCT CTC AAC TAC CCT CAG GAG GAA GCT ACG CTC AAT 144
   Glu Pro Gly Pro Ala Leu Asn Tyr Pro Gln Glu Glu Ala Thr Leu Asn
   35 40 45
   GAG ATG TTT CGA GAG GTG GAG GAG CTG ATG GAA GAC ACT CAG CAC AAA 192
   Glu Met Phe Arg Glu Val Glu Glu Leu Met Glu Asp Thr Gln His Lys
   50 55 60
   CTG CGC AGT GCC GTG GAG GAG ATG GAG GCG GAA GAA GCA GCT GCT AAA 240
   Leu Arg Ser Ala Val Glu Glu Met Glu Ala Glu Glu Ala Ala Ala Lys
   65 70 75 80
   ACG TCC TCT GAG GTG AAC CTG GCA AGC TTA CCT CCC AAC TAT CAC AAT 288
   Thr Ser Ser Glu Val Asn Leu Ala Ser Leu Pro Pro Asn Tyr His Asn
   85 90 95
   GAG ACC AGC ACG GAG ACC AGG GTG GGA AAT AAC ACA GTC CAT GTG CAC 336
   Glu Thr Ser Thr Glu Thr Arg Val Gly Asn Asn Thr Val His Val His
   100 105 110
   CAG GAA GTT CAC AAG ATA ACC AAC AAC CAG AGT GGA CAG GTG GTC TTT 384
   Gln Glu Val His Lys Ile Thr Asn Asn Gln Ser Gly Gln Val Val Phe
   115 120 125
   TCT GAG ACA GTC ATT ACA TCT GTA GGG GAT GAA GAA GGC AAG AGG AGC 432
   Ser Glu Thr Val Ile Thr Ser Val Gly Asp Glu Glu Gly Lys Arg Ser
   130 135 140
   CAT GAA TGT ATC ATT GAT GAA GAC TGT GGG CCC ACC AGG TAC TGC CAG 480
   His Glu Cys Ile Ile Asp Glu Asp Cys Gly Pro Thr Arg Tyr Cys Gln
   145 150 155 160
   TTC TCC AGC TTC AAG TAC ACC TGC CAG CCA TGC CGG GAC CAG CAG ATG 528
   Phe Ser Ser Phe Lys Tyr Thr Cys Gln Pro Cys Arg Asp Gln Gln Met
   165 170 175
   CTA TGC ACC CGA GAC AGT GAG TGC TGT GGA GAC CAG CTG TGT GCC TGG 576
   Leu Cys Thr Arg Asp Ser Glu Cys Cys Gly Asp Gln Leu Cys Ala Trp
   180 185 190
   GGT CAC TGC ACC CAA AAG GCC ACC AAA GGT GGC AAT GGG ACC ATC TGT 624
   Gly His Cys Thr Gln Lys Ala Thr Lys Gly Gly Asn Gly Thr Ile Cys
   195 200 205
   GAC AAC CAG AGG GAT TGC CAG CCT GGC CTG TGT TGT GCC TTC CAA AGA 672
   Asp Asn Gln Arg Asp Cys Gln Pro Gly Leu Cys Cys Ala Phe Gln Arg
   210 215 220
   GGC CTG CTG TTC CCC GTG TGC ACA CCC CTG CCC GTG GAG GGA GAG CTC 720
   Gly Leu Leu Phe Pro Val Cys Thr Pro Leu Pro Val Glu Gly Glu Leu
   225 230 235 240
   TGC CAT GAC CCC ACC AGC CAG CTG CTG GAT CTC ATC ACC TGG GAA CTG 768
   Cys His Asp Pro Thr Ser Gln Leu Leu Asp Leu Ile Thr Trp Glu Leu
   245 250 255
   GAG CCT GAA GGA GCT TTG GAC CGA TGC CCC TGC GCC AGT GGC CTC CTA     816
   Glu Pro Glu Gly Ala Leu Asp Arg Cys Pro Cys Ala Ser Gly Leu Leu
               260                 265                 270
   TGC CAG CCA CAC AGC CAC AGT CTG GTG TAC ATG TGC AAG CCA GCC TTC     864
   Cys Gln Pro His Ser His Ser Leu Val Tyr Met Cys Lys Pro Ala Phe
           275                 280                 285
   GTG GGC AGC CAT GAC CAC AGT GAG GAG AGC CAG CTG CCC AGG GAG GCC     912
   Val Gly Ser His Asp His Ser Glu Glu Ser Gln Leu Pro Arg Glu Ala
       290                 295                 300
   CCG GAT GAG TAC GAA GAT GTT GGC TTC ATA GGG GAA GTG CGC CAG GAG     960
   Pro Asp Glu Tyr Glu Asp Val Gly Phe Ile Gly Glu Val Arg Gln Glu
   305                 310                 315                 320
   CTG GAA GAC CTG GAG CGG AGC CTA GCC CAG GAG ATG GCA TTT GAG GGG    1008
   Leu Glu Asp Leu Glu Arg Ser Leu Ala Gln Glu Met Ala Phe Glu Gly
                   325                 330                 335
   CCT GCC CCT GTG GAG TCA CTA GGC GGA GAG GAG GAG ATT                1047
   Pro Ala Pro Val Glu Ser Leu Gly Gly Glu Glu Glu Ile
               340                 345